(19) Europäisches Patentamt
European Patent Office
Office européen des brevets

(11) EP 3 647 426 A1

(12) # EUROPEAN PATENT APPLICATION
published in accordance with Art. 153(4) EPC

(43) Date of publication:
**06.05.2020 Bulletin 2020/19**

(21) Application number: **18825349.6**

(22) Date of filing: **26.06.2018**

(51) Int Cl.:
***C12N 15/62*** *(2006.01)* ***C07K 7/00*** *(2006.01)*
***C07K 14/00*** *(2006.01)* ***C07K 16/00*** *(2006.01)*
***C07K 19/00*** *(2006.01)* ***C12N 1/15*** *(2006.01)*
***C12N 1/19*** *(2006.01)* ***C12N 1/21*** *(2006.01)*
***C12N 5/10*** *(2006.01)*

(86) International application number:
**PCT/JP2018/024197**

(87) International publication number:
**WO 2019/004213 (03.01.2019 Gazette 2019/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.06.2017 JP 2017124596**

(71) Applicant: **Riken**
**Wako-shi, Saitama 351-0198 (JP)**

(72) Inventors:
- **MIKOSHIBA, Katsuhiko**
**Wako-shi**
**Saitama 351-0198 (JP)**
- **KABAYAMA, Hiroyuki**
**Wako-shi**
**Saitama 351-0198 (JP)**

(74) Representative: **Graf von Stosch Patentanwaltsgesellschaft mbH**
**Prinzregentenstraße 22**
**80538 München (DE)**

(54) **FUSION PROTEIN**

(57) Provided is technology for using a given antigen-binding peptide as an intrabody that is functionable in a cell. An intracellular-stabilizer peptide consisting of 10 to 39 amino acids, at least 45% of the 10 to 39 amino acids being acidic amino acids, is fused with the antigen-binding peptide so as to be intracellularly expressed as a fusion protein.

EP 3 647 426 A1

## Description

Technical Field

The present invention relates to a fusion protein which has an antigen-binding function and is expressed in an intracellular environment.

Background Art

Intracellularly functionable antibodies, i.e., intrabodies are capable of affecting functions of cells of a higher organism by recognizing and binding to antigens (target molecules) in the cells. These antigens can be important intracellular therapeutic targets which can be inactivated upon binding to intrabodies. Further, in terms of research technique, use of intrabodies has been attracting attention as means for directly and specifically inhibiting functions of proteins by binding to antibodies inside cells.

To produce an intrabody, it is typical to (i) first use a standard method to prepare monoclonal-antibody-producing hybridomas capable of recognizing an antigen, (ii) then construct, from cDNA of each monoclonal-antibody-producing hybridoma, an intracellular expression vector containing DNA encoding a single chain Fv (scFv), and (iii) thus using a complex of heavy chain (VH) and light chain (VL) as an intrabody.

An antibody ordinarily functions by circulating in an extracellular environment (e.g., blood) inside the body and recognizing an extracellular antigen. It is thus assumed that an antibody acts in an extracellular environment. As such, in a case where an antibody is expressed in cytoplasm, the antibody suffers folding and stability issues leading to a lower expression level and a limited half-life of a domain of the antibody. It thus cannot be expected that any antibody functions as an intrabody. Further, scFv is constituted by a heavy chain and a light chain which are connected by a flexible peptide linker, and thus has a tertiary structure not as stable as an original antibody. Furthermore, even if scFv that functions in a test tube is identified, that scFv may not necessarily able to exhibit an expected function when expressed in a cell, since many aspects of an intracellular environment are not reflected in the environment inside the test tube.

Thus, there have been conducted studies on (i) regularity among antibodies capable of withstanding cytoplasmic conditions and (ii) a technique for expecting such antibodies, in order to enable screening of an antibody that functions as an intrabody. These are methods for selecting an antibody capable of functioning in cells. It is difficult, however, to actually obtain an intracellularly stable antibody with use of these methods. There has not been established a technology that utilizes an existing antibody or a newly isolated antibody as an intrabody.

Citation list

[Patent Literature]

[Patent Literature 1]
International Publication No. WO 03/014960

Summary of Invention

Technical Problem

It is an object of the present invention to provide a technology for using a given antigen-binding peptide as an intrabody that stably functions in a cell.

Solution to Problem

The inventors of the present invention conducted diligent study in order to attain the object. As a result, the inventors discovered that when an antigen-binding peptide fused with a peptide tag is expressed in a cell, functional stability of the antigen-binding peptide as an intrabody changes in accordance with changes caused by the peptide tag in values of net charge and isoelectric point (pI) of the antigen-binding peptide. In particular, by designing the peptide tag so that values of charge and pI of the intrabody are sufficiently low with respect to a pH environment on a cytoplasmic surface of an endosome rather than a pH environment of the cytoplasm, the inventors achieved that the antigen-binding peptide stably functions as an intrabody in a cell by being fused with that peptide tag.

Based on the above knowledge, the inventors of the present invention further conducted study and consequently completed the present invention.

That is, the present invention is as follows:

1) A fusion protein, containing:

an intracellular-stabilizer peptide; and an antigen-binding peptide,
the intracellular-stabilizer peptide consisting of 10 to 39 amino acids, at least 45% of the 10 to 39 amino acids being acidic amino acids,
the antigen-binding peptide containing at least one of heavy chain CDR1, heavy chain CDR2, heavy chain CDR3, light chain CDR1, light chain CDR2, and light chain CDR3.

2) The fusion protein as set forth in 1), wherein:

28% or less of the 10 to 39 amino acids of the intracellular-stabilizer peptide are basic amino acids; or
the intracellular-stabilizer peptide contains no basic amino acid.

3) The fusion protein as set forth in 2), wherein the intracellular-stabilizer peptide contains no basic amino acid.
4) The fusion protein as set forth in 2), wherein the intracellular-stabilizer peptide contains at least one histidine as a basic amino acid.
5) The fusion protein as set forth in any one 1) through 4), wherein:

the intracellular-stabilizer peptide consists of 14 to 25 amino acids, at least 50% of the 14 to 25 amino acids being acidic amino acids; and
the intracellular-stabilizer peptide containing neither a portion consisting of 8 or more consecutive acidic amino acids nor a portion consisting of 4 or more consecutive amino acids that are not acidic amino acids.

6) The fusion protein as set forth in 5), wherein the intracellular-stabilizer peptide contains neither a portion consisting of 3 or more consecutive acidic amino acids nor a portion consisting of 3 or more consecutive amino acids that are not acidic amino acids.
7) The fusion protein as set forth in 1), wherein the intracellular-stabilizer peptide contains
the following amino acid sequence (1) or (2):

$$-X_n-X_A-X_A-X_n-X_A-(X_A \text{ or } X_n)-X_n-X_A-(X_A \text{ or } X_n)-X_n-(X_A \text{ or } X_n)-X_A- \ldots(1);$$

or $-X_n-X_A-X_n-X_A-X_n-X_A-X_A-X_n-X_A-X_n-X_n-X_A-X_A-X_n-X_n-X_A-$ ...(2),
where $X_A$ is an acidic amino acid and $X_n$ is an amino acid that is not an acidic amino acid.
8) The fusion protein as set forth in 7), wherein $X_A$ is aspartic acid or glutamic acid, and $X_n$ is an amino acid selected from the group consisting of asparagine, glutamine, proline, tyrosine, and valine.
9) A polynucleotide encoding a fusion protein recited in any one of 1) through 8) above.
10) An expression vector containing a polynucleotide recited in 9) above.
11) A method for producing a cell capable of expressing an antigen-binding peptide in the cell, the method including the step of: introducing a polynucleotide recited in 9) or an expression vector recited in 10) into a cell.
12) A cell capable of expressing a fusion protein recited in any one of 1) through 8) above.

Brief Description of Drawings

Fig. 1 is a view illustrating results of evaluation of the expression and binding capacity of scFv-A36.
Fig. 2 is a view illustrating information of the amino acid sequence of CDR in $V_H$ region of scFv-A36.
Fig. 3 is a view illustrating results of evaluation of (i) the expression of scFv-GFPA36 and scFv-GFPM4 in cultured neurons and (ii) the activity of scFv-GFPA36 and scFv-GFPM4 to inhibit the function of a target molecule.
Fig. 4 is a view illustrating confirmation of aggregation of scFv-GFPA36 in neurons of mouse brain.
Fig. 5 is a view illustrating the pI and net charge of scFv-A36 and scFv-M4 and results of evaluation of the intracellular aggregation property of scFv-A36 and scFv-M4.
Fig. 6 is a view illustrating results of examination of the generality of the effects of s3Flag- and HA-peptide tagging on the increase in the net negative charge of intrabodies at the lower pH using other 94 scFv protein sequences obtained by NCBI blast search by using of scFv-A36 protein sequence as a query sequence.

Fig. 7 is a view illustrating results of examination of the biochemical property of s3Flag-scFvA-36-HA construct.

Fig. 8 is a view illustrating results of examination of intracellular expression of s3Flag-scFv-A36-HA and s3Flag-scFv-M4-HA in neurons of mouse brain.

Fig. 9 is a view illustrating results of examination of the expression patterns of s3Flag-scFv-A36-HA and s3Flag-scFv-M4-HA in substantia nigra region.

Fig. 10 is a view illustrating results of observation of long-term (6 months) expression of both s3Flag-scFv-A36-HA and s3Flag-scFv-M4-HA in dopamine neurons.

Fig. 11 is a view illustrating results of examination of the functional activity of s3Flag-scFv-A36-HA against Syt I in dopamine neurons *in vivo.*

Fig. 12 is a view illustrating results of motor behavior test and immunohistological staining of s3Flag-scFv-A36-HA-expressing mice and s3Flag-scFv-M4-HA-expressing mice.

Fig. 13 is a view illustrating results of examination of the expression and antitumor activity of STAND-Y13-259.

Fig. 14 is a view illustrating results of examination of the expression and antitumor activity of DE2.0-Y13-259-HA.

Fig. 15 is a view illustrating results of examination of a change in the net negative charge of scFv-6E, caused by fusion of scFv-6E and a tag.

Fig. 16 is a view illustrating results of observation of intracellular expression of STAND-6E-LYS and DE5.0-6E.

Fig. 17 a view illustrating results of α-synuclein aggregation assay.

Fig. 18 a view illustrating results of evaluation of α-synuclein aggregation.

Description of Embodiments

<0. Definition>

(Peptide)

In the present specification, the term "peptide" is interchangeable with "polypeptide" or "protein". A "peptide" includes a structure of amino acids linked by a peptide bond. A "peptide" may further include, for example, a structure of a sugar chain, an isoprenoid group, or the like. The term "peptide", unless otherwise specified, includes in its scope a peptide that contains an already known analog of a naturally-occurring amino acid having a function as well as the naturally-occurring amino acid.

(Acidic amino acid)

In the present specification, the term "acidic amino acid" refers to an amino acid having an isoelectric point of 3.99 or less. This amino acid may be a naturally-occurring amino acid or an analog of a naturally-occurring amino acid. Examples of a naturally-occurring acidic amino acid include aspartic acid and glutamic acid.

(Basic amino acid)

In the present specification, the term "basic amino acid" refers to an amino acid having an isoelectric point of 7.40 or more. This amino acid may be a naturally-occurring amino acid or an analog of a naturally-occurring amino acid. Examples of a naturally-occurring basic amino acid include histidine, lysine, and arginine.

(Amino acid that is neither acidic amino acid nor basic amino acid)

In the present specification, an amino acid that fits neither the above definition of an acidic amino acid nor the above definition of a basic amino acid in terms of pH of the amino acid itself is referred to generally as "an amino acid that is neither an acidic amino acid nor a basic amino acid" or "a substantially neutral amino acid". That is, such an amino acid has an isoelectric point of more than 3.99 and less than 7.40. The amino acid may be a naturally-occurring amino acid or an analog of a naturally-occurring amino acid.

(A and/or B)

In the present specification, the expression "A and/or B" is a concept covering both "A and B" and "A or B", and is interchangeable with "at least one of A and B".

<1. Fusion protein>

A fusion protein of the present invention is a fusion protein containing:

an intracellular-stabilizer peptide; and
an antigen-binding peptide,
the intracellular-stabilizer peptide consisting of 10 to 39 amino acids, at least 45% of the 10 to 39 amino acids being acidic amino acids,
the antigen-binding peptide containing at least one of heavy chain CDR1, heavy chain CDR2, heavy chain CDR3, light chain CDR1, light chain CDR2, and light chain CDR3.

The fusion protein of the present invention serves as an intrabody by being expressed in a given cell.

[Intracellular-stabilizer peptide]

(Peptide length and containing acidic amino acids)

The intracellular-stabilizer peptide can be constituted by any number of amino acids. The intracellular-stabilizer peptide is preferably a peptide consisting of 50 or less amino acids, more preferably a peptide consisting of 10 to 39 amino acids. Note that at least 45% of all amino acids constituting the intracellular-stabilizer peptide are acidic amino acids.

A percentage of the acidic amino acids among all amino acids constituting the intracellular-stabilizer peptide is not particularly limited, but is preferably 50% or more. The percentage of the acidic amino acids among all amino acids constituting the intracellular-stabilizer peptide may be, for example, 53% or more or 55% or more. The percentage may be preferably 60% or more, and may be preferably 65% or more or 70% or more. In general, an isoelectric point of a polypeptide containing acidic amino acids decreases as a percentage of the acidic amino acids increases.

The number of the acidic amino acids constituting the intracellular-stabilizer peptide is not particularly limited, provided that any of the above ranges of percentage of the acidic amino acids among all amino acids constituting the intracellular-stabilizer peptide is met. For example, the number of the acidic amino acids constituting the intracellular-stabilizer peptide is 6 or more or 7 or more, preferably 8 or more or 9 or more, more preferably 10 or more, even more preferably 11 or more, and particularly preferably 12 or more.

The acidic amino acids constituting the intracellular-stabilizer peptide are each preferably selected from the group consisting of aspartic acid and glutamic acid.

From the viewpoint of increasing a degree of contribution of the intracellular-stabilizer peptide in the fusion protein, the number of the amino acids constituting the intracellular-stabilizer peptide is preferably 14 or more, more preferably 15 or more, even more preferably 18 or more, and particularly preferably 20 or more. Further, from the viewpoint of reducing a size of the fusion protein, the number of the amino acids constituting the intracellular-stabilizer peptide is preferably 35 or less, more preferably 30 or less, and even more preferably 25 or less. Note that in a case where a length (the number of the amino acids constituting the intracellular-stabilizer peptide) of the intracellular-stabilizer peptide is 9 amino acids or less, the intracellular-stabilizer peptide may be inadequate from the viewpoint of contribution of the intracellular-stabilizer peptide in the fusion protein. Further, the intracellular-stabilizer peptide having a length of 9 amino acids or less may be inadequate also from the viewpoint of antigen-specificity which the intracellular-stabilizer peptide is required to have when necessary, for example, when an antibody capable of recognizing the intracellular-stabilizer peptide is to be produced. Further, in a case where the intracellular-stabilizer peptide has a length of 40 amino acids or more, the fusion protein has an unnecessarily large size, and can accordingly cause steric hindrance in antigen recognition by the antigen-binding peptide (described later). Note, however, that the intracellular-stabilizer peptide having a length of 40 amino acids or more can be used in a case where a peptide serving as a linker is provided between and linked to the intracellular-stabilizer peptide and the antigen-binding peptide so that the intracellular-stabilizer peptide does not interfere with the antigen recognition. In that case, a plurality of intracellular-stabilizer peptides (which may be a plurality of intracellular-stabilizer peptides of the same kind or may be a combination of different kinds of intracellular-stabilizer peptides) may be used by being linked to one another via a spacer between each adjacent ones of the intracellular-stabilizer peptides.

(Case in which basic amino acid is contained)

A percentage of basic amino acids among all amino acids constituting the intracellular-stabilizer peptide is not particularly limited, and is preferably 28% or less, more preferably 27% or less or 26.5% or less, even more preferably 25% or less, 20% or less, 15% or less, or 10% or less, and particularly preferably 9% or less, 8% or less, 7% or less, 6% or less, 5% or less, 4% or less, or 3% or less. In a particularly preferable embodiment, the intracellular-stabilizer peptide contains no basic amino acid.

A preferable range of the number of basic amino acids constituting the intracellular-stabilizer peptide is not particularly limited, provided that any of the above ranges of percentage of acidic amino acids among all amino acids constituting the intracellular-stabilizer peptide is met. For example, the number of basic amino acids constituting the intracellular-stabilizer peptide is 6 or less, preferably 5 or less or 4 or less, more preferably 3 or less, even more preferably 2 or less, and particularly preferably 1 or less.

In a case where the intracellular-stabilizer peptide contains a basic amino acid, it is preferable that the basic amino acid be selected from the group consisting of lysine and histidine, and it is more preferable that the intracellular-stabilizer peptide contain at least one histidine as the basic amino acid. Histidine has a characteristic of having the lowest isoelectric point among naturally-occurring basic amino acids. In a case where the intracellular-stabilizer peptide contains a plurality of basic amino acids, a percentage of histidine among the basic amino acids is preferably 30% or more, more preferably 50% or more, even more preferably 60% or more, and particularly preferably 100%. The number of histidine residues contained in the intracellular-stabilizer peptide is, for example, 6, 5, 4, 3, 2, or 1.

(Amino acid that is neither acidic amino acid nor basic amino acid)

The intracellular-stabilizer peptide may contain an amino acid (generally referred to as a "substantially neutral amino acid) that is neither an acidic amino acid nor a basic amino acid, which have been described above.

A percentage of substantially neutral amino acids among all amino acids constituting the intracellular-stabilizer peptide is 55% or less, preferably 50% or less, and more preferably 45% or less. In a case where the intracellular-stabilizer peptide contains a substantially neutral amino acid, a lower limit of a percentage of the substantially neutral amino acid among all amino acids constituting the intracellular-stabilizer peptide is not particularly limited, and is, in an aspect, 15% or more, 20% or more, or 25% or more.

When characteristics of the intracellular-stabilizer peptide as an antigen are taken into consideration, a percentage of hydrophobic amino acids (i.e., nonpolar, substantially neutral amino acids) among all amino acids constituting the intracellular-stabilizer peptide is preferably 25% or less, more preferably 20% or less, and even more preferably 15% or less. However, when a hydrophilic amino acid-hydrophobic amino acid balance in the intracellular-stabilizer peptide is taken into consideration, it may be preferable that one or more hydrophobic amino acids are contained in the intracellular-stabilizer peptide, on the premise that the above range of percentage of hydrophobic amino acids is met. In a case where the intracellular-stabilizer peptide contains hydrophobic amino acids, the number of the hydrophobic amino acids is, for example, 1, 2, 3, or 4. It may be preferable that the number of consecutive hydrophobic amino acids be 3 or less, and it may be more preferable that the number of consecutive hydrophobic amino acids be 2 or less.

In a case where the intracellular-stabilizer peptide contains a naturally-occurring substantially neutral amino acid, the substantially neutral amino acid is preferably selected from the group consisting of asparagine, phenylalanine (a hydrophobic amino acid), glutamine, tyrosine, serine, methionine (a hydrophobic amino acid), tryptophan (a hydrophobic amino acid), valine (a hydrophobic amino acid), glycine (a hydrophobic amino acid), leucine (a hydrophobic amino acid), isoleucine (a hydrophobic amino acid), and proline (a hydrophobic amino acid), more preferably selected from the group consisting of asparagine, glutamine, tyrosine, methionine, valine, glycine, leucine, isoleucine, and proline, and even more preferably selected from the group consisting of asparagine, glutamine, tyrosine, valine, and proline. In an aspect, the intracellular-stabilizer peptide contains at least one (preferably 1 or 2) proline, at least one (preferably 8 or less, for example, 7, 6, 5, 4, 3, or 2) amino acid selected from asparagine, glutamine, and tyrosine, and at least one (preferably 1 or 2) hydrophobic amino acid that is not proline.

Note that preferable types and arrangement of substantially neutral amino acids may be determined, for example, by taking account of: stability (resistance to oxidization, hydrolysis, and the like) of the intracellular-stabilizer peptide formed; whether or not an intramolecular S-S bond is formed; whether or not an intramolecular hydrogen bond is formed; whether or not an undesired motif is formed; and the like.

(Isoelectric point)

An isoelectric point of the intracellular-stabilizer peptide can be calculated from types of amino acids constituting the intracellular-stabilizer peptide. Calculation of the isoelectric point of the intracellular-stabilizer peptide can be carried out, for example, in accordance with descriptions of Protein Caluculator (http://protcalc.sourceforge.net) or the like.

(More specific examples of intracellular-stabilizer peptide)

The following description will discuss more specific examples of the intracellular-stabilizer peptide. Note that in the description below, $X_A$ refers to an acidic amino acid, and $X_n$ refers to an amino acid that is not an acidic amino acid, i.e., $X_n$ refers to a basic amino acid and a substantially neutral amino acid, which have been described above.

Note that all of the descriptions in the section [Intracellular-stabilizer peptide] apply to the specific examples below of the intracellular-stabilizer peptide. For example, all of the above descriptions of an acidic amino acid apply to $X_A$, and all of the above descriptions of a basic amino acid and a substantially neutral amino acid apply to $X_n$. Also with regard to a length etc. of the intracellular-stabilizer peptide, all of the above descriptions apply. Specifically, for example, the examples below of the intracellular-stabilizer peptide may each be an intracellular-stabilizer peptide in which 28% or less of the amino acids are basic amino acids or be an intracellular-stabilizer peptide which contains no basic amino acid (i.e., all $X_n$ are substantially neutral amino acids). Further, the intracellular-stabilizer peptide may be an intracellular-stabilizer peptide that contains at least one histidine as a basic amino acid.

(1) Aspect 1

An intracellular-stabilizer peptide that satisfies all of the conditions listed below.

- The intracellular-stabilizer peptide consists of 10 to 39 amino acids and preferably consists of 14 to 25 amino acids.
- Of the amino acids constituting the intracellular-stabilizer peptide, at least 45% are acidic amino acids, and preferably at least 50% are acidic amino acids.
- The intracellular-stabilizer peptide contains neither a portion consisting of 8 or more consecutive $X_A$ nor a portion consisting of 4 or more consecutive $X_n$. That is, in the intracellular-stabilizer peptide, the number of consecutive $X_A$ is 7 or less and the number of consecutive $X_n$ is 3 or less.

(2) Aspect 2

An intracellular-stabilizer peptide that satisfies the conditions of Aspect 1 above and satisfies the condition listed below.

- The intracellular-stabilizer peptide contains neither a portion consisting of 6 or more consecutive $X_A$ nor a portion consisting of 4 or more consecutive $X_n$. That is, in the intracellular-stabilizer peptide, the number of consecutive $X_A$ is 5 or less and the number of consecutive $X_n$ is 3 or less.

(3) Aspect 3

An intracellular-stabilizer peptide that satisfies the conditions of Aspect 1 above and satisfies the condition listed below. - The intracellular-stabilizer peptide contains neither a portion consisting of 6 or more consecutive $X_A$ nor a portion consisting of 3 or more consecutive $X_n$. That is, in the intracellular-stabilizer peptide, the number of consecutive $X_A$ is 5 or less and the number of consecutive $X_n$ is 2 or less.

(4) Aspect 4

An intracellular-stabilizer peptide that satisfies the conditions of Aspect 1 above and satisfies the condition listed below.

- The intracellular-stabilizer peptide contains neither a portion consisting of 3 or more consecutive $X_A$ nor a portion consisting of 3 or more consecutive $X_n$. That is, in the intracellular-stabilizer peptide, the number of consecutive $X_A$ is 2 or less and the number of consecutive $X_n$ is 2 or less.

(5) Aspect 5

An intracellular-stabilizer peptide that satisfies all of the conditions listed below. Note that Aspect 5 may or may not fall under any of Aspects 1 to 4.

- The intracellular-stabilizer peptide consists of 10 to 39 amino acids and preferably consists of 14 to 25 amino acids.
- Of the amino acids constituting the intracellular-stabilizer peptide, at least 45% are acidic amino acids, and preferably at least 50% are acidic amino acids.
- The intracellular-stabilizer peptide contains the following amino acid sequence:

$$-X_n-X_A-X_A-X_n-X_A-(X_A \text{ or } X_n)-X_n-X_A-(X_A \text{ or } X_n)-X_n-(X_A \text{ or } X_n)-X_A- \ldots(1)$$

(6) Aspect 6

An intracellular-stabilizer peptide that satisfies the conditions of Aspect 5 above, has 3 to 7 more $X_A$ or $X_n$ on a left side of the amino acid sequence (1) shown in Aspect 5, and has 0 to 6 more $X_A$ or $X_n$ on a right side of the amino acid sequence (1) shown in Aspect 5.

(7) Aspect 7

An intracellular-stabilizer peptide that satisfies all of the conditions listed below. Note that Aspect 7 may or may not fall under any of Aspects 1 to 4.

- The intracellular-stabilizer peptide consists of 10 to 39 amino acids and preferably consists of 14 to 25 amino acids.
- Of the amino acids constituting the intracellular-stabilizer peptide, at least 45% are acidic amino acids, and preferably at least 50% are acidic amino acids.
- The intracellular-stabilizer peptide contains the following amino acid sequence:
  $-X_n-X_A-X_n-X_A-X_n-X_A-X_A-X_n-X_A-X_n-X_n-X_A-X_A-X_n-X_n-X_A-$ ...(2).

(8) Aspect 8

An intracellular-stabilizer peptide that satisfies the conditions of Aspect 7 above, has 0 to 9 more $X_A$ or $X_n$ on a left side of the amino acid sequence (2) shown in Aspect 7, and has 0 to 9 more $X_A$ or $X_n$ on a right side of the amino acid sequence (2) shown in Aspect 7. Note, however, that a total of the numbers of the amino acids on the respective left and right sides of the amino acid sequence (2) may be preferably 10 or less, more preferably 9 or less, and even more preferably 6 or less or 5 or less.

(9) Aspect 9

An intracellular-stabilizer peptide that falls under any one of Aspects 1 to 8 above, wherein $X_A$ is aspartic acid or glutamic acid, and $X_n$ is an amino acid selected from the group consisting of asparagine, glutamine, proline, tyrosine, and valine.

(10) Aspect 10

An intracellular-stabilizer peptide that falls under any one of Aspects 5 to 6 above, wherein: $X_A$ is aspartic acid or glutamic acid; and among $X_n$, (i) one or more of 2th to 6th (preferably 2th to 4th, more preferably 2nd to 3rd) $X_n$ from the left side of the amino acid sequence (1) in the amino acid sequence (1) are each proline and (ii) each $X_n$ that is not proline is an amino acid selected from the group consisting of asparagine, glutamine, tyrosine, and valine. As an example, one of the 2nd to 3rd $X_n$ from the left side of the amino acid sequence (1) in the amino acid sequence (1) is proline, and the other $X_n$ contained in the amino acid sequence (1) are each an amino acid selected from the group consisting of asparagine, glutamine, valine, and tyrosine. As another example, one of the 2nd to 3rd $X_n$ from the left side of the amino acid sequence (1) in the amino acid sequence (1) is proline, and the other $X_n$ contained in the amino acid (1) are each an amino acid selected from the group consisting of asparagine, glutamine, and tyrosine.

(11) Aspect 11

An intracellular-stabilizer peptide that falls under any one of Aspects 7 to 8 above, wherein: $X_A$ is aspartic acid or glutamic acid; and among $X_n$, (i) one or more of 3rd to 6th (preferably 3rd to 5th, more preferably 4th to 5th) $X_n$ from the left side of the amino acid sequence (2) in the amino acid sequence (2) are each proline and (ii) each $X_n$ that is not proline is an amino acid selected from the group consisting of asparagine, glutamine, tyrosine, and valine. As an example, one of the 4th to 5th $X_n$ from the left side of the amino acid sequence (2) in the amino acid sequence (2) is proline, the other of the 4th to 5th $X_n$ is valine, and the other $X_n$ contained in the amino acid sequence (2) are each an amino acid selected from the group consisting of asparagine, glutamine, and tyrosine.

(12) Aspect 12

An intracellular-stabilizer peptide consists of any one of the following amino acid sequences.
MDYKDHDGDYKDHDIDYKDDDDK (SEQ ID NO: 1);
EEDQDDEDDEDQDD (SEQ ID NO: 2);
NDEYEDPDEQDDEND (SEQ ID NO: 3);
QDEVDEPEDEEDNDD (SEQ ID NO: 4);
QDEVDEPEDEDENDD (SEQ ID NO: 5);
QDEVDEPEDEDENQD (SEQ ID NO: 6);
QDNVDEPEDNDENQD (SEQ ID NO: 7);
QDNYDEPEDNDENQD (SEQ ID NO: 8);
EDNYDEPEDNDENQD (SEQ ID NO: 9);
DNNYDEQDENEQPED (SEQ ID NO: 10);
QENDYDEPEVNDENQD (SEQ ID NO: 11);
DEQENDYDEPEVNDENQD (SEQ ID NO: 12); and
DEQENDYDEPEVNDENQDYDE (SEQ ID NO: 13).

(13) Aspect 13

An intracellular-stabilizer peptide that satisfies all of the conditions listed below.

- The intracellular-stabilizer peptide consists of 10 to 39 amino acids and preferably consists of 14 to 25 amino acids.
- All amino acids constituting the intracellular-stabilizer peptide are acidic amino acids.

(Antigen-binding peptide)

(Structure)

A typical antibody structural unit is known to include a tetramer. Each tetramer is constituted by two identical pairs of polypeptide chains, each pair having one light chain (e.g., approximately 25 kDa) and one heavy chain (e.g., approximately 50 kDa to 70 kDa). The amino-terminal portion of each chain has a variable region of approximately 100 or more amino acids, and the variable region is primarily responsible for antigen (target molecule) recognition. The carboxy-terminal portion of each chain defines a constant region primarily responsible for effector function. Light chains are classified as either kappa or lambda. Heavy chains are classified as gamma, mu, alpha, delta, or epsilon, which in turn define the isotypes IgG, IgM, IgA, IgD and IgE, respectively, of the antibody. Within the light chain and the heavy chain, the variable region and the variable region are linked to each other by a J region of approximately 12 or more amino acids, and the heavy chain also includes a D region of approximately 10 more amino acids. The variable regions of each light/heavy chain pair form an antibody binding site. These chains all represent the same general structure of relatively conserved framework regions (FRs) linked to one another via three hypervariable regions (also referred to as complementarity determining regions, or CDRs). CDRs derived from the two chains of each pair are arranged by framework regions, so that binding to a specific epitope is made possible.
The term "antigen-binding peptide" refers to a peptide which (i), in a given monoclonal antibody or in a peptide capable of specifically binding to an antigen (a target molecule) similarly as a monoclonal antibody, contributes to binding to the antigen and (ii) has all or part of a region of the antibody which region has an antigen binding capacity. In the present invention, the "antigen-binding peptide", for example, contains at least one of heavy chain CDR1, heavy chain CDR2, heavy chain CDR3, light chain CDR1, light chain CDR2, and light chain CDR3 of a given monoclonal antibody. It is preferable that the antigen-binding peptide contains at least 2, at least 3, at least 4, at least 5, or all of heavy chain CDR1, heavy chain CDR2, heavy chain CDR3, light chain CDR1, light chain CDR2, and light chain CDR3.
A monoclonal antibody from which the antigen-binding peptide of the present invention is produced may be a natural antibody or an antibody which is produced by genetic recombinant technology. The natural antibody is not limited to any particular one, and can be derived from various species of organism such as a human, a mouse, a rat, a monkey, a goat, a rabbit, a camel, a llama, a cow, and a chicken. The antibody produced by the genetic recombinant technology is not limited to any particular one, and can be, for example, (i) a synthesized antibody produced from a natural antibody, (ii) a recombinant antibody, or (iii) a mutated antibody. Examples of the antibody also include antibodies obtained by subjecting antibodies already produced by genetic recombinant technology to modification that is similar to the aforementioned genetic modification of natural antibodies. The antigen-binding peptide of the present invention is a peptide which includes the full length or part of $F(ab')_2$, F(ab'), Fab', Fab, Fv (variable fragment of antibody), scFv, dsFv (disulphide stabilized Fv), dAb (single domain antibody), a diabody, a minibody, or VHH derived from any of the above antibodies. Examples of the "peptide capable of specifically binding to an antigen similarly as a monoclonal antibody" include: a peptide aptamer having high binding specificity for an antigen; a binding domain of a protein (e.g., fibronectin) derived from a living organism having binding specificity for a target molecule; and the like.

[Additional functional peptide]

The fusion protein of the present invention may further contain one or more functional peptides other than the intracellular-stabilizer peptide and the antigen-binding peptide. Examples of such a functional peptide include a purification tag peptide, a detection peptide, a degradation promoting peptide (e.g., an HSC70 binding peptide, an XIAP RING domain peptide), a Neh2 domain peptide (a peptide derived from Nrf2), a stress-responsive degradation peptide such as an oxygen-dependent degradation domain (a peptide derived from Hif1$\alpha$), a drug-binding peptide, and the like.

The purify tag peptide is not limited to any particular one, and can be a purification tag peptide containing acidic amino acids in a proportion of 15% or more, such as an HA tag sequence (YPYDVPDYA (SEQ ID NO: 14)) and a PA tag sequence (GVAMPGAEDDVV (SEQ ID NO: 15)). In a case where the fusion protein contains the tag sequence containing acidic amino acids in a proportion of 15% or more, a further increase in negative charge of the fusion protein is achieved also at low pH. It is more preferable that the purification tag peptide contain acidic amino acids in a proportion of 20% or more.

The detection peptide is not limited to any particular one, and can be, for example, a fluorescent protein, an enzyme which emits light or undergoes a change in color through a reaction, or the like. The fluorescent protein can be, but is not limited to, BFP, EBFP, CFP, ECFP, Cypet, AmCyan1, GFP, EGFP, YFP, Venus, mKO, mOrange, RFP, DsRed, tdTomato, mcherry, mStrawberry, Azalea, mPlum, mAG, Kaede, Dronpa, Keima, KikG, KikGR, UnaG or the like. The enzyme which emits light or undergoes a change in color through a reaction can be, but is not limited to, peroxidase, alkaline phosphatase, $\beta$-D-galactosidase, glucose oxidase, glucose-6-phosphate dehydrogenase, alcohol dehydrogenase, malate dehydrogenase, penicillinase, catalase, apoglucose oxidase, urease, luciferase, acetylcholinesterase, or the like.

[Structure of fusion protein]

The term "fusion protein" refers to a protein which has at least two different types of peptides that are linked to each other by a covalent bond, directly or via a linker. The linker is not limited to any particular one. In order to impart flexibility, the linker preferably mainly contains, for example, an amino acid having a low-molecular side chain, such as glycine, alanine, and serine. It is preferable that 80%, 90%, or more of the linker sequence contain a glycine residue, an alanine residue, or a serine residue, and it is particularly preferable that 80%, 90%, or more of the linker sequence contain a glycine residue or a serine residue.

The intracellular-stabilizer peptide and the antigen-binding peptide contained in the fusion protein may be linked to each other in any order. The intracellular-stabilizer peptide and the antigen-binding peptide may be arranged in this order from the N-terminal side of the fusion protein, or the antigen-binding peptide and the intracellular-stabilizer peptide may be arranged in this order from the N-terminal side of the fusion protein. Further, the intracellular-stabilizer peptide and the antigen-binding peptide may be arranged such that a direction of the N-terminal to the C-terminal of the fusion protein is opposite to a direction of the N-terminal to the C-terminal of the intracellular-stabilizer peptide and/or the antigen-binding peptide themselves. Further, a plurality of intracellular-stabilizer peptides (which may be a plurality of intracellular-stabilizer peptides of the same type or may be a combination of intracellular-stabilizer peptides of different types) may be arranged such that each of the plurality of intracellular-stabilizer peptides is respectively provided on both ends of the antigen-binding peptide.

The additional functional peptide may also be provided in any arrangement. In a case where the functional peptide is a purification tag peptide containing acidic amino acids in a proportion of 15% or more, the purification tag peptide is preferably arranged such that the antigen-binding peptide is interposed between the purification tag peptide and the intracellular-stabilizer peptide ("the intracellular-stabilizer peptide - the antigen-binding peptide - the purification tag peptide" or "the purification tag peptide - the antigen-binding peptide - the intracellular-stabilizer peptide). This arrangement allows achieving a positional balance of overall charge of the fusion protein. Accordingly, it becomes possible to maintain the fusion protein more stable in a cell. The detection peptide may also be provided in any arrangement. From the viewpoint of detectability, it is preferable that the detection peptide be located at the very N-terminal or the very C-terminal of the fusion protein.

Further, identical or different intracellular-stabilizer peptides may be arranged such that each of the intracellular-stabilizer peptides is respectively linked to both ends (i.e., the N-terminal and the C-terminal) of the antigen-binding peptide. In this case, the fusion protein has an increased negative charge also at lower pH. This enables more successfully preventing aggregation in cells.

The fusion protein of the present invention, due to containing the intracellular-stabilizer peptide, has a low isoelectric point as compared with a case in which the antigen-binding peptide is present alone. This allows the fusion protein to undergo less aggregation in a cell as compared with a case in which the antigen-binding peptide is expressed alone in a cell. Thus, the fusion protein can stably function as an intrabody.

In an example, the fusion protein of the present invention preferably has a net negative charge at pH 7.4, more preferably has a net negative charge at pH 6.6, more preferably has a net negative charge at pH 6.0, and even more preferably has a net negative charge at pH 5.0.

The number of amino acids constituting the "fusion protein" is not particularly limited, and is, for example, 1000 or less, preferably 600 or less, more preferably 550 or less. The number of amino acids constituting the "fusion protein" is not particularly limited, and is, for example, 100 or more, 250 or more, or 300 or more.

<2. Polynucleotide>

A polynucleotide of the present invention encodes the fusion protein of the present invention. The term "polynucleotide" may refer to any of a DNA molecule, an RNA molecule, and a hybrid molecule of DNA and RNA. The term "polynucleotide" may refer to a double-stranded polynucleotide or a single-stranded polynucleotide.

The polynucleotide of the present invention can be prepared by a well-known genetic engineering technique, chemical synthesis method, or the like. A specific base sequence of the polynucleotide of the present invention can be easily designed by a person skilled in the art from an amino acid sequence of an intended fusion protein and with reference to, for example, the codon table.

Further, the polynucleotide of the present invention may contain: a regulatory sequence such as a promoter (SV40 promoter, MMTV-LTR promoter, EF1$\alpha$ promoter, CMV promoter, or the like), an enhancer, a ribosome binding site, a splice signal, and a terminator; a selective marker sequence; and the like, as necessary.

Further, by adding, as necessary, a signal peptide sequence to be localized in a nucleus, a mitochondria, or an endoplasmic reticulum or immediately below a plasma membrane, it is possible to localize the fusion protein of the present invention and accordingly control an intracellular location where the fusion protein functions as an intrabody.

The polynucleotide of the present invention may be integrated into a vector. The term "vector" refers to a vehicle for causing a desired polynucleotide to be introduced into a host cell and expressed in the host cell. Examples of the vector include: a viral vector; and a nonviral vector such as a plasmid vector, a bacteria vector, a phage vector, a phagemid vector, and a cosmid vector. Examples of the viral vector include adenovirus, adeno-associated virus (AAV), retrovirus (RSV, MMTV, MOMLV, and the like), lentivirus, Sendai virus, herpes simplex virus, and the like. For introduction of the polynucleotide of the present invention into a cell *in vivo,* it is preferable to use a viral vector. For introduction of the polynucleotide of the present invention into a cell *in vitro,* either one of a viral vector and a nonviral vector can be used.

The vector may contain, in addition to DNA to be expressed, for example, a regulatory sequence such as a promoter (SV40 promoter, MMTV-LTR promoter, EF1$\alpha$ promoter, CMV promoter, and the like), an enhancer, a ribosome binding site, a splice signal, a terminator, and the like; and, as necessary, a selective marker sequence (ampicillin-resistant gene, kanamycin-resistant gene, streptomycin-resistant gene, chloramphenicol-resistant gene, and the like) and the like. Each promoter may be a constitutive promoter or an inducible promoter.

Construction of an expression vector can be carried out by, for example, a well-known genetic engineering technique.

<3. Usage>

[Pharmaceutical use]

(Pharmaceutical composition)

The present invention also provides a pharmaceutical composition containing the above-described fusion protein, polynucleotide, or vector.

The pharmaceutical composition of the present invention may further contain a component other than the above-described fusion protein, polynucleotide, or vector. The component other than the above-described fusion protein, polynucleotide, or vector is not limited to any particular one, and can be, for example, a pharmaceutically acceptable carrier, lubricant, preservative, stabilizer, wetting agent, emulsifier, osmotic pressure controlling salt, buffer agent, stabilizer, preservative, excipient, antioxidant, viscosity modifier, coloring agent, flavoring agent, sweetener, or the like. In a case where the pharmaceutical composition is provided as an aqueous solution, pure water (sterilized water), physiological saline, phosphate buffered saline, or the like can be used as a carrier. In a case where the pharmaceutical composition is provided as an appropriate solution other than the above, an organic ester (e.g., glycol, glycerol, olive oil, or the like) that can be introduced into a living organism can be used as a carrier.

The pharmaceutical composition may be contained in a container, a package, a dispenser, or the like together with an instruction manual.

(Use in treatment and prevention of disease)

The above-described pharmaceutical composition may be used, for example, for treatment and/or prevention of a disease associated with an antigen.

As an aspect of the pharmaceutical composition of the present invention, the polynucleotide or the vector of the present invention is introduced into a target cell, and the fusion protein of the present invention is expressed in the target cell so that a function of an antigen associated with a disease is controlled (i.e., suppressed or activated, preferably suppressed) by the antigen-binding peptide. This enables treatment and/or prevention of the disease.

As another aspect of the pharmaceutical composition of the present invention, a mechanism for transporting an antibody from an extracellular environment into a cell is used to cause the fusion protein of the present invention to enter a target cell and function as an intrabody. This enables treatment and/or prevention of a disease.

As another aspect of the pharmaceutical composition of the present invention, in a mechanism (recycling mechanism) in which an antibody moves from an extracellular environment into a cell and then is transported into the extracellular environment again, stability of the fusion protein of the present invention in a target cell is improved so that the fusion protein functions as an antibody inside and/or outside the cell. This enables treatment and/or prevention of a disease.

As another aspect of the pharmaceutical composition of the present invention, a cell which is a collected cell treated so as to be capable of expressing the fusion protein of the present invention and in which the fusion protein of the present invention is expressed is administered to a target. This enables treatment and/or prevention of a disease. In the above aspect, the collected cell may be a homogeneous syngeneic cell (an autologous cell) that is collected from an individual to which the cell is to be administered or may be a heterogeneous syngeneic cell (a heterologous cell) that is collected from an individual different from the individual to which the cell is to be administered.

In any aspect, a localization signal for localization may be added so that the fusion protein of the present invention is localized in a specific organelle when the fusion protein is expressed in a cell.

An aspect of the term "treatment" encompasses reduction or alleviation, delaying of progress, treatment, and the like of at least one symptom associated with a target disease. An aspect of the term "prevention" encompasses prevention of development and the like of at least one symptom associated with a target disease.

A living organism which is a subject of treatment and/or prevention can be, for example, a human or a non-human animal, more specifically, a vertebrate such as fish, a bird, and a mammal. The mammal can be a laboratory animal such as a mouse, a rat, a rabbit, a guinea pig, and a primate other than a human; a pet animal such as a dog and a cat; a farm animal such as a pig, a cow, a goat, a sheep, and a horse; or a human.

Exemplary diseases which are treated or prevented include cancers, tumors, nervous system diseases (central nervous system diseases, peripheral nervous system diseases), infectious (viral infection, bacterial infection, and the like) diseases, autoimmune diseases or allergy diseases, inflammatory diseases, and the like.

Exemplary cancers or tumors include, but are not limited to, lingual cancer, gingival cancer, malignant lymphoma, malignant melanoma (melanoma), maxillary cancer, nasal cancer, nasal cavity cancer, laryngeal cancer, pharyngeal cancer, glioma, myeloma, glioma, neuroblastoma, papillary carcinoma of thyroid, follicular carcinoma of thyroid, medullary carcinoma of thyroid, primary pulmonary carcinoma, squamous cell carcinoma, adenocarcinoma, alveolar carcinoma, large cell undifferentiated carcinoma, small cell undifferentiated carcinoma, carcinoid, testicular tumor, prostatic cancer, breast cancer (e.g., papillary adenocarcinoma, comedocarcinoma, mucous tumor, medullary carcinoma, lobular carcinoma, scirrhous carcinosarcoma, metastatic tumor), mammary Paget disease, mammary sarcoma, bone tumor, thyroid gland cancer, gastric cancer, liver cancer, acute myelocytic leukemia, acute promyelocytic leukemia, acute myelomonocytic leukemia, acute monocytic leukemia, acute lymphocytic leukemia, acute undifferentiated leukemia, chronic myeloid leukemia, chronic lymphocytic leukemia, adult T cell leukemia, malignant lymphoma (e.g., lymphosarcoma, reticulum cell sarcoma, Hodgkin disease or the like), multiple myeloma, primary macroglobulinemia, infantile leukemia, esophageal carcinoma, gastric cancer, gastrocolic leiomyosarcoma, gastrointestinal malignant lymphoma, pancreas-gall bladder cancer, duodenal cancer, large bowel cancer, primary cancer of liver (e.g., hepatocellular carcinoma, bile duct cancer or the like), hepatoblastoma, uterine intraepithelial carcinoma, cervical squamous cell carcinoma, adenocarcinoma of uterus, adenosquamous carcinoma of uterus, uterine body adenocarcinoma, uterine sarcoma, uterine carcinosarcoma, invasive hydatidiform mole of uterus, malignant chorioepithelioma of uterus, uterine malignant melanoma, ovarian cancer, mesodermal mixed tumor, renal carcinoma, renal pelvic transitional cell carcinoma, ureteral transitional cell carcinoma, papillary carcinoma of urinary bladder, bladder transitional cell carcinoma, urethral squamous cell carcinoma, urethral adenocarcinoma, Wilms tumor, rhabdomyosarcoma, fibrosarcoma, osteosarcoma, chondrosarcoma, synovial sarcoma, myxosarcoma, liposarcoma, Ewing sarcoma, skin squamous cell carcinoma, skin basal cell carcinoma, skin Bowen disease, skin Paget disease, skin malignant melanoma, malignant mesothelioma, metastatic adenocarcinoma, metastatic squamous cell carcinoma, metastatic sarcoma, mesothelioma (e.g., pleural mesothelioma, peritoneal mesothelioma, pericardial mesothelioma or the like) and the like.

Exemplary neurodegenerative diseases include, but are not limited to, Parkinson's disease, Alzheimer's disease, Huntington's disease, prion disease, frontotemporal dementia, Amyotrophic Lateral Sclerosis (ALS), Spinalbulbar Muscular Atrophy (SBMA or Kennedy's Disease), Dentatorubropallidoluysian Atrophy (DRPLA), Spinocerebellar Ataxia (e.g., SCA-1 through SCA-7), dementia, schizophrenia, depression, manic-depressive psychosis, neurosis, psychosomatic disorder, cerebral infarction, multiple sclerosis, progressive supranuclear paralysis, multiple system atrophy, spinocerebellar degeneration, cerebellar degeneration, abnormality in cerebral metabolism, abnormality in cerebral circulation, autonomic imbalance, various abnormalities in the endocrine system associated with the central nervous system, sleep disorder, neurological symptoms (nausea, vomiting, dry mouth, loss of appetite, dizziness, or the like), dyskinesia, learning disability, and the like.

Exemplary infectious diseases include, but are not limited to, diseases caused by infection with a pathogenic virus such as human immunodeficiency virus (HIV), human T cell leukemia virus (e.g., HTLV-I), hepatitis virus (e.g., hepatitis A, B, C, D, or E virus), influenza virus, herpes simplex virus, West Nile fever virus, human papillomavirus, encephalitis virus, or Ebola virus; diseases caused by infection with pathogenic bacteria such as chlamydia, mycobacteria, or Legionella; diseases caused by infection with a pathogenic yeast such as aspergillus or Candida; diseases caused by infection by a pathogenic protozoan such as malarial parasites or *Trypanosoma* parasites; and the like.

A route and method of administration are not particularly limited, and can be selected as appropriate in accordance with a target disease. The pharmaceutical composition may be administered to an affected site directly or indirectly. The route and method of administration may be administration of cells in which the fusion protein of the present invention has been expressed. In an example, the route of administration may be oral, intravenous, intramuscular, subcutaneous, intratumoral, rectal, intraarterial, intraportal, intraventricular, transmucosal, transdermal, intranasal, intraperitoneal, intrapulmonary, intrauterine, and the like routes. Topical administration, a method using a gene gun, and the like may also be employed.

A dose and a frequency of administration of the pharmaceutical composition may be selected appropriately according to the degree of a symptom, the age, gender, and body weight of the patient, the administration form, the specific type of disease, and the like.

[Use as research tool]

(Research reagent)

The present invention also provides a research reagent containing the above-described polynucleotide or vector. The research reagent may further contain a component other than the above-described polynucleotide or vector. The component other than the above-described polynucleotide or vector may be one described in the above section "(Pharmaceutical composition)".

The research reagent may be contained in a container, a package, a dispenser, or the like together with an instruction manual.

[Usage as research tool]

The fusion protein of the present invention can be used for analysis of a function of an antigen (e.g., a protein, a polypeptide, or the like) in a cell. In an example, an antigen-binding peptide for the antigen whose function is to be analyzed is intracellularly expressed as the fusion protein of the present invention, so that the function of the antigen is controlled (preferably, inhibited) to enable the analysis of the function of the antigen.

A target cell is not limited to any particular one. Examples of the target cell include a human cell and a non-human animal cell, and more specific examples of the target cell include a vertebrate cell such as a fish cell, an avian cell, and a mammalian cell. Examples of the mammalian cell include a cell of: a laboratory animal such as a mouse, a rat, a rabbit, a guinea pig, and a primate other than a human; a pet animal such as a dog and a cat; a farm animal such as a pig, a cow, a goat, a sheep, and a horse; or a human. The cell may be a cultured cell or an *in vivo* cell (an undissociated cell in a living organism). Preferable examples of the cell encompass a cultured cell of a human, an *in vivo* cell of a human, a cultured cell of a non-human disease-model animal, and an *in vivo* cell of a non-human disease-model animal.

In a case of introducing a viral vector into a target cell, the introduction can be carried out by a method such as suspension of the viral vector in a cell culture medium. In a case of introducing a nonviral vector into a target cell, the introduction can be carried out by a method such as electroporation method, microinjection method, lipofection method, calcium phosphate method, or DEAE dextran method.

An indicator of an outcome of inhibition of a function of an antigen can be, for example, a phenotype change manifested in a cell, a tissue, or an individual. Analysis of a function of an antigen can be carried out in accordance with these indicators. Examples of an index of a change in a cell include a phenotype change of a cell, for example, a quantitative and/or qualitative change of a produced substance, a change in proliferative activity, a change in cell count, a change in morphology, a change in characteristics, apoptosis induction, and the like. As a produced substance, it is possible to use a secretory protein, a surface antigen, an intracellular protein, mRNA, and the like. As a change in morphology, it is possible to use a change in formation of a cell process and/or the number of cell processes, a change in oblateness, a change in extensibility/aspect ratio, a change in cell size, a change in internal structure, a change in atypia/uniformity and cell density of a cell population, and the like. These changes in morphology can be confirmed by observation under a microscope. As a change in characteristics, it is possible to use changes in anchorage dependency, cytokine-dependent responsiveness, hormone dependency, drug resistance, cell motility, cell migratory activity, pulsatility, an intracellular substance, and the like. Examples of cell motility include cell infiltration activity and cell migratory activity. As a change in the intracellular substance, it is possible to use enzyme activity, amount of mRNA, amount of an intracellular messenger such as $Ca^{2+}$ or cAMP, amount of an intracellular protein, and the like. As a tissue-related indicator, a functional change according to the tissue to be used can serve as a detection indicator. As a living organism-related indicator, a change in tissue weight, a change in a blood system (e.g., a change in blood cell count), a change in amount of protein, a change in enzyme activity, a change in amount of electrolyte, a change in a circulatory system (e.g., a change in blood pressure or heart rate), and the like can be used.

A method for measuring these detection indicators is not particularly limited, and it is possible to use light absorbance, light emission, color, fluorescence, radioactivity, fluorescence polarization degree, surface plasmon resonance signal, time-resolved fluorescence, mass, absorption spectrum, light scattering, fluorescence resonance energy transfer, and the like. These measurement methods are well-known to a person skilled in the art, and can be selected as appropriate in accordance with a purpose. For example, absorption spectrum can be measured by a generally used photometer, plate reader, or the like, light emission can be measured by a luminometer or the like, and fluorescence can be measured by a fluorometer or the like. Mass can be measured by a mass spectrometer. Radioactivity can be measured using a measurement device such as a gamma counter depending on the type of radiation. Fluorescence polarization degree can be measured using BEACON (Takara Shuzo Co., Ltd.). Surface plasmon resonance signal can be measured using BIACORE. Time-resolved fluorescence, fluorescence resonance energy transfer, and the like can be measured using ARVO or the like. Further, a flow cytometer and the like can be used for measurement. These methods of measurement can be used in such a manner that two or more types of detection indicators are measured by one measurement method, and if convenient, two or more types of measurements can be performed simultaneously and/or continuously to enable measuring a greater number of detection indicators. For example, fluorescence and fluorescence resonance energy transfer can be simultaneously measured using a fluorometer.

The fusion protein of the present invention can be used for observation of a behavior of an antigen (e.g., a protein, a polypeptide, or the like) in a cell. In an example, the observation of the behavior of the antigen can be carried out in such a manner that (i) an antigen-binding peptide for the antigen whose behavior is to be observed is intracellularly expressed as the fusion protein of the present invention and (ii) a reaction between the antigen and the fusion protein is detected. The observation of the behavior of the antigen encompasses observation of expression of the antigen, localization of the antigen, and the like at a certain point in time or over time.

The detection of the reaction between the antigen and the fusion protein can be carried out, for example, by using fluorescence, light emission, color, or the like of a detection peptide contained in the fusion protein.

<4. Method for intracellular expression of antigen-binding peptide>

The present invention also provides a method for intracellular expression of an antigen-binding peptide. This expression method includes a step of causing the above-described polynucleotide (i.e., a polynucleotide encoding the fusion protein of the present invention) to be expressed in a cell.

The expression may be constitutive expression or transient expression. The expression may also be inducible expression. For constitutive expression, a polynucleotide in which a promoter capable of constitutive expression is linked to an upstream gene of the fusion protein may be used. For transient expression, for example, RNA instead of DNA may be introduced into and expressed in a cell. For inducible expression, a polynucleotide in which an inducible promoter is linked to an upstream gene of the fusion protein may be used. Then, an inducing factor (e.g., IPTG or the like) may be taken into the cell when the antigen-binding peptide is expressed.

Examples of a target cell include the cells listed in the section "<3. Usage>".

In the expression method of the present invention, the antigen-binding peptide is expressed in a state where the antigen-binding peptide is fused with an intracellular-stabilizer peptide (that is, as a fusion protein). The fusion protein of the present invention, due to containing the intracellular-stabilizer peptide, has a low isoelectric point as compared with a case in which the antigen-binding peptide is present alone. This allows the fusion protein to undergo less aggregation in a cell as compared with a case in which the antigen-binding peptide is expressed alone in a cell. Thus, the fusion protein can stably function as an intrabody.

In an embodiment, the expression method of the present invention further includes a step of introducing the above-described polynucleotide into a cell. The above-described polynucleotide may be introduced in the form of a vector. Examples of a specific introduction method include the methods described in the section "<3. Usage>". As such, the present invention further provides a method for producing a cell capable of expressing an antigen-binding peptide in the cell. This production method further includes a step of introducing the above-described polynucleotide (i.e., a polynucleotide encoding the fusion protein of the present invention) or the above-described expression vector into a cell. The present invention further provides a cell capable of expressing the fusion protein of the present invention.

<5. Kit>

The present invention also provides a kit for producing the above-described polynucleotide encoding a fusion protein containing an intracellular-stabilizer peptide and an antigen-binding peptide, the kit containing a polynucleotide encoding the intracellular-stabilizer peptide.

The kit of the present invention may be a versatile kit that allows easily producing, for a desired antigen-binding peptide, a polynucleotide encoding a fusion protein.

The polynucleotide of the present invention encoding an intracellular-stabilizer peptide may be integrated into the above-described vector. Further, the polynucleotide may contain a factor (e.g., a promoter, a ribosome binding site, a terminator, and the like) necessary for protein expression, a selective marker, a restriction enzyme recognition site, or the like.

Further, the kit of the present invention may further contain at least one of a buffer, a restriction enzyme, another necessary reagent, an instrument, an instruction manual, and the like.

The following will provide Examples to more specifically describe embodiments of the present invention. As a matter of course, the present invention is not limited to Examples provided below, and details of the present invention can be realized in various manners. Further, the present invention is not limited to the embodiments described above, and it may be varied in various ways within the scope of the appended claims. Thus, an embodiment based on a combination of technical means disclosed in different embodiments is encompassed in the technical scope of the present invention. Furthermore, all of the publications and patents cited in the present specification are incorporated herein by reference in their entirety.

[Examples]

[Laboratory animals]

All experimental procedures were carried out in accordance with the guidelines of the Animal Experiment Committee of RIKEN. Mice which were used were housed on a 12 h:12 h light/dark cycle, with the dark cycle occurring from 20:00 to 8:00.

[Antibodies]

Antibodies used in this study are listed in Table S1.

[Isolation of antibody-reactive phages]

An scFv gene was isolated by the Recombinant Phage Antibody System (RPAS) (GE Healthcare). DNA sequences of scFv-A36 are deposited in the DNA database of Japan (DDBJ) under accession number AB472376.

[Construction of expression vectors]

Expression vectors constructed based on PCR using specific primers are described below.

[Production of recombinant virus vectors]

ScFv-GFPA36 and scFv-GFPM4 were produced using AAV1 serotype, and s3Flag-scFv-A36-HA and s3Flag-scFv-M4-HA were produced using AAV9 serotype. Details of AAV vector plasmids and production of the AAV vector plasmids are described below.

[Stereotaxic injection of AAV vectors]

Male wild B6 mice or male DAT-cre (+/-) mice (8 weeks old) were anesthetized with isoflurane (Escain; Mylan) via an anesthetizer (MK-A110; Muromachi) and placed into a stereotaxic frame (Stereotaxic Just for Mouse, Muromachi). AAV vectors were injected into the substantia nigra of the right hemisphere (coordinates relative to bregma in millimeters, AP; -3.08, LR; -1.25, DV; -4.5).

[Purification of recombinant scFv antibodies]

Recombinant antibody scFv-GFPA36 was expressed in *E. coli* strain BL21 and purified under denatured or non-denatured condition using Ni-NTA agarose chromatography. Recombinant antibody s3Flag-scFv-A36-HA was purified under non-denatured condition using anti-Flag (M2) antibody-conjugated affinity beads (Sigma) from E. *coli* strain BL21 co-transformed with pT-Trx vector expressing thioredoxin (Trx) to enhance soluble expression of foreign proteins (Yasukawa et al., 1995).

[Immunofluorescence staining]

Immunostaining was performed by a standard technique. Immunofluorescence signals were visualized with Alexa Fluor 488- or Alexa Fluor 594-labeled secondary antibodies (Invitrogen). Fluorescence-labeled preparations were imaged under a Fluoview FV1000 confocal microscope (Olympus) or a BZ-9000 fluorescence microscope (Keyence).

[Measurement of antibody affinity by ELISA]

The antibody binding affinity of GST-Syt I-C2A was measured by ELISA as described below.

[Behavior test]

Modified rotarod test was performed with previously reported rotarod test (Shiotsuki et al., 2010) with a modification. In the present study, rotarod for mice (MK-610A, Muromachi) was equipped with a large rod (9 cm in diameter) covered by anti-slip tapes (Nitoflon adhesive tapes, No. 903UL, 0.13 mm in thickness, Nitto denko).

[ScFv proteins aligned with scFv-A36]

Accession number and amino acid residues of scFv proteins aligned with scFv-A36 are listed in Table S2.

[Laboratory animals]

*Dat*+ / *IRES-cre* mice (Backman et al., 2006) were purchased from Jackson laboratory. AAV virus vectors expressing scFv genes were used together with a DAT-Cre mouse line to selectively express antibody genes in dopamine neurons of substantia nigra. All mice having virus vectors used for microdialysis and behavioral tests were each a male littermate from mated heterozygotes. Balb/c, Balb/c-nu, and wild-type C57B6/J mice purchased from Charles Liver Japan were used for antibody production or experiments using lentivirus vectors and AAV vectors, and primary dopamine neuron culture.

[Isolation of antibody-reactive phages]

In scFv, the heavy- and light-chain variable regions of the antibody are fused by a glycine linker encoded in a single gene. RPAS (GE Healthcare) allows large repertoires of scFv to be displayed on the surface of M13 phages. Total RNA was isolated from mouse (Balb/c) spleen immunized with GST-Syt II-C2A (Fukuda et al., 1994; Fukuda et al., 1999). The heavy- and light-chain variable regions (VH and VL, respectively) were amplified in two separate reactions by using degenerate primers (GE Healthcare). The resultant PCR products were joined by a linker encoding a flexible 15 amino acid chain of (Gly4-Ser)3. The VH-glycine linker-VL complex (scFv) was subcloned into the *Sfi* I and *Not* I site of the pCANTAB 5E vector (GE Healthcare). Recombinant phage antibodies were generated by transformation of E. *coli* TG-1 cells with a phagemid vector containing scFv cDNA and infection of M13-KO7 helper phage. Isolation of antibody-reactive phages was performed by biopanning according to the manufacturer's instruction manual. Log-phase TG-1 cells were infected with antibody-reactive phage. Individual antibody-displayed phages from the phage library were screened by ELISA using recombinant GST-Syt II-C2A bound to the microtiter wells. The antibody-reactive phages were visualized by horseradish peroxidase (HRP)-conjugated anti-M13 antibody. Sequencing of the antibody-reactive scFv clone (termed scFv-A36) was performed by an automated DNA sequencer.

[Construction of expressing vectors]

Based on the scFv-A36 cDNA sequence, two linker primers were designed for PCR amplification in which Kozak sequence, T7 peptide, and *Bam*HI restriction enzyme sites were introduced into the 5' flanking region of A36, and *Mun*I site, hexa histidine residues, and a *Not* I site were introduced into the 3' flanking region of A36 (Table 1).

[Table 1]

| |
|---|
| Kozak (underline) - T7 peptide (bold) - *Bam*HI (broken line) linker primer (SEQ ID NO: 16):<br>Sense; 5'-GCCGCCACCATGGCTAGCATGACTGGTGGACAGCAAATGGGTGGATCCTATGCGGCCCAGCCGGCCAGGGCC-3' |
| *Mun*I site (double line) - hexa His (italics) - *Not* I (underline) linker primer (SEQ ID NO: 17):<br>Antisense; 5'-CGGCGGCCGCTCA*ATGATGATGATGATGATG*CAATTGCCGTTTTATT-TCCAACTTTGTCCC-3' |

Then, the PCR product was directly ligated into the pGEM-T-easy cloning vector (Promega, Tokyo, Japan), (the resultant product was named pGEM-scFv-A36; A of Fig. 8). An enhanced green fluorescent protein (EGFP) fragment was obtained from the pEGFP-C 1 vector (Clontech) by PCR using the following primers (Table 2).

[Table 2]

| |
|---|
| B*am*HI (broken line) linker primer (SEQ ID NO: 18):<br>Sense; 5'-GCGGATCCATGGTGAGCAAGGGCGAGGAG-3' |
| Bgl II (double line) linker primer (SEQ ID NO: 19):<br>Antisense; 5'-CGAGATCTTCAGAAGAACTCGTCAAGAAGGCG-3' |

Then, the digested EGFP fragment was ligated into the *Bam*HI site of pGEM-scFv-A36 to produce a vector pGEM-scFv-GFP A36. To construct pET-scFv-GFP A36, the *Bam*HI-*Not*I digested fragment of pGEM-scFv-GFPA36 was ligated into the *Bam*HI and *Not*I sites of a modified pET3a (M. Fukuda, unpublished data) *E. coli* expression vector (Novagen). For transient expression of scFv-GFPA36 driven by a cytomegalovirus (CMV) promoter in mammalian cells, the *Not*I fragment of pGEM-scFv-GFPA36 was ligated into pIRES vector (Invitrogen) to produce the vector pIRES-scFv-GFP A36. For the construction of scFv-A36 mutants, a DNA fragment including CDR1 and CDR3 regions of the heavy chain of A36 was amplified by PCR using the two following degenerate primers (Table 3). Note that N is A, C, G, or T (equimolar).

[Table 3]

| |
|---|
| *Hind*III (underline) linker primer (SEQ ID NO: 20):<br>Sense; 5'-GCAAAGCTTCTGGCTTCNNNNNNNNNNNNNNNNNNNNNNNNTGGGTGAAGCAGAGGCCTGCACAGG-3' |
| *Bst* EII (double line) linker primer (SEQ ID NO: 21):<br>Antisense; 5'-GGAGACGGTGACCGTGGTCCCTTGGCCCCANNNNNNNNNNNNNNNNNNNNNNNNAGCACAGTAATAGACGGCAGTGTCCTCAG-3' |

Then, the CDR1 and CDR3 mutant fragments were digested using *Hind*III and *Bst*EII, and were ligated into the *Hind*III and *Bst* EII site of the parental A36. From these DNA fragments, a mutant scFv-displayed phage library was generated as described above. Multiple alignment of amino acid sequences of scFvs was performed by CLUSTALW (version. 2.1: http: / / clustalw.ddbj.nig.ac.jp/index.php?lang=ja) (Thompson et al.,1994). 3×Flag tag (MDYKDHDGDYKDHDIDYKDDDDK (SEQ ID NO: 1)) and HA tag (YPYDVPDYA (SEQ ID NO: 14)) fused scFv constructs (s3Flag-scFv-HA) were synthesized and codon-optimized for the expression in mice. For transient expression in mammalian cells, the s3Flag-scFv-HA fragments were cloned into the pEF-BOS vector (Mizushima and Nagata, 1990). The s3Flag-scFv-HA fragments were cloned into the pET3a vector for expression and purification of s3Flag-scFv-HA proteins in *E. coli* BL21 cells.

[Cell culture and transfection]

Primary dopamine neuron cultures were prepared from the ventral mesencephalon of embryonic-day 13-14 male and female mouse embryos. Briefly, ventral mesencephalon was dissociated by treatment with trypsin (0.25% for 20 min at 37°C), followed by trituration with a fire-polished Pasteur pipette in neurobasal medium supplemented with 10% FBS containing DNase I. Dissociated cells ($6 \times 10^4$) were plated on poly-L-lysine (1 μg/ml)-coated glass coverslips (Fisherbrand, diameter; 12 mm) in a 24-well plates (Iwaki), and then cultured with 500 μl of neurobasal medium supplemented with B27 (Invitrogen). The cells at 7 days *in vitro* (DIV) in each well were infected with 5 μl of AAV vectors (Titer: $1 \times 10^{11}$ pg/ml) to express GFP-tagged scFv proteins, and followed by immunocytochemistry or dopamine release assay 7 days after infection. 293T and COS-7 cells obtained from RIKEN Bioresource Center Cell Bank (Tsukuba, Japan) were cultured with DMEM supplemented with 10% FBS. These cells were transfected with expression vectors by Lipofectamine2000 according to the manufacturer's instruction manual (Invitrogen) and were used for immunochemical analysis 1 day after transfection.

[Purification of recombinant scFv antibodies]

*E. coli* strain BL21 (DE3) (Novagen) was used for expression of scFv-GFPA36. The expression of scFv-GFPA36 was induced by 1 mM isopropyl-1-thio-β-D-galactopyranoside (IPTG) for 3 h at 30°C. ScFv-GFPA36 protein was solubilized with a buffer (8 M urea, 0.1 M $NaH_2PO_4$, 10 mM Tris-HCl [pH 8.0]) and purified by Ni-NTA Agarose chromatography (Qiagen) according to the manufacturer's recommendations. Under denaturing conditions, the column was washed with 4 ml of a buffer (8 M urea, 0.1 M $NaH_2PO_4$, 10 mM Tris-HCl [pH 6.3]). ScFv-GFPA36 protein was then eluted from the column with 1 ml of a buffer (8 M urea, 0.1 M $NaH_2PO_4$, 0.01 M Tris-HCl [pH 4.5]). The eluted scFv-GFPA36 protein was dialyzed with a buffer (10 mM HEPES-KOH; pH 7.2). To purify scFv-GFPA36 under non-denature condition, the cells expressing scFv-GFPA36 were re-suspended in a PBS buffer containing protease inhibitor cocktail and sonicated on ice, and then solubilized for 1h at 4°C by the addition of Triton X-100 (1%). After centrifugation, the supernatant was subjected to Ni-NTA (nickel-nitrilotriacetic acid, GE Healthcare) chromatography. Expressed scFv-GFPA36 was eluted from the column with a buffer (10 mM HEPES-KOH; pH 7.2) containing 5 mM Histidine and followed by dialysis against a buffer (10 mM HEPES-KOH; pH 7.2, 150 mM NaCl).

To purify s3Flag-scFv-A36-HA under non-denatured condition, E. *coli* BL21 (DE3) competent cells (Novagen) were co-transformed with pET3a vector (Novagen) expressing s3Flag-scFv-A36-HA and pT-Trx vector expressing thioredoxin (Trx) to enhance soluble expression of foreign proteins (Yasukawa et al., 1995). Log phase transformed cells were induced by the addition of 1 mM IPTG. The cells were collected by centrifugation 3 h after induction. The cells were re-suspended in a PBS buffer containing protease inhibitor cocktail. The cell suspension was sonicated on ice and solubilized for 1 h at 4°C by the addition of Triton X-100 (1%). After centrifugation and filtration with 0.45 μm pore filter, the supernatant was subjected to purification chromatography using anti-Flag (M2) antibody-conjugated affinity beads (Sigma). Expressed s3Flag-scFv-A36-HA was purified according to the manufacturer's recommendations. s3Flag-scFv-A36-HA was eluted from the column with a buffer (50mM Tris-HCl, pH 7.4, 150 mM NaCl) containing 3 × Flag peptide (1 mg/ml) and dialyzed against a buffer (20 mM Hepes-KOH, pH 7.2, 50 mM NaCl).

[Measurement of antibody affinity by ELISA]

Purified GST-Syt I-C2A (0.25 pmol) in buffer was coated in 96-well plates for 16 h at room temperature. Each wells was blocked with 5% skim milk in a PBS buffer for 2 h at room temperature and was incubated with 0.22 nM to 36 nM purified s3Flag-scFv-A36-HA. The binding was quantified by incubation with mouse anti-Flag (M2) primary antibody and HRP-conjugated anti-mouse secondary antibody. Specific binding was calculated by subtracting the binding of s3Flag-scFv-A36-HA to 0.25 pmol GST-coated wells. Nonlinear regression of the s3Flag-scFv-A36-HA binding data was performed with the Hill-Langmuir equation,

$$B = B_{max} \frac{[\text{s3F-scFv-A36-HA}]}{K_d + [\text{s3F-scFv-A36-HA}]}$$

where B is the concentration of s3Flag-scFv-A36-HA bound to the GST-Syt I-C2A, $B_{max}$ is the concentration of the total binding sites, [s3Flag-scFv-A36-HA] is the free s3Flag-scFv-A36-HA concentration, and $K_d$ is the dissociation constant.

[Immunofluorescence staining]

Immunocytochemistry and immunohistochemistry were performed by standard technique as follows. The cells were fixed with 4% PFA for 2 min at room temperature, followed by permeabilization with 0.3% Triton X-100 in PBS for 2 min at room temperature. The cells were then immediately washed three times with a blocking solution (1% BSA and 0.1% Triton X-100 in PBS), followed by incubation with the blocking solution for 1 h at room temperature and then with the primary antibodies for 2 h at room temperature. Immunofluorescence signals were visualized by incubation with Alexa Fluor 488- or Alexa Fluor 594-labeled secondary antibodies (Invitrogen). The primary antibodies and the secondary antibodies conjugated with Alexa Fluor dyes are listed in Table S1. In the experiments quantifying the percentage of the cells with intrabodies-formed aggregates, at least 100 cells in a dish per each experiment were counted for quantification of percentage of cells with aggregates. The data were obtained from three independent experiments. For immunohistochemistry, mice were fixed in 4% paraformaldehyde. Cryosections of the mice's brains (16 μm in thickness) were permeabilized with 0.3% Triton X-100 in PBS for 2 h at room temperature and incubated with primary antibodies in blocking solution (1% BSA, 0.1% Triton X-100) for 16 h at 4°C. Immunofluorescence signals were visualized with Alexa Fluor 488- or Alexa Fluor 594-labeled secondary antibodies (Invitrogen). Fluorescence-labeled preparations were imaged under a Fluoview FV1000 confocal microscope (Olympus) or a BZ-9000 fluorescence microscope (Keyence).

[Immunoblot analysis]

Flag-tagged full-length mouse Syt I-XI were prepared as described previously (Fukuda et al., 1999). Total homogenate of Flag-tagged Syt I-XI transiently expressed in COS-7 cells was subjected to 10% sodium dodecyl sulfate-polyacrylamide gel electrophoresis (SDS-PAGE) and transferred to a polyvinylidene difluoride (PVDF) membrane. Purified scFv-GFPA36 (1.6 μg/ml) was used as the primary antibody. HRP-labeled anti-T7 monoclonal antibody (1/5000 dilution, Merck Millipore) was used as the secondary antibody. Immunoreactive bands were visualized by an enhanced chemiluminescence (ECL) detection system (GE Healthcare). To ensure that equivalent amounts of Flag-tagged Syts have been loaded, the blots were reprobed with anti-Flag antibody. To investigate whether purified s3Flag-A36-HA recognized mouse Syt I/II in the brain, the mouse brain tissues were isolated and homogenized in a homogenization buffer (20 mM HEPES-KOH, pH 7.2, 150 mM NaCl, 0.5% TrintonX-100, and complete protease inhibitor cocktail; Roche) and agitated for 1h at 4°C. Total homogenate of 2.5-pg protein was subjected to western blotting using purified s3Flag-A36-HA as a primary antibody. Immunoreactive bands were visualized by ECL using anti-Flag antibody (the secondary antibody) and HRP-labeled anti-mouse antibody. In the experiments using the mouse brain injected with AAV vectors, the mouse brain tissues were isolated and homogenized in a buffer (PBS, 0.5% NP40, 1 mM 2ME, 2 mM EDTA, and complete protease inhibitor cocktail) and followed by shearing with 27-G needle syringe, and agitating for 1 h at 4°C. The total homogenate was subjected to western blotting using anti-TH, anti-Tubulin, anti-Syt I antibodies as primary antibodies.

[Immunoprecipitation]

COS-7 cells were cotransfected with pIRES-scFv-GFPA36 or pIRES-scFv-GFPM4 and pEF-BOS-Flag-Syt I, or control vector (pEF-BOS). Transfection of plasmid DNA was performed by LipofectAMINE (Invitrogen) according to the manufacturer's recommendations. Two days after transfection, the cells were scraped and homogenized in a buffer (10 mM HEPES-KOH [pH 7.2], 100 mM NaCl, 1 mM β-mercaptoethanol). The homogenate was centrifuged at 1,200 × g for 5 min at 4°C, and the supernatant was solubilized with a lysis buffer (10 mM HEPES-KOH [pH 7.2], 0.1% Triton X-100, 100 mM NaCl, 1 mM β-mercaptoethanol) for 1 h at 4°C. After centrifugation at 20,400 × g for 15 min at 4°C, the supernatant was transferred to a new tube, and Flag-Syt I or II was immunoprecipitated by anti-Flag antibody (Sigma) and protein A sepharose (GE Healthcare). After washing the beads with lysis buffer, the beads were boiled in 1 × SDS sample buffer (62.5 mM Tris-HCl [pH 6.8], 2% SDS, 2% β-mercaptoethanol, 0.001% bromophenol blue [BPB], 10% glycerol). After centrifugation at 13,000 × g for 5 min, the supernatant was used for western blot analysis. Coimmunoprecipitated scFv-GFPA36 was first detected by HRP-labeled anti-T7 antibody. Blots were reprobed with anti-Syt I antibody (Stressgen) to ensure that Flag-Syt I was precipitated. To examine the interaction of Syt I and intracellular s3Flag-scFv-A36-HA, 293T cells were cotransfected with pEF-BOS-Syt I and pEF-BOS-s3Flag-scFv-A36-HA or pEF-BOS-s3Flag-scFv-M4-HA vector using Lipofectamine2000. Twenty-eight hours after transfection, the cells were scraped, lysed in a buffer (20 mM HEPES-NaOH [pH 7.4], 150 mM NaCl, 0.5% Triton X-100, protease inhibitor (EGTA-free, Roche)) for 1 h, and followed by shearing with a 27-gauge needle syringe. The homogenate was centrifuged at 1,5000 rpm for 10 min at 4°C, and the supernatant was transferred to a new tube containing $CaCl_2$ (500 μM) and was subjected

to immunoprecipitation by anti-HA agarose (Sigma). After washing the beads with lysis buffer containing 500 μM $CaCl_2$, the beads were boiled in 1 × SDS sample buffer for 3 min. After centrifugation at 12,000 × rpm for 5 min, the supernatant was used for western blot analysis. Coimmunoprecipitated Syt I was detected by the primary anti-Syt I (Stressgen) antibody and the secondary HRP-labeled anti-mouse-IgG Light chain specific antibody. Blots were also probed with anti-Flag (M2) primary antibody (Sigma) and anti- HRP-labeled anti-mouse-IgG Light chain specific secondary antibody to ensure that scFv proteins were precipitated.

[Produce of AAV vectors]

The AAV9/3 vector plasmids contained the human synapsin I promoter (hSynIp), followed by the inverted cDNA of interest between the double loxP sequence (loxP-lox2722) derived from pAAV-DIO-eNpHR-YFP vector (addgene: #26966), a 241-bp fragment (FrgH) containing nucleotides 2519-2760 derived from rat tau 3'-UTR (Brun et al., 2003), a woodchuck hepatitis virus posttranscriptional regulatory element (WPRE), and a simian virus 40 polyadenylation signal sequence (SV40pA) between the inverted terminal repeats of the AAV3 genome. The AAV9 vp cDNA with a mutation (T466F) (Gao et al., 2004) was synthesized as described previously (Petrs-Silva et al., 2011). The recombinant AAV9/3 vectors were produced by transient transfection of HEK293 cells using the vector plasmid, an AAV3 rep and AAV9 vp expression plasmid, and the adenoviral helper plasmid pHelper (Agilent Technologies) as described previously (Li et al., 2006), resulting in the vectors, AAV9/3-hSynIp-DIO-s3Flag-scFv-A36 or -M4-HA. The recombinant viruses were purified by isolation from two sequential continuous CsCl gradients, and the viral titers were determined by qRT-PCR.

The AAV1 vector plasmids contained the hSynIp, followed by the cDNA of interest, FrgH, WPRE, and SV40pA between the inverted terminal repeats of the AAV1 genome. Recombinant AAV1 vectors were produced as described above, resulting in the vectors AAV1-hSynIp-scFvGFPA36 or M4.

[Stereotaxic injection of AAV vectors]

Male wild mice or female DAT-cre (+/-) mice (8 weeks old) were anesthetized with isoflurane (Escain; Mylan). The skull at injection site was then cut by a drill (Model 1474 Stereotaxic drill, Muromachi). AAV vectors were injected into the substantia nigra of the right hemisphere (coordinates relative to bregma in millimeters, AP; -3.08, LR; -1.25, DV; -4.5) using a 33-gauge needle and a 5-μl syringe (Model 75 RN SYR, Hamilton) controlled by an injection pump (Legato 130, Muromachi) at 0.2 μl $min^{-1}$ for 2 μl. The needle was left in place after injection for 10 min. At least 33 days after injection, the mice were used for microdialysis, behavior test, or immunochemical analysis.

[Measurement of dopamine release using primary cultured dopamine neurons]

The primary dopamine neurons 7 days after infection with AAV vectors were washed two times with 500 μl of a pre-warmed PSS-LK buffer (20 mM HEPES-NaOH, pH 7.4, 140 mM NaCl, 4.7 mM KCl, 2.5 mM $CaCl_2$, 1.2 mM $MgSO_4$, 1.2 mM $KH_2PO_4$, 11 mM glucose), and the cells were then incubated with 200 μl of a PSS-LK buffer for 5 min at 37°C. After the PSS-LK buffer was collected, the cells were stimulated with 200 μl of a PSS-HK buffer (20 mM HEPES-NaOH, pH 7.4, 85 mM NaCl, 60 mM KCl, 2.5 mM $CaCl_2$, 1.2 mM $MgSO_4$, 1.2 mM $KH_2PO_4$, 11 mM glucose) for 5 min at 37°C. Collected supernatants were immediately mixed with 10 μl of 0.1 M perchloric acid (PCA) containing 50 μM EDTA-2Na, and then sonicated for 30 sec and followed by centrifugation at 20,000 g and 0°C for 15 min. The supernatant (190 μl) was transferred to a new tube and mixed with 1.7 μl of 5 M $CH_3COONa$ to adjust the pH to approximately 3.0. After the PSS-HK buffer was collected, the cells were lysed with 200 μl of 5 mM PCA containing 2.5 μM EDTA-2Na. The samples (200 μl) were sonicated and mixed with 2.4 μl of 5 M $CH_3COONa$. All samples were then filtered through 0.45 μm polyvinylidene fluoride (PVDF) micropore filters (Millipore), and the filtrates were analyzed by high-pressure liquid chromatography (HPLC) coupled to an electrochemical detection system (ECD300, Eicom) according to a previous report (Kabayama et al., 2013). The amount of dopamine released from the primary cultured dopamine neurons exposed to highly-concentrated KCl is expressed as a percentage of the total dopamine contained in the cells.

[*In vivo* microdialysis in freely moving mice]

Mice were each anesthetized with isoflurane (Escain; Mylan) via a small-animal anesthetizer (MK-A110; Muromachi) and placed into a stereotaxic frame (Stereotaxic Just for Mouse, Muromachi). A hole was made for implantation of a steel guide cannula (AG-4; Eicom) on the striatum of the right hemisphere (coordinates relative to bregma, AP; +0.6 mm, LR; -2.0 mm, DV; -2.0 mm), and another hole was made to anchor a stabilizing screw. The guide cannula and the stabilizing screw were fixed with dental cement (Unifast3; GC). After the dental cement completely dried, a microdialysis probe (A-I-4-02; Eicom) was inserted into the striatum through the guide. The implantation of microdialysis probes was completed in 3.5 h to 4.5 h after the mice started to be anesthetized. Each mouse inserted with a microdialysis probe was placed onto a paper towel (Kimtowel, Nippon Paper Crecia) in a transparent microdialysis cage (20 cm in length × 20 cm in width × 21 cm in height) with water and food. On the next day following implantation surgery, the mouse was placed onto a new paper towel (Comfort200, Nippon Paper Crecia) in a transparent microdialysis cage without food and water. To measure basal dopamine release, collection of dialysate was conducted between 10:00 and 11:00 on the next day following the probe-implantation surgery. The microdialysis probe was continually perfused with a Ringer's solution containing 147 mM NaCl, 4.02 mM KCl, 2.25 mM $CaCl_2$ using a syringe pump (ESP-32, Eicom) at a rate of 1 μl $min^{-1}$. The dialysate was collected at 4°C automatically at 10 min intervals into a plastic microtube preloaded with 5 μl of 0.1 M $CH_3COOH$/250 μM EDTA-2Na using a refrigerated fraction collector (Refrigerated collector 820, Univentor). The collection of dialysate was conducted 16.5 h to 17.5 h after the implantation of the microdialysis probes. All dialysate samples were stored at 4°C and analyzed by HPLC on the following day.

[Biopsy of brain section]

The brains were collected immediately after decapitation, and 50-pm-thick frozen coronal sections were prepared. Circular tissue punches were collected from 12 substantia nigra sections and 8 striatum sections (4 anterior sections from coordinates, AP; +0.6 mm, and 4 posterior sections from coordinates, AP; +0.6 mm) using disposable biopsy needles (Biopsy Punch; Kai Medical) with a diameter of 1.5 mm. The samples were homogenized in 0.1 M perchloric acid containing 0.1 mM EDTA and centrifuged for 15 min at 20,000 × g at 4°C. The supernatant was then filtered through a 0.22 μm polyvinylidene fluoride (PVDF) filter (GV Durapore, Millipore), and the filtrate samples were analyzed by HPLC.

[HPLC]

The samples derived from primary cultured neurons and biopsy of brain sections were analyzed by HPLC (ECD300, Eicom) according to a previously reported protocol (Kabayama et al., 2013). The microdialysis samples were analyzed by HPLC (ECD500, Eicom) according to the manufacturer's notes.

[Behavior test]

Rotarod test with a rod (3 cm in diameter) has been generally used for motor skill learning of mice. A previous study demonstrated in a modified rotarod test for mice that a steeper learning curve was obtained by the rotarod test employing a combination of a large drum and slow rotation (9 cm in diameter and 10 rpm) (Shiotsuki et al., 2010). In the present study, rotarod for mice (MK-610A, Muromachi) was equipped with a large rod (9 cm in diameter) covered by anti-slip tapes (Nitoflon adhesive tapes, No. 903UL, 0.13 mm in thickness, Nitto denko). Prior to training sessions, habituation was performed by keeping each mouse on a stationary drum for 3 min on the first day. The habituation was repeated every day for 1 min just before the session. The rotation was set at a slow speed (5 rpm, 2.8 m/min on the surface). The mouse was placed back on the drum immediately after falling, up to 5 times in one session. The test was repeated one session a day for five consecutive days. The latency to falling was recorded manually and the total latencies on the rod before (Day 1) and after (Day 5) training were analyzed by two-way repeated measures ANOVA.

[Protein sequence analysis]

Isometric point and net charge at intracellular pH (7.4, 7.03, 6.60) were calculated from the amino-acid sequence of each intrabody including peptide tags using Protein calculator v3.4 developed by Chris Putnam ant the Scrips Research Institute (http://protcalc.sourceforge.net/).

[Statistical analysis]

All the data are representative of at least three independent experiments. The results are expressed as the mean ± SEM. The data were analyzed by two-tailed unpaired t-test, two-tailed non-repeated ANOVA followed by Newman-Keuls post hoc multiple comparison test, or two-way repeated measures ANOVA followed by bonferroni post hoc multiple comparison test with the GraphPad Prism 4.0 program (GraphPad Software). $P < 0.05$ was considered statistically significant.

<Example 1: Isolation of antibody specific binding to the C2A domain of Syt I/II>

First, an ScFv gene encoding an antibody specific for the C2A domain of Syt I/II (Syt I/II-C2A) was isolated by using a phage display system. To investigate specificity of isolated scFv-displayed phages, the C2B domain of Syt I/II (Syt I/II-C2B) was used as a negative control. Enzyme-linked immunosorbent assay (ELISA) demonstrated that one of the isolated ScFv-displayed phages (termed scFv-A36) bound to glutathione S-transferase (GST)-Syt II-C2A and did not bind to GST-Syt II-C2B or GST (A of Fig. 1). From cDNA sequence, an open reading frame (361 amino acids; accession no. AB472376) which contained 723 nucleotides and in which a heavy chain and a light chain were fused with each other by a glycine linker was revealed (B of Fig. 1, upper panel). To visualize and to purify expressed scFv-A36, T7, EGFP, and His tags were fused to the amino terminal or the carboxyl terminal of A36 (termed scFv-GFPA36; B of Fig. 1, lower panel). ScFv-GFPA36 was expressed in E. *coli* and was purified through the Ni-NTA column. Western blot analysis showed that the purified scFv-GFPA36 protein recognized only full-length Syt I/II and did not recognize the other 9 isoforms exogenously expressed in COS-7 cells (C of Fig. 1). These results indicated that scFv-GFPA36 is an antibody specific for the Syt I/II-C2A domain. Amino acid sequences of three CDRs of the light-chain variable region of scFv-A36 were identical to those of scFv encoding CRA5, which is an antibody for the immunoglobulin E (IgE) receptor (accession no. BAB82458, data not shown). This suggested that CDRs of the light chain in scFv-A36 may not play a key role in the determination of binding specificity with Syt I/II. On the other hand, it is known that heavy chain CDR3 is the most hypervariable region in antibodies and has the potential to generate approximately $10^{14}$ varieties in humans (Sanz, 1991), thus contributing to the interaction of antigen (Chothia and Lesk, 1987). In fact, high diversity was found between the amino acid sequence of heavy chain CDR3 in scFv-A36 and the amino acid sequences of heavy chain CDR3 in other 4 scFvs deposited in the National Center for Biotechnology Information (NCBI) DNA database (Fig. 2). This suggested that heavy chain CDR3 of scFv-A36 might be important for binding specificity. Then, an attempt was made to construct a negative control antibody from scFv-GFPA36 by PCR-based mutagenesis. Totally 16 amino acids in heavy chain CDR1 (8 amino acids) and CDR3 (8 amino acids) of A36 were mutated with degenerate primers. In the ELISA, it was found that one of the isolated mutant ScFv phage clones (termed scFv-M4) did not bind to the GST-Syt II-C2A protein (D of Fig. 1), and nucleotide sequences in CDR1 and CDR3 of scFv-M4 were determined (Fig. 2). It was thus determined that the M4 gene was to be used as a negative control (termed scFv-GFPM4) for further functional analysis of scFv-GFPA36. Next, whether scFv-GFPA36 can be expressed in mammalian cells and bind to intracellular antibody was examined. ScFv-GFPA36 was coimmunoprecipitated with anti-Flag antibody only when both scFv-GFPA36 and Flag-Syt I were coexpressed in COS-7 cells, whereas scFv-M4 was not coimmunoprecipitated with anti-Flag antibody (E of Fig. 1). This indicated that the intracellularly expressed scFv-GFPA36 can bind to expressed Syt I.

<Example 2: Characterization of intracellular scFv-A36 *in vitro* and *in vivo*>

Because Syt I/II act as major $Ca^{2+}$ sensor for fast neurotransmitter release in the brain, the inhibitory effect of scFv-A36 on dopamine release was examined next. To express scFv-GFPA36 and scFv-GFPM4, AAV vectors were constructed (termed AAV-scFv-GFPA36 and AAV-scFv-GFPM4, respectively). Primary cultured dopamine neurons at DIV7 prepared from substantia nigra of mouse embryo were infected with the AAV vectors. The cells were fixed and analyzed 7 days after infection with AAV. Both scFv-GFPA36 and scFv-GFPM4 were diffusely expressed without aggregates in the neurons (Fig. 3, A through C; scFv-GFPA36, D through F; scFv-GFPM4). ScFv expression was also confirmed by western blot analysis (G of Fig. 3, top; scFv, middle; Syt I, bottom; Actin). Then, comparison of depolarization-induced dopamine release was made. In primary cultured cells, depolarization-induced dopamine release was extracellular $Ca^{2+}$ dependent (H of Fig. 3, right column). This $Ca^{2+}$-dependency was consistent with previous reports (Rouge-Pont et al., 1999). Depolarization-induced dopamine release in scFv-GFPA36-expressing cells was significantly lower than in scFv-GFPM4-expressing cells or non-infected control cells (H of Fig. 3). These results indicated that the intrabodies can be expressed in primary cultured neurons and scFv-GFPA36 inhibits the function of the C2A domain of Syt I/II. Next, whether these intrabodies are stably expressed in neurons of mouse brain was examined. Adult mice (P56) were unilaterally injected with AAV-scFv-GFPA36 ($1.6 \times 10^8$ vg/mouse) into the substantia nigra. Four weeks after injection, scFv-GFPA36 expression was observed in almost all dopamine neurons. However, intense aggregates were observed in some neurons in substantia nigra compacta (SNc) (A through C of Fig. 4, arrows). These fluorescence signals were not observed in non-AAV injected SNc of left hemispheres (D through F of Fig. 4). These results indicated that intrabodies expressing stably in *in vitro* cultured cells do not always express stably *in vivo.*

<Example 3: Improvement of stability of intrabodies by tagging of highly negative charged 3xFlag peptide and modestly negative charged HA peptide>

There is a positive correlation between the net negative charge of intrabodies at cytoplasmic pH 7.4 and stability of intrabodies at cytoplasm in mammalian cells (Kvam et al., 2010). For example, $V_H$H-H7 intrabody that has a high net negative charge (-5.0) at pH 7.4 is well expressed at cytoplasm with less aggregates in mammalian cells (Kvam et al., 2010). This suggests that the stable intracellular expression of intrabodies can be predicted in a case where the intrabodies have a net negative charge value of less than -5.0 at pH 7.4. In fact, scFv-GFPA36 and scFv-GFPM4 that have the same net negative charge -5.0 at pH 7.4 were stably expressed in primary cultured dopamine neurons for 7 days (Fig.

3). However, scFv-GFPA36 and scFv-GFPM4 formed intracellular aggregates in dopamine neurons of mouse brain (Fig. 4). Under the circumstances, in silico analysis was conducted in order to design stable intrabodies *in vivo.* It has been reported that the resting intracellular pH in hippocampal neurons is ~7.03-7.35 (Ruffin et al., 2014), and a previous study using pH-sensitive indicator pHluorin targeted to endosome has revealed that the local cytoplasmic surface pH of endosome is 6.73±0.08 (Mitsui et al., 2011). In view of this, a comparison was made between (i) the pI and net charge of scFv-A36 and scFv-M4 and (ii) those of other peptide tags, at cytoplasmic pH 6.60, 7.03, and 7.40, respectively (A of Fig. 5). Fusion of T7-, EGFP-, and His-tags to scFv-A36 or scFv-M4 increased the net negative charge at pH 7.4. However, the net negative charge of scFv-GFPA36 and scFvGFPM4 dramatically decreased at a lower pH 7.03 and pH 6.6. This suggested the reduced stability of scFv-GFPA36 and sFv-GFPM4 at cytoplasmic pH 7.03 and 6.6. To improve the net charge of intrabodies even at the lower pH, short peptide tags, 3×Flag tag (MDYKDHDGDYKDHDIDYKDDDDK (SEQ ID NO: 1); termed s3Flag) and HA tag were tested. These tags themselves have a strong net negative charge (s3Flag; -7.0) and a modest net negative charge (HA; -2.2), respectively. From in silico analysis, it was demonstrated that fusion of s3Flag tag and HA tag to scFv-A36/M4 proteins (termed s3Flag-scFv-HA) drastically increased their net negative charge even at pH 6.6 (A of Fig. 5). Next, the generality of the effects of s3Flag- and HA-peptide tagging on the increase in the net negative charge of intrabodies at the lower pH was investigated using other 94 scFv protein sequences (Table S2) obtained by NCBI blast search with scFv-A36 protein sequence (Fig. 6). A comparison was made between the net charges of 94 scFv proteins fused with different peptide tags at pH 7.4 (A of Fig. 6, upper panels) and pH 6.6 (A of Fig. 6, lower panels). The average of the net negative charges of scFv proteins fused with T7-, EGFP-, and His-tags was statistically increased compared with that of scFv proteins without peptide tag at pH 7.4 (upper panels), but not at pH 6.6 (lower panels). In contrast, the average of the net negative charge of scFv proteins fused with s3Flag- and HA-tags was statistically increased compared with that of scFv proteins with or without other peptide tags, both at pH 7.4 and 6.6. Proteins have a net positive charge under a pH below the pI of the proteins, or have a net negative charge under a pH above the pI of the proteins. The average of estimated pI of scFv proteins fused with s3Flag tag and HA tag was significantly lower than those of scFv proteins fused with or without T7 tag, EGFP tag, and His tag (A of Fig. 5, B of Fig. 6). These indicated that the effects of s3Flag tag and HA tag on the increase in the net negative charge of scFv proteins at pH 6.6 were caused by the decrease in the pI of the scFv proteins. Further, a correlation between amino acid identity of scFv proteins to scFv-A36 and the net charge of scFv proteins fused with s3Flag tag and HA tag at pH 7.4 was tested (C of Fig. 6). No statistical correlation was found between the amino acid identity and the net charge. This indicates that structural identity of scFv proteins to scFv-A36 itself does not affect the effect of the fusion of s3Flag tag and HA tag to other scFv proteins on the increase in the net negative charge. Taken together, these results indicated that the fusion of s3Flag- and HA-peptide tags to scFv proteins generally increases the net negative charge of the scFv proteins at cytoplasmic pH 6.6 to 7.4 by the strong net negative charge of these peptide tags and by the decrease in the pI of the scFv proteins.

Next, the stability of scFv-A36 and scFv-M4 intrabodies fused with these peptide tags at cytoplasm in mammalian cells was examined. Immunocytochemical analysis revealed that s3Flag-scFv-A36-HA and s3Flag-scFv-M4-HA were diffusely expressed with little aggregate in HeLa cells (B of Fig. 5, lower panels), whereas intracellular scFv-GFPA36 and scFv-GFPM4 formed aggregates in HeLa cells (B of Fig. 5, upper panels). The average of s3Flag-scFv-A36-HA- or s3Flag-scFv-M4-HA-expressing cells with aggregates was significantly decreased compared with that of scFv-GFPA36- or scFv-GFPM4-expressing cells with aggregates (C of Fig. 5). These results indicated that s3Flag tag and HA tag could improve stability of intrabodies in the cultured cells.

<Example 4: s3Flag-scFv-A36-HA interacts with Syt I-C2A domain and inhibits $Ca^{2+}$-dependent interaction between Syt I and Syntaxin1-SNARE domain>

Subsequently, the biochemical property of s3Flag-scFvA-36-HA construct was examined. The binding affinity of purified s3Flag-scFv-A36-HA with GST-Syt I-C2A was measured. S3Flag-scFv-A36-HA was purified from E. *coli* using anti-Flag antibody-conjugated affinity beads. ELISA experiments demonstrated that purified s3Flag-scFv-A36-HA interacted with Syt I-C2A, but not Syt I-C2B (A of Fig. 7). This specificity of s3Flag-scFv-A36-HA was consistent with the results of scFv-GFPA36 as shown in Fig. 1. ELISA experiments using different concentration of s3Flag-scFv-A36-HA demonstrated that kd value of s3Flag-scFv-A36-HA against SytI-C2A is approximately 11.97 nM (B of Fig. 7). In addition, western blot analysis revealed that s3Flag-scFv-A36-HA specifically recognized mouse brain Syt I/II (C of Fig. 7). These results indicated that s3Flag-scFv-A36-HA is a highly specific intrabody having high affinity. Next, whether intracellular s3Flag-scFv-A36-HA can bind to Syt I in cells was investigated using 293T cells. Syt I was co-immunoprecipitated with s3Flag-scFv-A36-HA, not s3Flag-scFv-M4-HA (D of Fig. 7). This indicates that intracellular s3Flag-scFv-A36-HA can bind to Syt I in mammalian cells. These raise the possibility that s3Flag-scFv-A36-HA inhibits its interaction. To test this, the cell lysate show in D of Fig. 7 was subjected to GST-pull down assay. Precipitation was carried out using purified GST-syntaxin1-SNARE in the presence of 500 μM $Ca^{2+}$, and the precipitates were analyzed by western blotting. Co-precipitates of Syt I with GST-syntaxin1-SNARE in the cells expressing s3Flag-scFv-A36-HA were decreased by approximately 46.5% compared with the cells expressing s3Flag-scFv-M4-HA (E, F of Fig. 7). This indicates that intracellular s3Flag-scFv-A36-HA interferes with the interaction between Syt I and Syntaxin I in the cells by interacting with the C2A domain of Syt I.

<Example 5: Stable intracellular expression of s3Flag-scFv-HA *in vivo*>

Next, whether s3Flag-scFv-HA is stably expressed in neurons of mouse brain was investigated. AAV9/3-hSynIp-DIO-s3Flag-scFv-A36 (or M4)-HA was injected to the substantia nigra of right hemisphere in DAT-cre mice. Immunohistochemistry revealed that s3Flag-scFv-A36-HA and s3Flag-scFv-M4-HA were stably expressed at cytoplasm of dopamine neurons in SNc and VTA of right hemispheres 33 days after AAV9/3 vectors injection (A through L of Fig. 8, arrows). These expression patterns were observed in almost all broad range of substantia nigra region (~768 μm) (A through J of Fig. 9). Further, expression of both s3Flag-scFv-A36-HA and s3Flag-scFv-M4-HA in SN was confirmed using western blot analysis (K of Fig. 9, upper panel). In striatum where striatal neurons receive long afferent inputs from mid brain dopamine neurons, expression level of s3Flag-scFv-A36-HA was higher than that of s3Flag-scFv-M4-HA (K of Fig. 9, upper panel). This suggests that s3Flag-scFv-A36-HA binds to endogenous Syt I and is transported along with Syt I from cell body to axonal terminals in striatum. In addition, stable expression of both s3Flag-scFv-A36-HA and s3Flag-scFv-M4-HA was observed 6 months after AAV9/3 injection without any neuronal damage of dopamine neurons (Fig. 10). To validate the functional activity of s3Flag-scFv-A36-HA against Syt I in dopamine neurons of SN *in vivo,* basal dopamine release in striatum of awake mice 33 days after AAV9/3 injection was measured using microdialysis. A comparison of dopamine release in striatum was made among three groups: s3Flag-scFv-A36-HA-expressing mice, s3Flag-scFv-M4-HA-expressing mice, and non-AAV-injected mice. It was found that dopamine release in striatum of s3Flag-scFv-A36-HA-expressing mice was significantly decreased compared with that of s3Flag-scFv-M4-HA-expressing mice (by 55.3%) or no AAV9/3-injected mice (by 52.7%) (B, C of Fig. 11). However, total amount of dopamine in striatum and substantia nigra was not statistically different between all three groups (D of Fig. 11). Taken together, these results indicated that s3Flag-scFv-A36-HA inhibited dopamine release which is dependent on neural activity and extracellular $Ca^{2+}$ from axonal terminal of dopamine neurons.

<Example 6: Inhibition of motor learning by intracellular expression of s3Flag-scFv-A36-HA in dopamine neurons>

Parkinson's disease (PD) is a neurodegenerative disorder that is accompanied by motor deficits including tremor, rigidity, slowness of movement, and postural instability. Most characteristic features in patients are loss of dopamine neurons mainly in SNc and a decrease in dopamine concentration in the striatum. Thus, to examine the direct role of striatum dopamine release in motor behavior, open field test and modified rotarod test were performed. AAV9/3-hSynIp-DIO-s3Flag-scFv-A36 (or M4)-HA was bilateral injected into substantia nigra of DAT-cre mice. Four weeks after AAV injection, the mice were subjected to open field test, and 5 or 6 days later, to modified rotarod test. There was no difference of total distance, speed of movement, center of time, and total rearing number between s3Flag-scFv-A36-HA- and s3Flag-scFv-M4-HA-expressing mice (A through D of Fig. 12). Next, motor learning was examined using rotarod test as reported previously (Shiotsuki et al., 2010) with a slightly modification (see the section of experimental procedures). Five days after training, s3Flag-scFv-M4-HA-expressing mice stayed on the rod for a longer time than s3Flag-scFv-A36-HA-expressing mice (E of Fig. 12, P = 0.0132). After motor behavior test, the mouse brain was analyzed by immunohistochemistry. Bilateral expression of 3Flag-scFv-M4-HA and s3Flag-scFv-A36-HA proteins in DA neurons of substantia nigra was confirmed (F of Fig. 12, arrows).

<Example 7: Evaluation of antitumor activity>

Next, anti-Kras monoclonal antibody (Y13-259; SEQ ID NO: 22) was fused with s3Flag and HA to produce s3Flag-scFv-Y13-259-HA (STAND-Y13-259; SEQ ID NO: 23) in a similar manner to the above Examples, and evaluation was made as to whether or not s3Flag-scFv-Y 13-259-HA had antitumor activity. Since DNA sequence of Y13-259 was not deposited in the database, a mouse codon-optimized sequence was designed. DNA sequence of STAND-Y13-259 is represented by SEQ ID NO: 24. DNA sequence of myc-Y13-259 is represented by SEQ ID NO: 25. DNA synthesis was outsourced to Genescript.

(1) In silico analysis

In designing STAND-Y13-259, in silico analysis was conducted, which demonstrated that fusion of s3Flag tag and HA tag drastically increased the net negative charge of scFv-Y13-259 even at pH 6.6 (A of Fig. 13).

(2) Evaluation of binding capacity of STAND-Y13-259

Next, the binding affinity between STAND-Y13-259 and GST-Kras was measured by ELISA in a manner described in the above Examples. The measurement confirmed that STAND-Y 13-259 also maintained a high Kras-binding activity (B of Fig. 13). Subsequently, MIA PaCa2 cells were infected with a vector obtained by integrating only STAND-Y13-259, myc-Y13-259 (SEQ ID: NO. 26), or scFv-Y13-259 into lentivirus, and were subjected to immunocytological staining. As a result, it was confirmed that conventional myc-Y13-259 was unstable and did not express or expressed with aggregates in the cells, whereas STAND-Y13-259 expressed stably (C of Fig. 13). Further, to confirm interaction in endogenous Kras cells, coimmunoprecipitation using anti-HA antibody was performed, which confirmed binding between endogenous Kras and STAND-Y 13-259 in MIA PaCa2 cell-derived samples infected with STAND-Y13-259 (D of Fig. 13).

(3) Evaluation of antitumor activity

Next, the viability of MIA PaCa2 cells infected using lentivirus cell vectors in a manner as described above was measured by MTS analysis 4 days after infection. The measurement indicated that the viability is significantly reduced by the expression of STAND-Y13-259 (E of Fig. 13). Subsequently, evaluation was made as to whether the intracellular expression of STAND-Y13-259 could suppress cancer development in a mouse xenotransplantation model in which pancreas cancer cells had been subcutaneously transplanted. The above-described STAND-Y13-259-expressing lentivirus vector was administered to subcutaneous tumor in nude mice once a week for 4 weeks, and it was thus revealed that the lentivirus vector strongly inhibits cancer (F, G of Fig. 13). Expression of STAND-Y13-259 in tumor excised 25 days after initial infection was also confirmed (H of Fig. 13).

<Example 8: Evaluation of other peptide tags>

To evaluate the performance of other peptide tags, DE2.0 tag and DE5.0 tag were produced, and the stabilization effect of DE2.0 tag and DE5.0 tag was studied. In a similar manner to Example 7, the following fusion proteins containing DE2.0 tag (DEQENDYDEPEVNDENQDYDE (SEQ ID NO: 13)) were produced: DE2.0-Y13-259 (SEQ ID NO: 27) obtained by fusing DE2.0 tag with the N-terminal of an anti-Kras monoclonal antibody-derived peptide (Y13-259; SEQ ID NO: 22); DE2.0-Y13-259-HA (SEQ ID NO: 28) obtained by further fusing HA peptide with the C-terminal of DE2.0-Y13-259; DE2.0-Y13-259-PA (SEQ ID NO: 29) obtained by further fusing PA peptide with the C-terminal of DE2.0-Y13-259; and protein STAND-6E-LYS obtained by fusing DE2.0 tag with both of the N- and C-terminals of scFV sequence (scFv-6E) of an antibody (Barkhordarian et al., 2006: Protein Engineering, Design and Selection, Volume 19, Issue 11, 1 November 2006, Pages 497-502) specifically binding to aggregated and fibrous human $\alpha$-synuclein. Heat shock cognate protein 70 (HSC70)-binding peptide (MARVKKDQAEPLHRKFERQPPG (SEQ ID NO: 31)) as a functional peptide was further fused with STAND-6E-LYS, at a position downstream of DE2.0 tag on the C-terminal side, and HA peptide was further added to a position downstream of HSC70-binding peptide. The full length of the amino acid sequence of STAND-6E-LYS thus produced is represented by SEQ ID NO: 32.

As a fusion protein containing DE5.0 tag (DDDEDEDDEDEDEDEDEDEDED (SEQ ID NO: 33)), DE5.0-6E (amino acid sequence: SEQ ID NO: 35, base sequence: SEQ ID NO: 36) was produced by fusing DE5.0 tag with the N-terminal of scFv-6E and further fusing T7 tag (SEQ ID NO: 34: MASMTGGQQMG) with the C-terminal of scFv-6E. All of the DNA encoding these fusion proteins were human codon-optimized, and were synthesized by GenScript.

Note that DNA sequences of DE2.0-Y13-259-HA and STAND-6E-LYS are represented by SEQ ID NO: 30 and SEQ ID NO: 37, respectively.

[Table 4]

DNA and Amino Acid Sequence of STAND-6E-LYS

DNA sequence: DE2.0 tag (underline), scFv-6E (italics), HSC70-binding peptide (wavy underline), HA tag (underline in italics), wherein gccaccatgg at 5' terminal is Kozak sequence and tga at 3' terminal is stop codon:

(continued)

gccaccatggatgaacaggagaacgattatgatgagccagaagtgaacgacgagaaccaggattatgatgagggatccgccgaggtg
cagctgttggagtctgggggaggcttggtacagcctggggggtccctgagactctcctgtgcagcctctggattcacctttagcagc
tatgccatgagctgggtccgccaggctccagggaaggggctggagtgggtctcatatattgctagtggtggtgatactacaaattac
gcagactccgtgaagggccggttcaccatctccagagacaattccaagaacacgctgtatctgcaaatgaacagcctgagagccgag
gacacggccgtatattactgtgcgaaaggtgcttctgcttttgactactggggccagggaaccctggtcaccgtctcgagcggtgga
ggcggttcaggcggaggtggcagcggcggtggcgggtcgacggacatccagatgacccagtctccatcctccctgtctgcatctgta
ggagacagagtcaccatcacttgccgggcaagtcagagcattagcagctatttaaattggtatcagcagaaaccagggaaagcccct
aagctcctgatctatgctgcatcctatttgcaaagtggggtcccatcaaggttcagtggcagtggatctgggacagatttcactctc
accatcagcagtctgcaacctgaagattttgcaacttactactgtcaacagagttctaatgatccttatacgttcggccaagggacc
aaggtggaaatcaaacggagatctatggatgaacaggagaacgattatgatgagccagaagtgaacgacgagaaccaggattatgat
gagatggccagagtgaagaaggaccaggctgagcctctccatagaaagtttgagagacagccaccagggtacccttacgatgtgcct
gactacgcttga

Amino acid sequence

MDEQENDYDEPEVNDENQDYDEGSAEVQLLESGGGLVQPGGSLRLSCAASGFTFSSYAMSWVRQAPGKGLEWVSYIASGGDTTNYAD
SVKGRFTISRDNSKNTLYLQMNSLRAEDTAVYYCAKGASAFDYWGQGTLVTVSSGGGGSGGGGSGGGGSTDIQMTQSPSSLSASVGD
RVTITCRASQSISSYLNWYQQKPGKAPKLLIYAASYLQSGVPSRFSGSGSGTDFTLTISSLQPEDFATYYCQQSSNDPYTFGQGTKV
EIKRRSMDEQENDYDEPEVNDENQDYDEMARVKKDQAEPLHRKFERQPPGYPYDVPDYA

[Table 5]

DNA and amino acid sequence of DE5.0-6E

DE5.0-6E

DNA sequence: DE5.0 tag (underline), scTv-6E (italics), T7 tag (wavy underline)

atggatgacgacgaggacgaagacgacgaagatgaggatgaagatgaagacgaggatgaggacgaggacggatctgccgaagtccag
cttctcgagtctggcggcgggttggtgcagcctggaggatccctgagattatcctgtgcagctagcgggttcactttctcgtcctat
gccatgtcatgggttcgccaagctcccggcaaagggttggagtgggtgagctacattgccagtggaggcgatactaccaattacgcg
gacagtgtgaagggtaggtttaccattagccgggataacagcaagaacactctctacctgcagatgaactctctgagagcggaggat
acagccgtgtactattgcgcaaaaggcgcatccgctttcgactattggggtcaaggcactctcgtaaccgtgtctagtggcggaggt
ggttcagggggcggcggatcaggagggggaggcagtacagatatccagatgacacagagtccctcctctctgtccgcttctgtcggc
gatcgagtcacgattacgtgtcgtgctagtcagtctatcagctcatacctcaattggtaccagcagaagcctggaaaagcccccaaa
ctgctgatctatgccgcctcctacctacagagcggtgttccaagccgcttttcaggcagcgggtctgggacagatttcactctgacc
atatcctcgcttcagccagaggactttgcaacctactattgccagcagtcctcaaatgacccgtatacctttggacaagggaccaag
gtggaaatcaagcggaggagcatggccagcatgacaggtgggcaacaaatgggctga

Amino acid sequence

MDDDEDEDDEDEDEDEDEDEDEDGSAEVQLLESGGGLVQPGGSLRLSCAASGFTFSSYAMSWVRQAPGKGLEWVSYIASGGDTTNYA
DSVKGRFTISRDNSKNTLYLQMNSLRAEDTAVYYCAKGASAFDYWGQGTLVTVSSGGGGSGGGGSGGGGSTDIQMTQSPSSLSASVG
DRVTITCRASQSISSYLNWYQQKPGKAPKLLIYAASYLQSGVPSRFSGSGSGTDFTLTISSLQPEDFATYYCQQSSNDPYTFGQGTK
VEIKRRSMASMTGGQQMG

From in silico analysis, it was found that a drastic increase in the net negative charge of scFv-Y13-259 is caused also by fusion of only DE2.0 tag (A of Fig. 13). It was also found that additional fusion of HA or PA caused further increase in the net negative charge (A of Fig. 13). From in silico analysis similarly conducted with respect to scFv-6E, it was demonstrated that the net negative charge of scFv-6E was drastically increased by fusion with DE2.0 tag, DE2.0 tag, and HA tag or by fusion with DE5.0 tag (Fig. 15).

DE2.0-Y13-259-HA-encoding DNA was introduced into MIA PaCa2 cells with use of a lentivirus vector. The introduction confirmed expression of DE2.0-Y13-259-HA in the cells without aggregates, and thus confirmed stronger binding of DE2.0-Y13-259-HA to endogenous Kras in the cells than STAND-Y13-259 (A of Fig. 14). The cancer development inhibitory effect of the DE2.0-Y13-259-HA-expressing lentivirus vector was evaluated using a mouse xenotransplantation model in which pancreas cancer cells had been subcutaneously transplanted, and it was thus revealed that the lentivirus vector strongly inhibited cancer (B of Fig. 14). These results indicated that the present invention is extremely effective for the development of a peptide tag capable of causing an antibody to be stably expressed in cells.

STAND-6E-LYS-encoding DNA and DE5.0-6E-encoding DNA were cloned into pEF-BOS vector, which is a mammalian expression vector, to produce expression vectors (STAND-6E-LYS expression vector; pEF-BOS-STAND-6E-LYS, DE5.0-6E expression vector; pEF-BOS-DE5.0-6E). STAND-6E-LYS expression vector and DE5.0-6E expression vector were introduced into HeLa cells with use of Lipofectamin2000, cultured for 18 hours, and then fixed with 4% PFA. STAND-6E-LYS and DE5.0-6E were subjected to staining with HA antibody and T7 antibody, respectively. It was confirmed that both STAND-6E-LYS and DE5.0-6E were expressed in the cells without aggregates (Fig. 16).

Subsequently, the effect of STAND-6E-LYS on α-synuclein aggregation was examined. α-synuclein aggregation assay was performed with use of α-synuclein Fibril (Tarutani et al., 2018: Acta Neuropathol Commun. 2018 Apr 18; 6(1): 29.), which was obtained by subjecting human recombinant α-synuclein purified from E. *coli* to sonication to make human recombinant α-synuclein fibrous and aggregated. Human-derived SHSY5Y cells were used in the assay. HSC70-binding peptide integrated into STAND-6E-LYS consists of HSC70-binding motif (VKKDQ (SEQ ID NO. 38)) and HSC70-binding consensus motif (KFERQ (SEQ ID NO. 39)) of α-synuclein. It has been demonstrated *in vitro* and *in vivo* that fusion between a peptide binding to mutant huntingtin protein and a HSC70-binding peptide allows degradation of mutant huntingtin protein by chaperon-mediated autophagy (CMA) to be promoted. Provision of a peptide (including scFv) capable of specifically binding to a target molecule would thus enable CMA-dependent degradation of the target protein molecule. pEGFP-α-synuclein vector expressing a protein (EGFP-synuclein) obtained by fusing the full length of human α-synuclein with green fluorescence protein EGFP was constructed, and was introduced by gene delivery into SHSY5Y cells together with STAND-6E-LYS expression vector, with use of XtremGENE9 (Sigma). Six hours later, Fibril protein was introduced into the cells with use of MultiFectam (Promega), cultured for 3 days, and then fixed with 4% PFA. Then, STAND-6E-LYS was detected with use of anti-HA antibody, and staining was carried out using anti-phosphorylated α-synuclein antibody (Mouse, WAKO). The gene delivery and protein introduction by XtremeGENE9 and MultiFectam were performed according to the manufacturers' standard protocols. The occurrence ratio of the number of cells in which GFP-synuclein was aggregated into dots and the occurrence ratio of phosphorylated synuclein positive cells were measured to quantify aggregates caused by Fibril.

In approximately 14% of cells into which a control vector had been introduced, Fibril caused EGFP-synuclein to be localized into dots. It was found that these dots of synuclein were co-stained by anti-PSer129-synuclein antibody which detects phosphorylation of the 129th amino acid of α-synuclein, which phosphorylation is a marker of the brain of a patient with Parkinson's disease (Fig. 17). This indicates that aggregated synuclein which had entered the cells from outside caused aggregation of EGFP-synuclein which had had a normal structure capable of being expressed in the cells. On the other hand, aggregation was reduced to 2% in cells expressing STAND-6E-LYS (Fig. 18).

These results indicated that the present invention was extremely effective for causing an antigen-binding peptide to be intracellularly expressed without aggregates and thus maintaining the function of the antigen-binding peptide.

[Table 6]

Table S1

| Antibody | Application | Description | Source |
|---|---|---|---|
| Anti-T7 | WB, ICC | mouse monoclonal | Merckmillipere69522 |
| HEP-conjugated anti-T7 | WB | mouse monoclonal | Novagen 69048 |
| Anti-Flag | WB, IP, ICC, IHC | mouse monoclonal | Sigma M2 |
| Anti-Flag-heads | Purification | mouse monoclonal | Sigma F2425 |
| Anti-HA | WB, IP | mouse monoclonal | Sigma A2095 |
| Anti-HA agarose | IP | mouse monoclonal | Sigma A2095 |
| Anti-Myc | ICC | rabbit polyclonal | MBL 562 |
| Anti-GST | IP | goat polyclonal | Amersham pharmacia |
| HRP-conjugated anti-M13 | ELISA | mouse monoclonal | Amersham pharmacia |
| Anti-GFP | WB, ICC, IHC | rabbit polyclonal | MBL 598 |
| Anti-Actin | WB | rabbit polyclonal | Sigma A2066 |
| Anti-Tubulin | WB | mouse polyclonal | Sigma T9026 |
| Anti-MAP2 | ICC | mouse monoclonal | Chemicon MAB3418 |

(continued)

| Antibody | Application | Description | Source |
|---|---|---|---|
| Anti-SytI | WB | mouse monoclonal | Stressgen SYA148 |
| Anti-TH | WB, ICC, IHC | chicken polyclonal | Abcam ab76442 |

[Table 7]

Table S2. scFv proteins aligned with scFv-A36: Accession number|: number of amino acid residues

| | | |
|---|---|---|
| \|ADC53432.1\|:3-244 | \|AAC26537.1\|:1-228 | \|ADJ00222.1\|:3-248 |
| \|ADB65759-1\|:3-242 | \|CCG26105.1\|:20-266 | \|ACB88023.1\|:1-242 |
| \|ADC53454.1\|:3-243 | \|AAC26550.1\|:1-229 | \|AAC26530.1\|:1-231 |
| \|AAC26528.1\|:1-228 | \|CAA10318.1\|:3-248 | \|ACS12913.1\|:3-240 |
| \|ADB65756.1\|:3-244 | \|S41374:1-247 | \|AAL50781.1\|:10-234 |
| \|ADB65755.1\|:3-244 | \|AAY8890S.1\|:3-240 | \|AAA83267.1\|:1-246 |
| \|ADB65753-1\|.3-241 | \|CAA10385.1\|:1-243 | \|ACB97617.1\|:3-238 |
| \|AEK20780.1\|:3-237 | \|AAC04222.1\|:39-276 | \|AAP23214.1\|:3-247 |
| \|AAQ83756.1\|:1-248 | \|ACZ65029.1\|:3-236 | \|AAY44382-2\|:3-245 |
| \|1DZB\|A:1-239 | \|AAC26540.1\|:1-226 | \|BAN78505.1\|:8-247 |
| \|ADB65760.1\|:3-238 | \|ADN42857.1\|:25-265 | \|BAA22843.1\|:3-242 |
| \|CAC14154.1\|:2-247 | \|ACN88153.1\|:3-241 | \|AAB03768.1\|:2-239 |
| \|AEK20779.1\|:3-242 | \|ACN88155.1\|:3-241 | \|BAN78507.1\|:8-247 |
| \|1QOK\|A:27-268 | \|AAX07566.1\|: 3-240 | \|AF074900_1\|:3-245 |
| \|BAT46707.1\|:3-240 | \|AAA19165.1\|:23-265 | \|AAQ83757.1\|:1-241 |
| \|ADB65757.1\|:3-241 | \|AAKS5297.1\|AP402255_1:3-243 | \|AAK85298.1\|AF402256_1:3-248 |
| \|AAY88907.1\|:3-240 | \|CAD58896.1\|:3-233 | \|AAK56283.1\|:1-239 |
| \|ABN79462.1\|:2-242 | \|ACA49232.1\|:3-247 | \|AAD33867.1\|AF141321_1:3-243 |
| \|ADN42859-1\|:25-265 | \|ADC53451.1\|:3-248 | \|AAB65160.1\|:1-243 |
| \|AAF82630.1[:3-243 | \|5AAW\|A:2-243 | \|AAY44384.2\|:3-245 |
| \|AAD51317.1\|:1-241 | \|AAC26549.1\|:1-2:32 | \|BAN78506.1\|:8-247 |
| \|ALJ99801.1\|:3-251 | \|CAA103S6.1\|:1-249 | \|AEX60024.1\|:43-282 |
| \|AAC26545.1\|:1-228 | \|ACB97619.1\|:3-238 | \|AAC25685-1\|:1-239 |
| \|AAN32896.1\|AF488378_1:3-244 | \|ADC5345S.1\|:3-238 | \|AAA75173.1\|:23-270 |
| \|3UYP\|A:5-246 | \|AAC26546.1\|:1-232 | \|AEX57225.1\|:58-297 |
| \|ADB65758.1\|:3-241 | \|CAA94521.1\|:1-248 | \|AAL25135.1\|AF434672_1:1-237 |
| \|AAC52185.1\|:3-242 | \|AAF82631.1\|:3-242 | \|AAU10332.1\|:22-271 |
| \|ADC53456.1\|:3-246 | \|AAF85943.1\|:1-249 | \|AHM25305.1\|:3-249 |
| \|AAU11282.1\|:2-240 | \|CCG26106.1\|:20-263 | \|CAC42244.1\|:3-244 |
| \|A56446:3-242 | \|ACV91950.1\|:2-239 | \|ACN88154.1\|:3-241 |
| \|CAA82617.1\|:1-247 | \|AAG44840.1\|:1-250 | \|ACN87219.1\|:3-243 |
| | | JC-5322:1-233 |

Industrial Applicability

The present invention is applicable, for example, to various research and to medical science such as diagnosis and treatment.

## Claims

1. A fusion protein, comprising: an intracellular-stabilizer peptide; and an antigen-binding peptide, the intracellular-stabilizer peptide consisting of 10 to 39 amino acids, at least 45% of the 10 to 39 amino acids being acidic amino acids, the antigen-binding peptide containing at least one of heavy chain CDR1, heavy chain CDR2, heavy chain CDR3, light chain CDR1, light chain CDR2, and light chain CDR3.

2. The fusion protein as set forth in claim 1, wherein: 28% or less of the 10 to 39 amino acids of the intracellular-stabilizer peptide are basic amino acids; or the intracellular-stabilizer peptide contains no basic amino acid.

3. The fusion protein as set forth in claim 2, wherein the intracellular-stabilizer peptide contains no basic amino acid.

4. The fusion protein as set forth in claim 2, wherein the intracellular-stabilizer peptide contains at least one histidine as a basic amino acid.

5. The fusion protein as set forth in any one of claims 1 through 4, wherein: the intracellular-stabilizer peptide consists of 14 to 25 amino acids, at least 50% of the 14 to 25 amino acids being acidic amino acids; and
the intracellular-stabilizer peptide containing neither a portion consisting of 8 or more consecutive acidic amino acids nor a portion consisting of 4 or more consecutive amino acids that are not acidic amino acids.

6. The fusion protein as set forth in claim 5, wherein the intracellular-stabilizer peptide contains neither a portion consisting of 3 or more consecutive acidic amino acids nor a portion consisting of 3 or more consecutive amino acids that are not acidic amino acids.

7. The fusion protein as set forth in any one of claims 1 through 6, wherein the intracellular-stabilizer peptide contains the following amino acid sequence (1) or (2):

$$-X_n-X_A-X_A-X_n-X_A-(X_A \text{ or } X_n)-X_n-X_A-(X_A \text{ or } X_n)-X_n-(X_A \text{ or } X_n)-X_A- \ldots(1);$$

or
$-X_n-X_A-X_n-X_A-X_n-X_A-X_A-X_n-X_A-X_n-X_n-X_A-X_A-X_n-X_n-X_A-$...(2),
where $X_A$ is an acidic amino acid and $X_n$ is an amino acid that is not an acidic amino acid.

8. The fusion protein as set forth in claim 7, wherein $X_A$ is aspartic acid or glutamic acid, and $X_n$ is an amino acid selected from the group consisting of asparagine, glutamine, proline, tyrosine, and valine.

9. A polynucleotide encoding a fusion protein recited in any one of claims 1 through 8.

10. An expression vector comprising a polynucleotide recited in claim 9.

11. A method for producing a cell capable of expressing an antigen-binding peptide in the cell, the method comprising the step of:
introducing a polynucleotide recited in claim 9 or an expression vector recited in claim 10 into a cell.

12. A cell capable of expressing a fusion protein recited in any one of claims 1 through 8.

FIG. 1

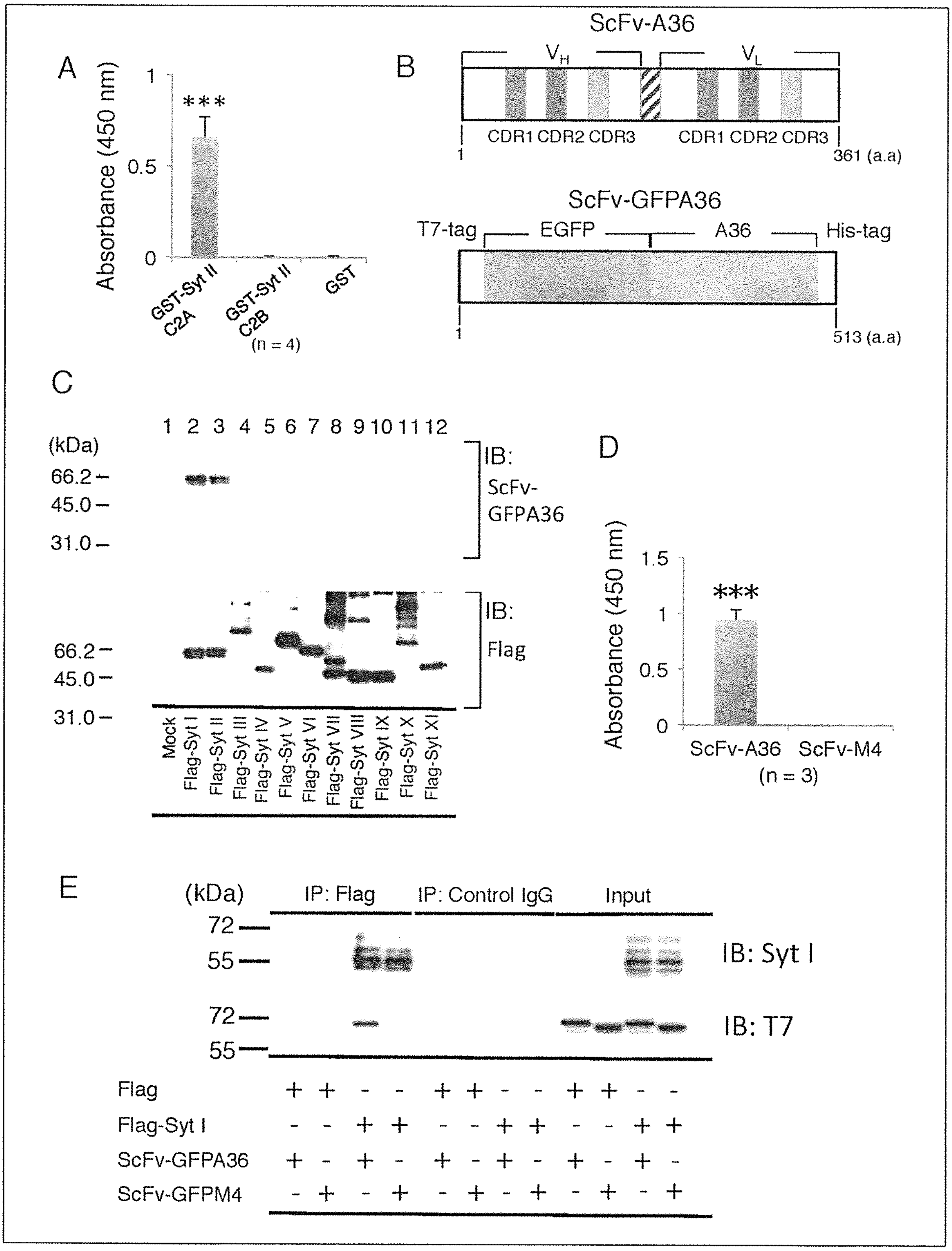
A
Absorbance (450 nm)
1
0.5
0
***
GST-Syt II C2A
GST-Syt II C2B
GST
(n = 4)
B
ScFv-A36
VH
VL
CDR1 CDR2 CDR3
CDR1 CDR2 CDR3
1
361 (a.a)
ScFv-GFPA36
T7-tag
EGFP
A36
His-tag
1
513 (a.a)
C
1 2 3 4 5 6 7 8 9 10 11 12
(kDa)
66.2
45.0
31.0
IB: ScFv-GFPA36
IB: Flag
Mock
Flag-Syt I
Flag-Syt II
Flag-Syt III
Flag-Syt IV
Flag-Syt V
Flag-Syt VI
Flag-Syt VII
Flag-Syt VIII
Flag-Syt IX
Flag-Syt X
Flag-Syt XI
D
Absorbance (450 nm)
1.5
1
0.5
0
***
ScFv-A36
ScFv-M4
(n = 3)
E
(kDa)
IP: Flag
IP: Control IgG
Input
72
55
IB: Syt I
IB: T7
Flag + + - - + + - - + + - -
Flag-Syt I - - + + - - + + - - + +
ScFv-GFPA36 + - + - + - + - + - + -
ScFv-GFPM4 - + - + - + - + - + - +

FIG. 2

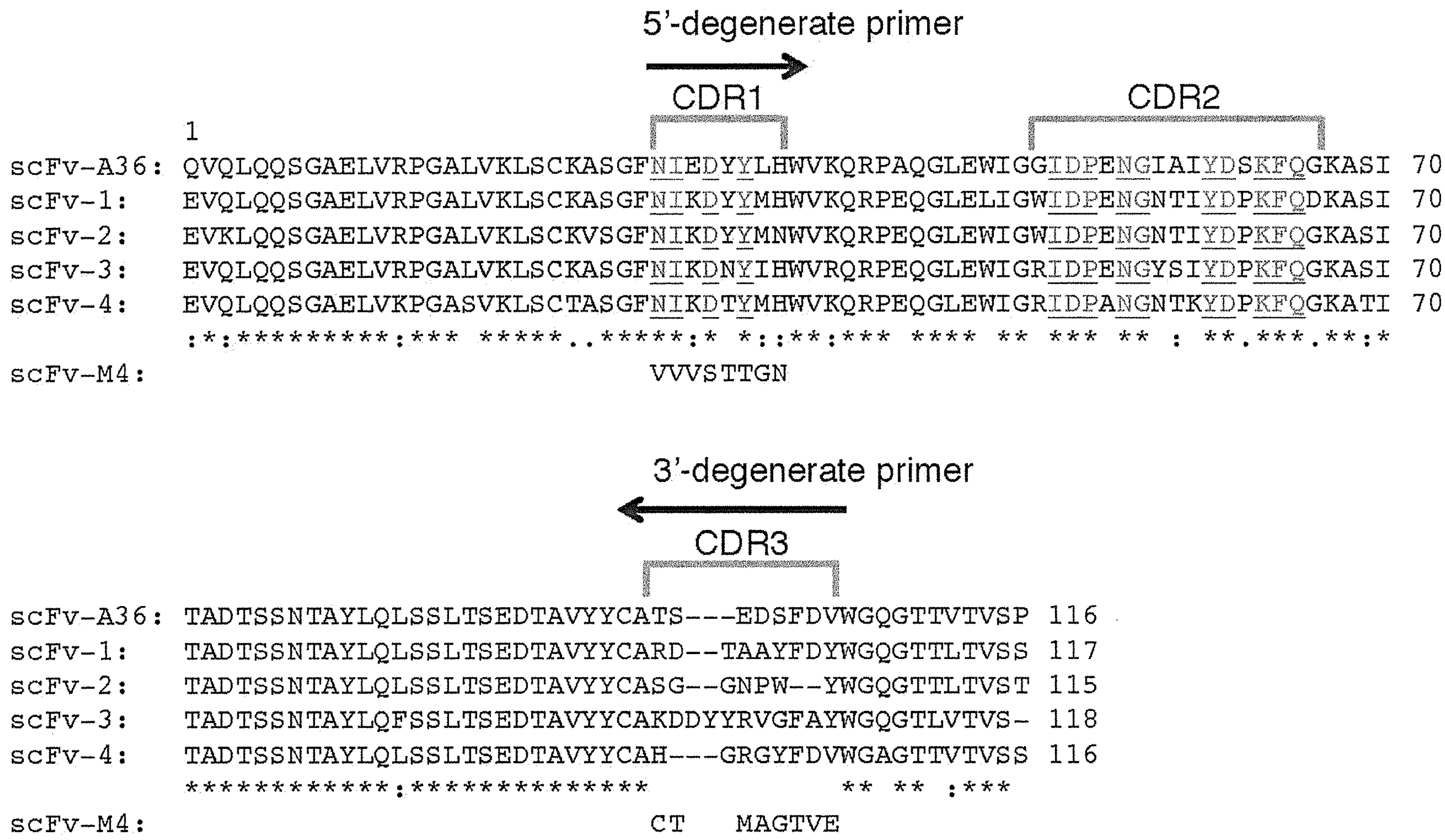
5'-degenerate primer
CDR1
CDR2
1
scFv-A36: QVQLQQSGAELVRPGALVKLSCKASGFNIEDYYLHWVKQRPAQGLEWIGGIDPENGIAIYDSKFQGKASI 70
scFv-1: EVQLQQSGAELVRPGALVKLSCKASGFNIKDYYMHWVKQRPEQGLELIGWIDPENGNTIYDPKFQDKASI 70
scFv-2: EVKLQQSGAELVRPGALVKLSCKVSGFNIKDYYMNWVKQRPEQGLEWIGWIDPENGNTIYDPKFQGKASI 70
scFv-3: EVQLQQSGAELVRPGALVKLSCKASGFNIKDNYIHWVRQRPEQGLEWIGRIDPENGYSIYDPKFQGKASI 70
scFv-4: EVQLQQSGAELVKPGASVKLSCTASGFNIKDTYMHWVKQRPEQGLEWIGRIDPANGNTKYDPKFQGKATI 70
:*:*********:*** *****..*****:* *::**:*** **** ** *** ** : **.***.**:*
scFv-M4: VVVSTTGN
3'-degenerate primer
CDR3
scFv-A36: TADTSSNTAYLQLSSLTSEDTAVYYCATS---EDSFDVWGQGTTVTVSP 116
scFv-1: TADTSSNTAYLQLSSLTSEDTAVYYCARD--TAAYFDYWGQGTTLTVSS 117
scFv-2: TADTSSNTAYLQLSSLTSEDTAVYYCASG--GNPW--YWGQGTTLTVST 115
scFv-3: TADTSSNTAYLQFSSLTSEDTAVYYCAKDDYYRVGFAYWGQGTLVTVS- 118
scFv-4: TADTSSNTAYLQLSSLTSEDTAVYYCAH---GRGYFDVWGAGTTVTVSS 116
***********:************** ** ** :***
scFv-M4: CT MAGTVE

FIG. 3

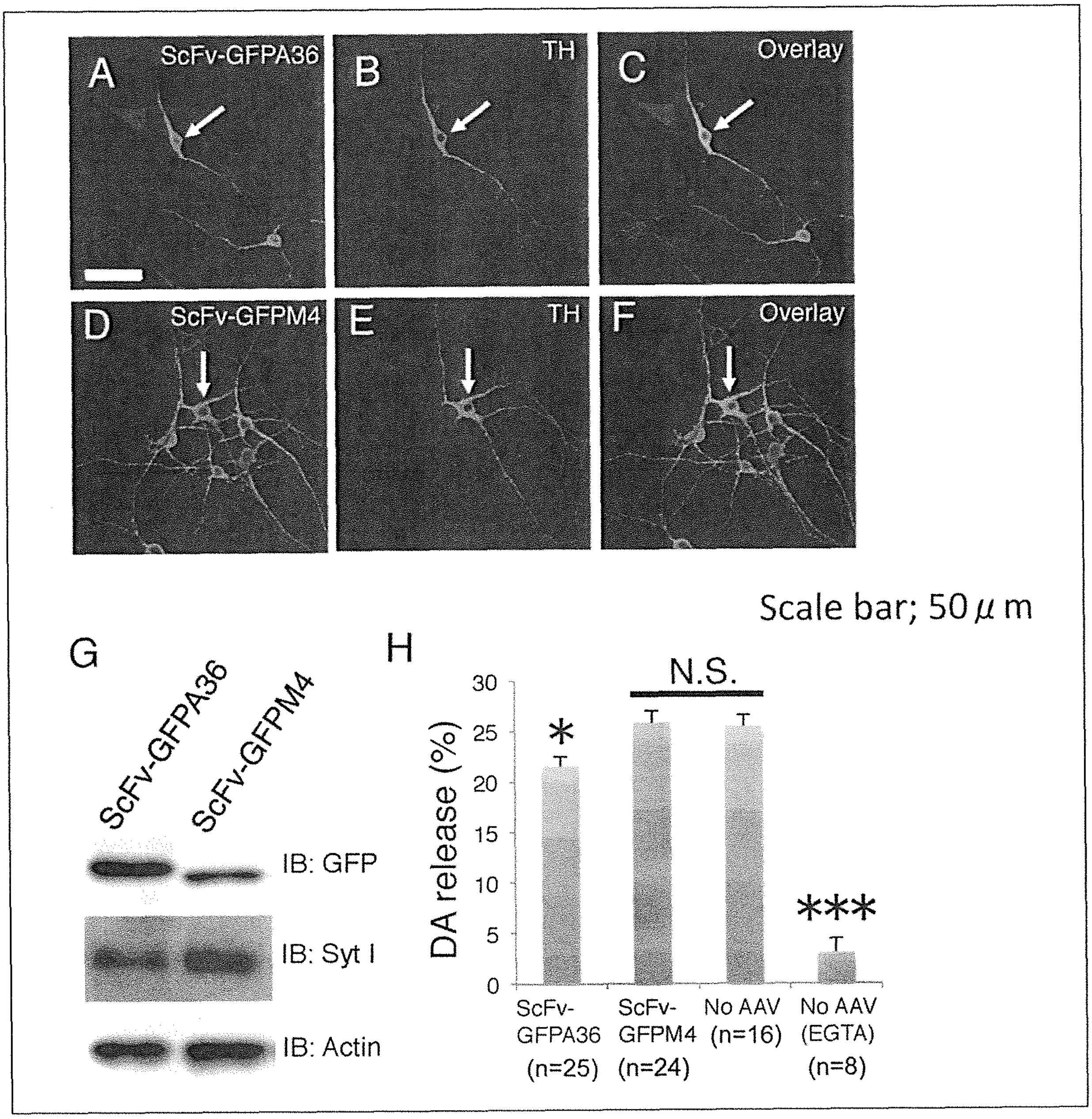
A
ScFv-GFPA36
B
TH
C
Overlay
D
ScFv-GFPM4
E
TH
F
Overlay
Scale bar; 50 μm
G
ScFv-GFPA36
ScFv-GFPM4
IB: GFP
IB: Syt I
IB: Actin
H
N.S.
DA release (%)
30
25
20
15
10
5
0
*
***
ScFv-GFPA36 (n=25)
ScFv-GFPM4 (n=24)
No AAV (n=16)
No AAV (EGTA) (n=8)

FIG. 4

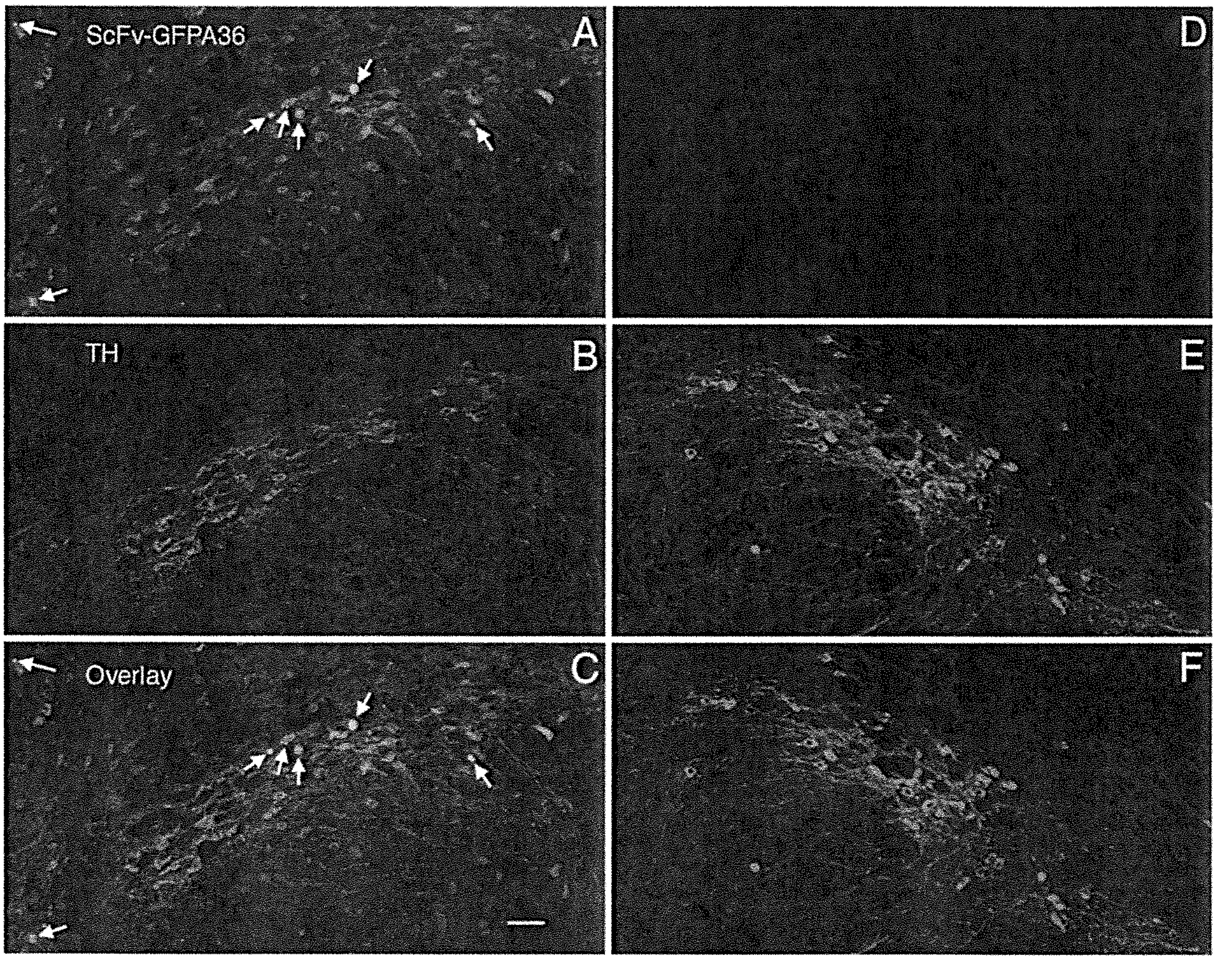


Scale bar; 50 $\mu$ m

FIG. 5

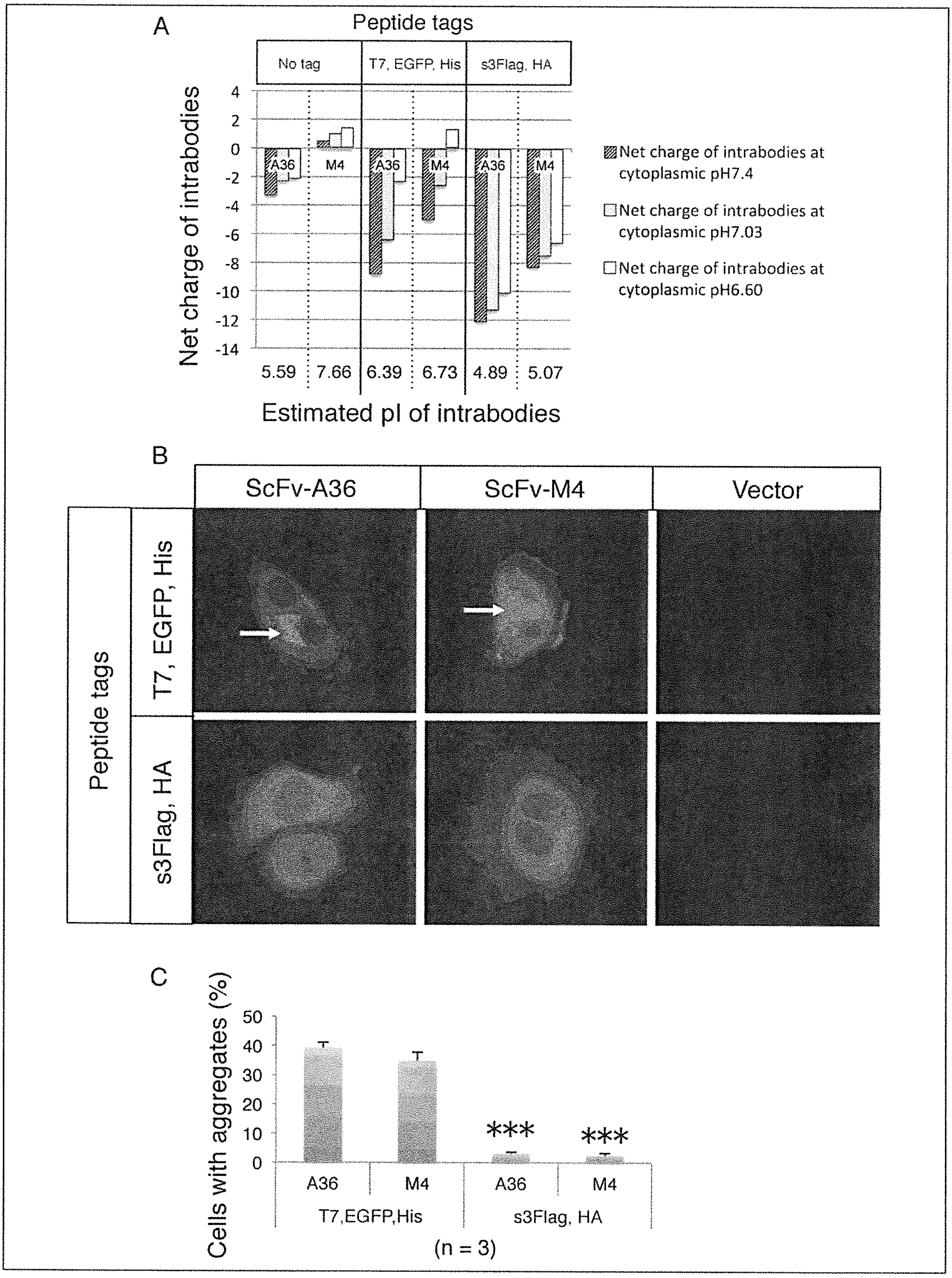

A
Peptide tags
No tag
T7, EGFP, His
s3Flag, HA
Net charge of intrabodies
4
2
0
-2
-4
-6
-8
-10
-12
-14
A36
M4
A36
M4
A36
M4
5.59
7.66
6.39
6.73
4.89
5.07
Estimated pI of intrabodies
Net charge of intrabodies at cytoplasmic pH7.4
Net charge of intrabodies at cytoplasmic pH7.03
Net charge of intrabodies at cytoplasmic pH6.60
B
ScFv-A36
ScFv-M4
Vector
Peptide tags
T7, EGFP, His
s3Flag, HA
C
Cells with aggregates (%)
50
40
30
20
10
0
***
***
A36
M4
A36
M4
T7,EGFP,His
s3Flag, HA
(n = 3)

FIG. 6

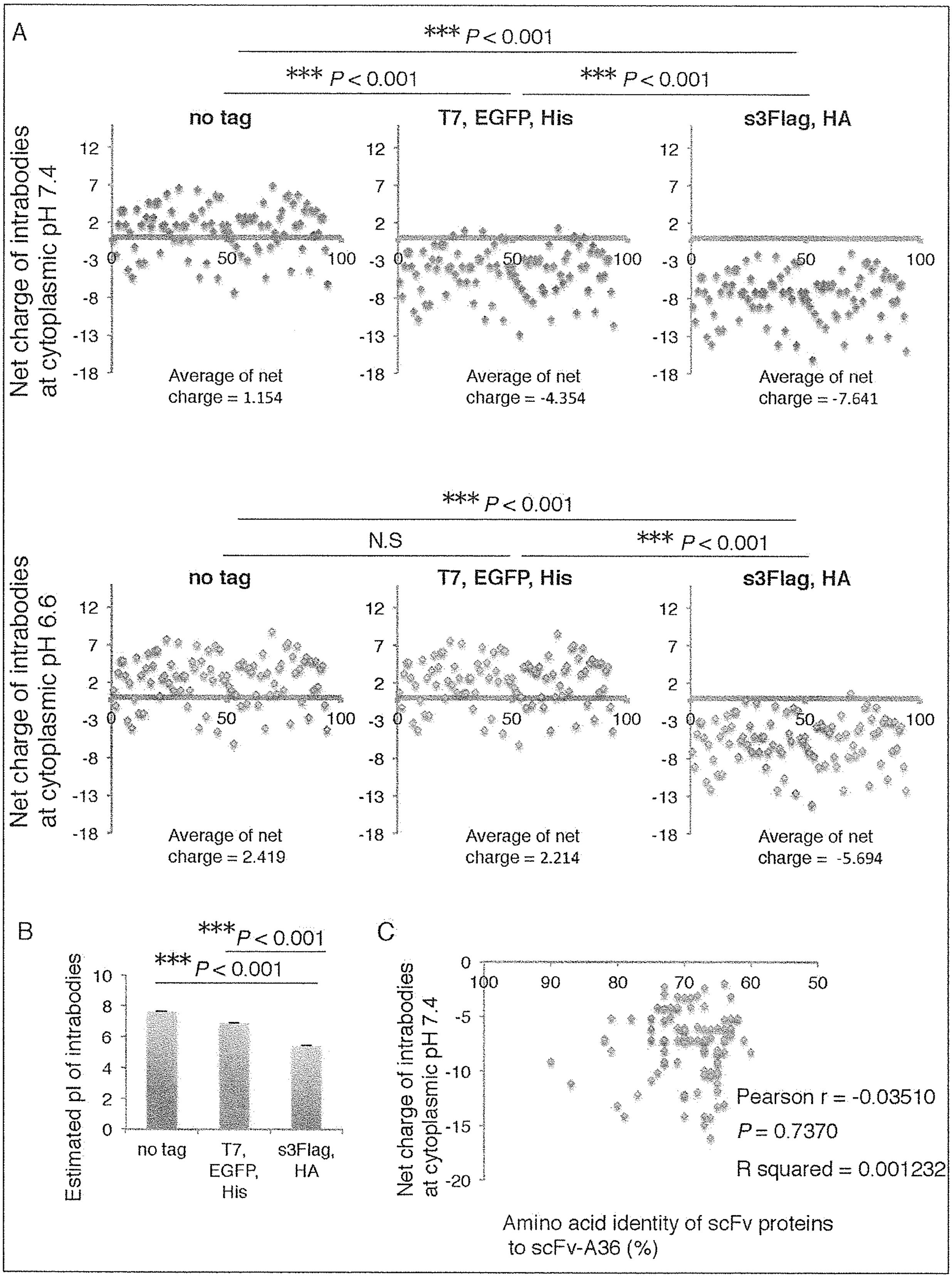
A
*** P < 0.001
*** P < 0.001
*** P < 0.001
no tag
T7, EGFP, His
s3Flag, HA
Net charge of intrabodies at cytoplasmic pH 7.4
12
7
2
-3
-8
-13
-18
0
50
100
Average of net charge = 1.154
Average of net charge = -4.354
Average of net charge = -7.641
*** P < 0.001
N.S
*** P < 0.001
no tag
T7, EGFP, His
s3Flag, HA
Net charge of intrabodies at cytoplasmic pH 6.6
Average of net charge = 2.419
Average of net charge = 2.214
Average of net charge = -5.694
B
*** P < 0.001
*** P < 0.001
Estimated pI of intrabodies
10
8
6
4
2
0
no tag
T7, EGFP, His
s3Flag, HA
C
Net charge of intrabodies at cytoplasmic pH 7.4
0
-5
-10
-15
-20
100
90
80
70
60
50
Pearson r = -0.03510
P = 0.7370
R squared = 0.001232
Amino acid identity of scFv proteins to scFv-A36 (%)

FIG. 7

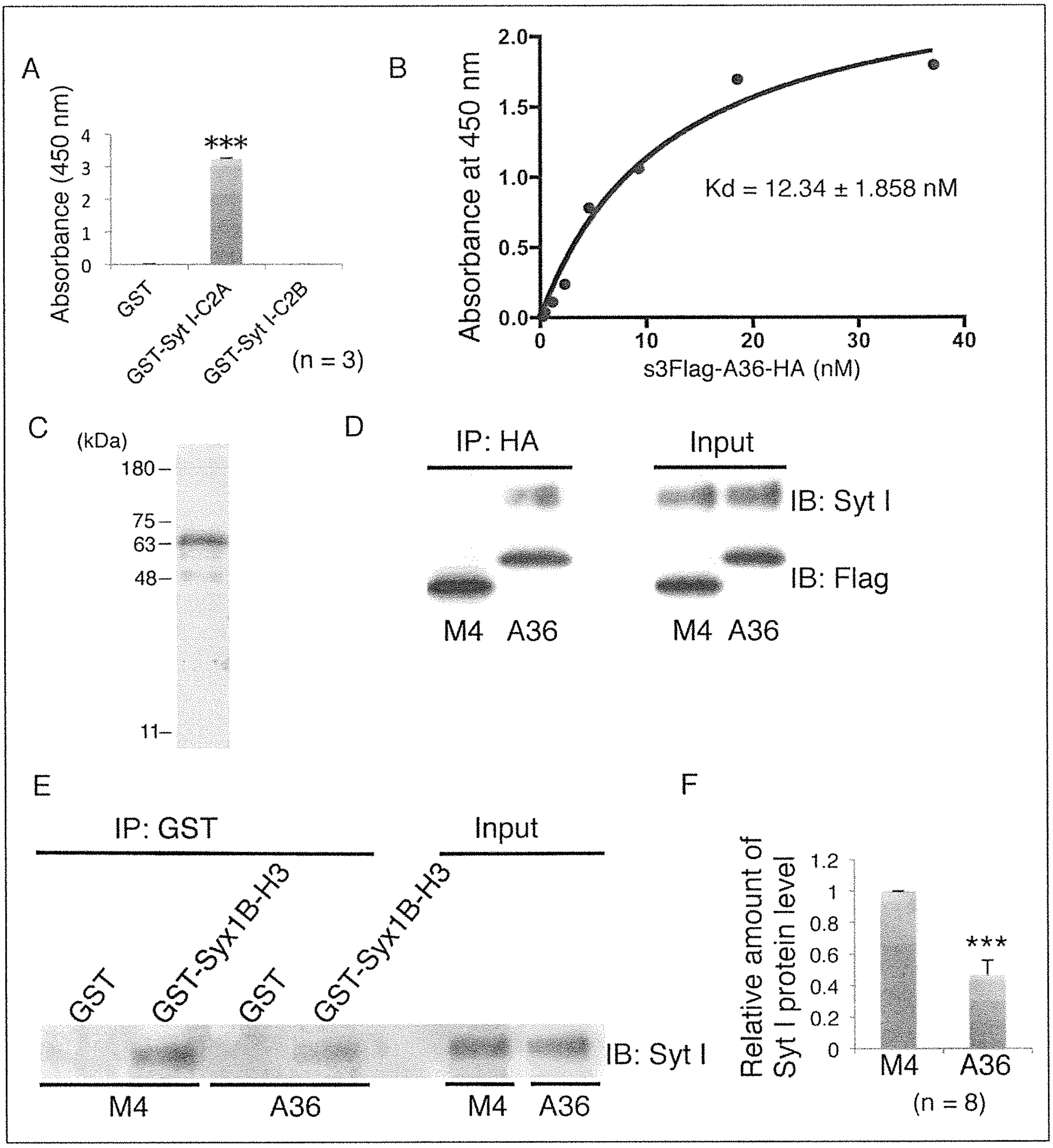

A
Absorbance (450 nm)
4
3
2
1
0
***
GST
GST-Syt I-C2A
GST-Syt I-C2B
(n = 3)
B
Absorbance at 450 nm
2.0
1.5
1.0
0.5
0.0
0
10
20
30
40
s3Flag-A36-HA (nM)
Kd = 12.34 ± 1.858 nM
C
(kDa)
180
75
63
48
11
D
IP: HA
Input
IB: Syt I
IB: Flag
M4 A36
M4 A36
E
IP: GST
Input
GST
GST-Syx1B-H3
GST
GST-Syx1B-H3
IB: Syt I
M4
A36
M4
A36
F
Relative amount of Syt I protein level
1.2
1
0.8
0.6
0.4
0.2
0
***
M4
A36
(n = 8)

FIG. 8

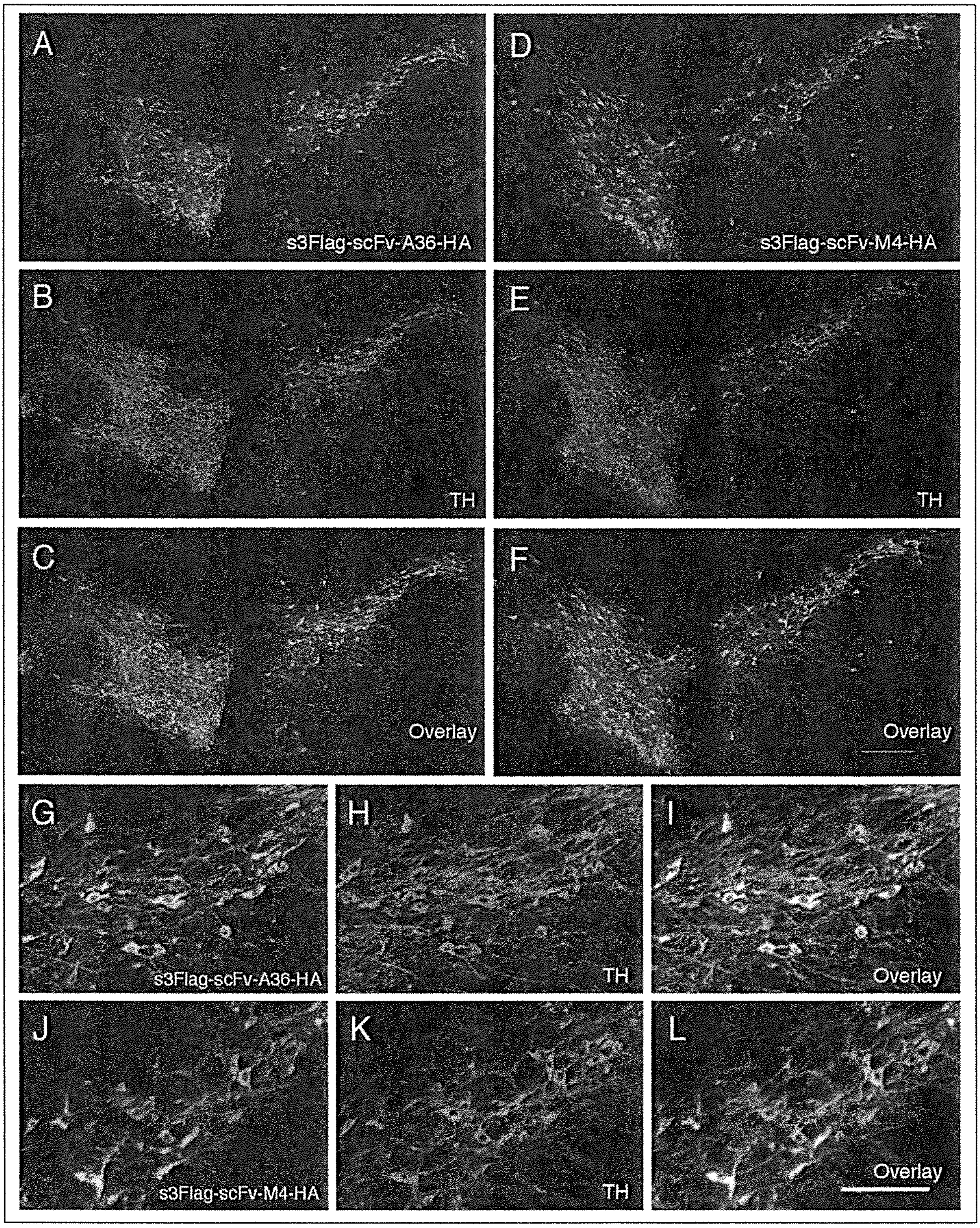
A
s3Flag-scFv-A36-HA
D
s3Flag-scFv-M4-HA
B
TH
E
TH
C
Overlay
F
Overlay
G
s3Flag-scFv-A36-HA
H
TH
I
Overlay
J
s3Flag-scFv-M4-HA
K
TH
L
Overlay

FIG. 9

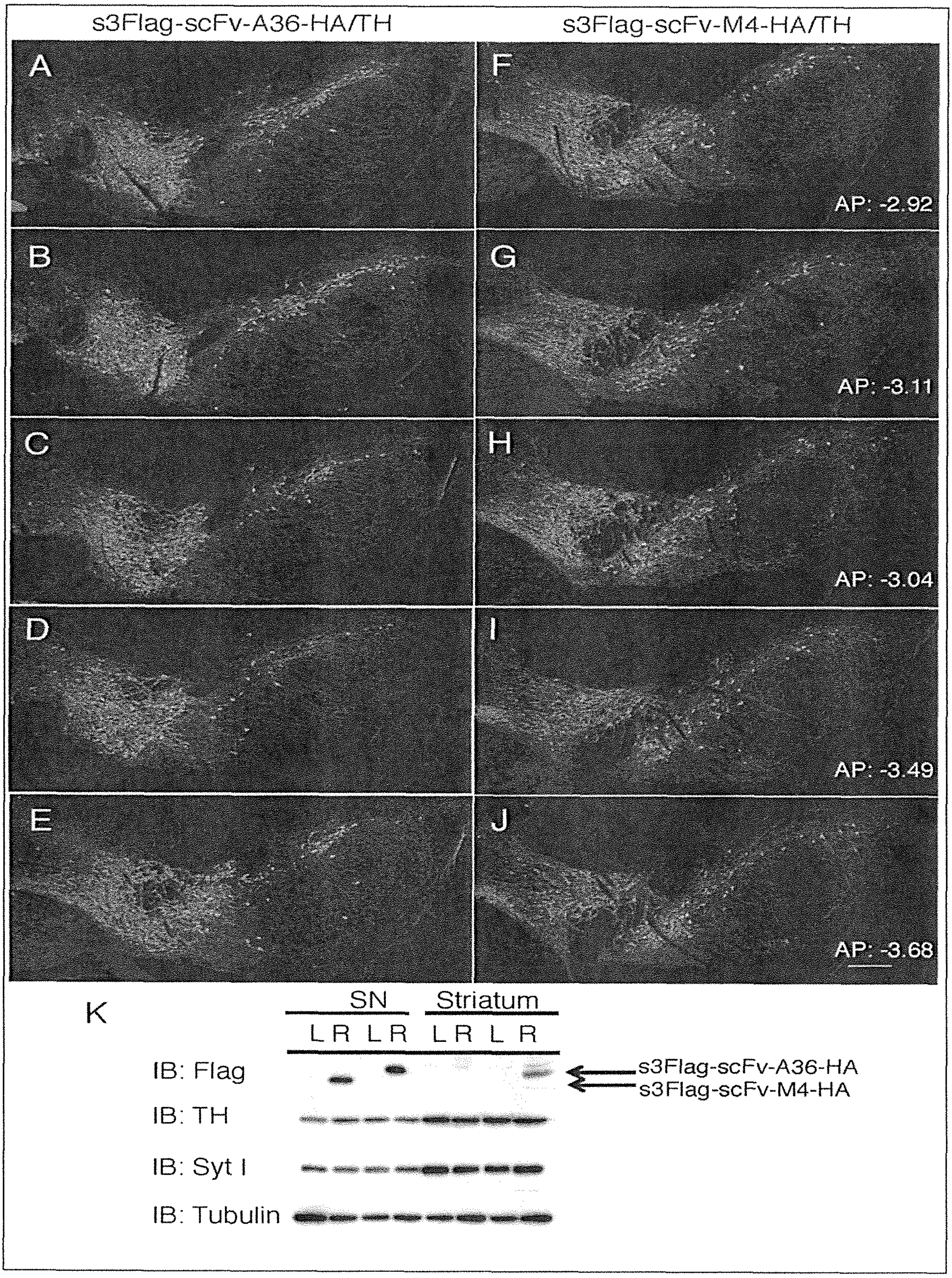
s3Flag-scFv-A36-HA/TH
s3Flag-scFv-M4-HA/TH
A
B
C
D
E
F
G
H
I
J
AP: -2.92
AP: -3.11
AP: -3.04
AP: -3.49
AP: -3.68
K
SN
Striatum
L R L R L R L R
IB: Flag
IB: TH
IB: Syt I
IB: Tubulin
s3Flag-scFv-A36-HA
s3Flag-scFv-M4-HA

FIG. 10

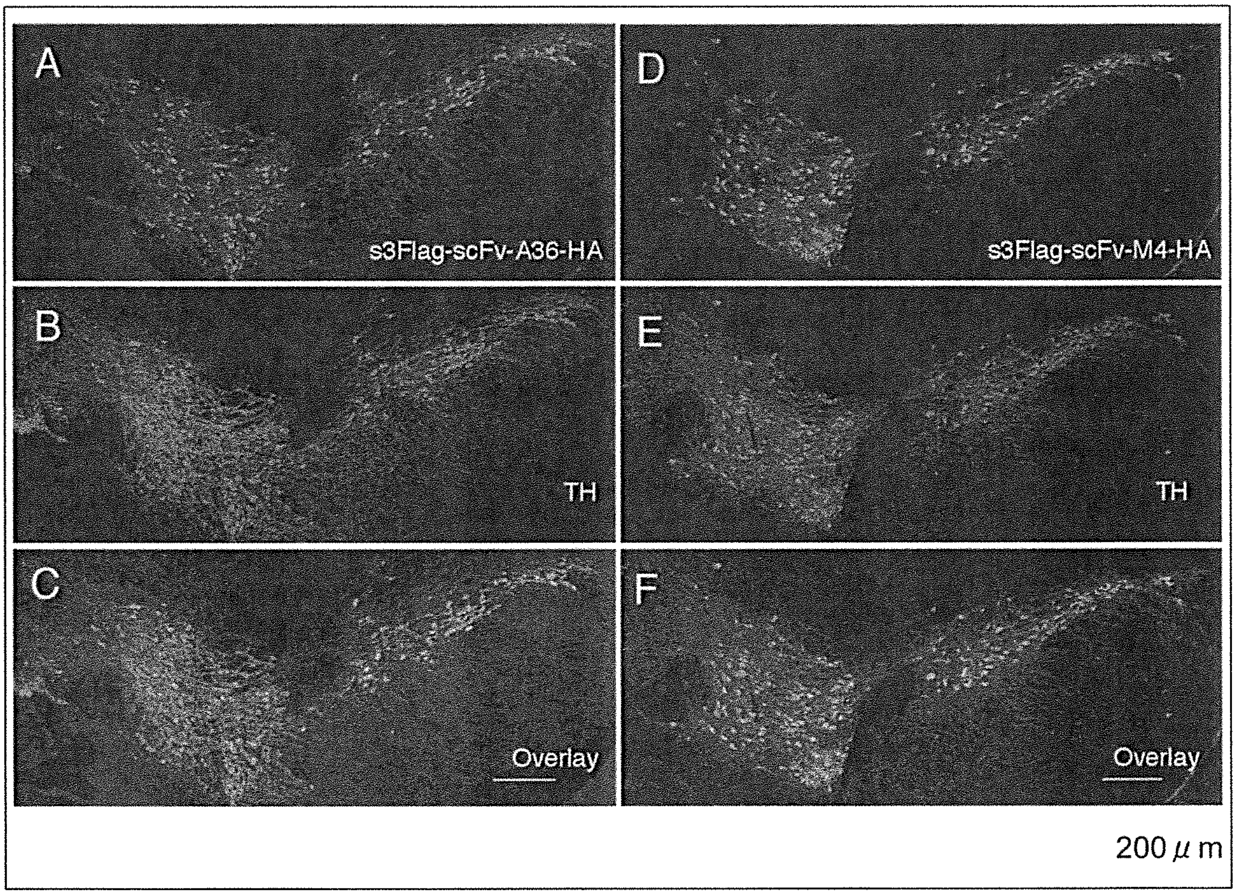
A
s3Flag-scFv-A36-HA
B
TH
C
Overlay
D
s3Flag-scFv-M4-HA
E
TH
F
Overlay
200 μ m

FIG. 11

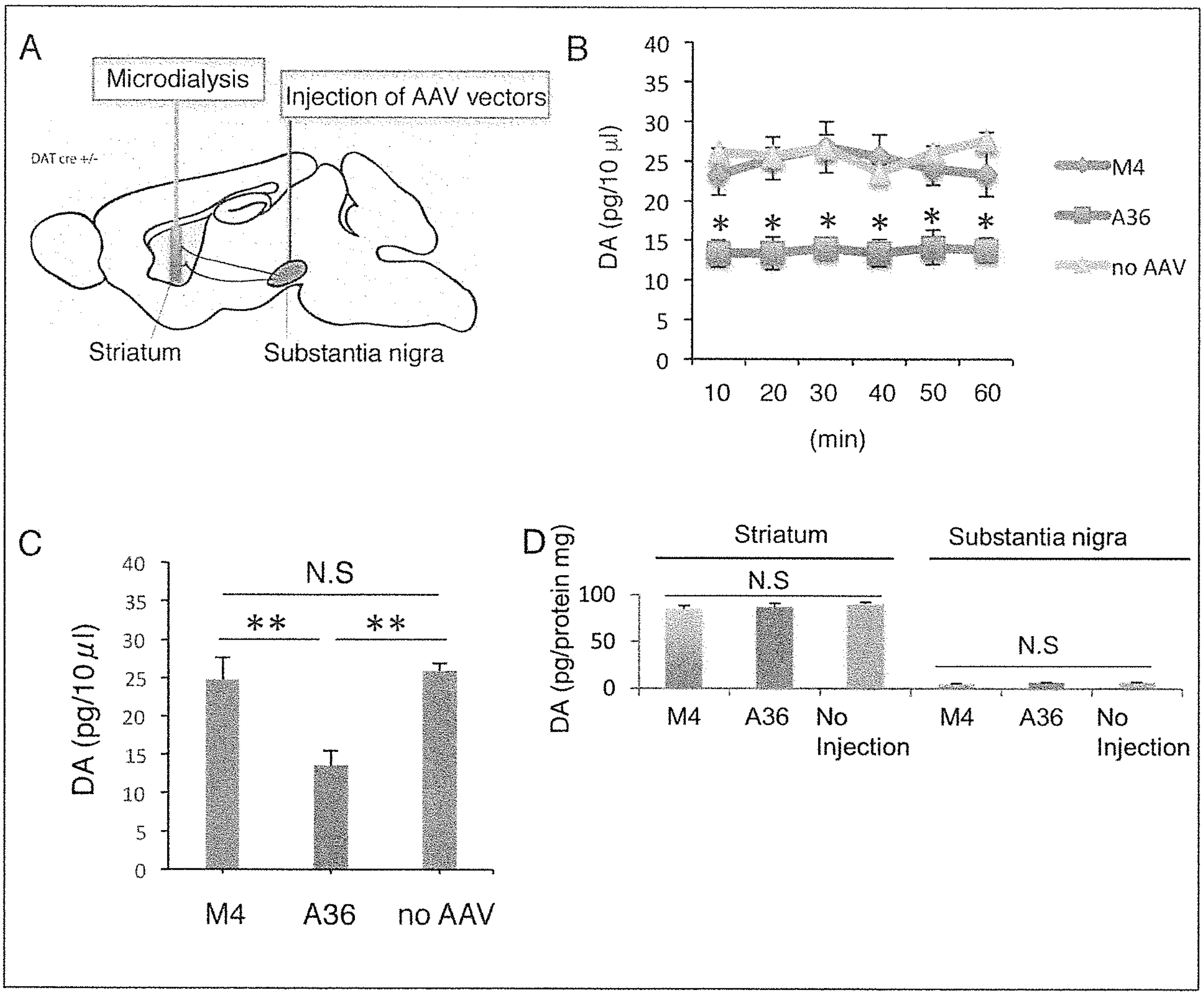
A
Microdialysis
Injection of AAV vectors
DAT cre +/-
Striatum
Substantia nigra
B
DA (pg/10 μl)
40
35
30
25
20
15
10
5
0
10 20 30 40 50 60
(min)
M4
A36
no AAV
C
DA (pg/10μl)
N.S
**
**
M4 A36 no AAV
D
DA (pg/protein mg)
Striatum
Substantia nigra
N.S
N.S
100
50
0
M4 A36 No Injection
M4 A36 No Injection

FIG. 12

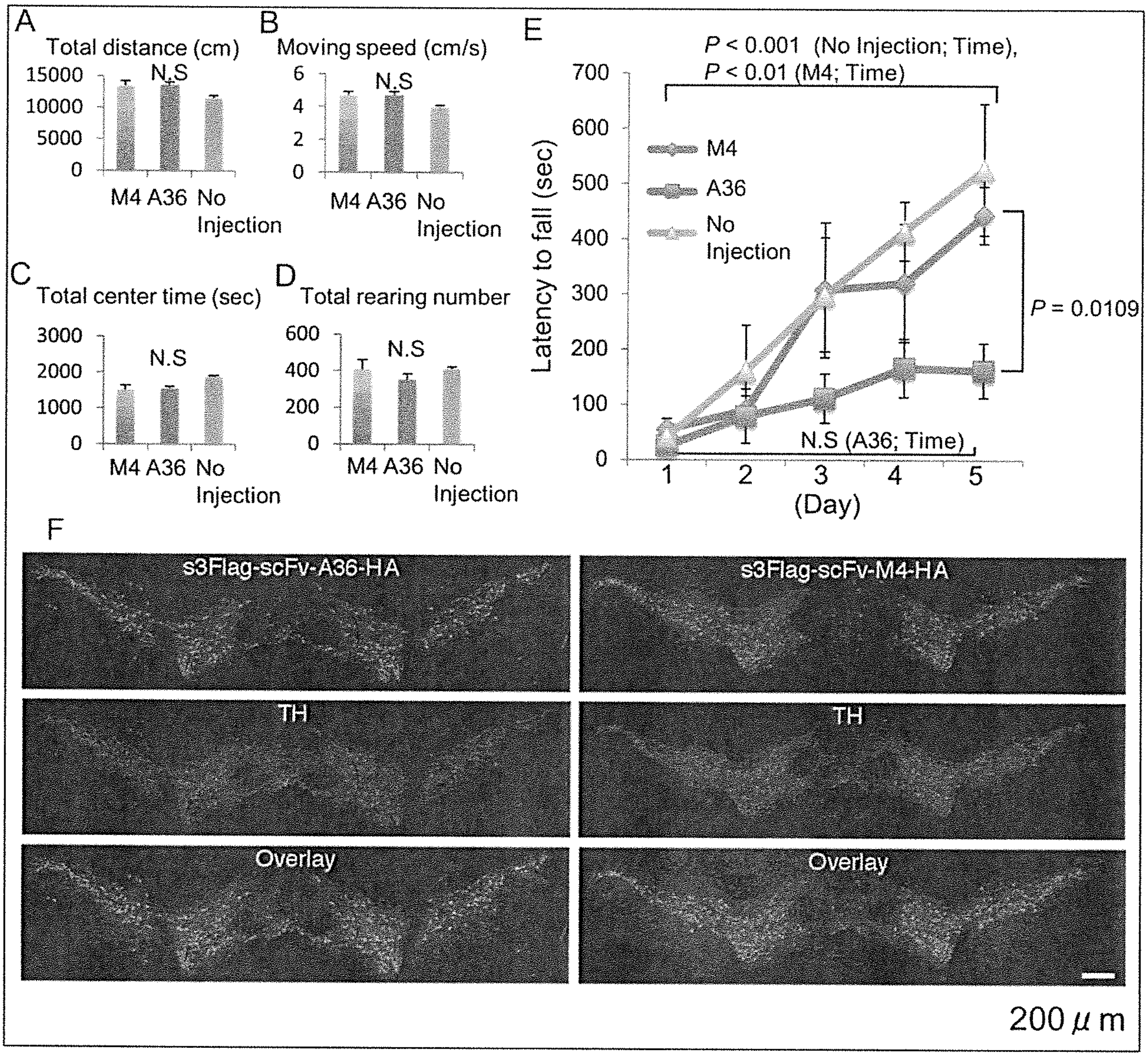
A
Total distance (cm)
N.S
15000
10000
5000
0
M4 A36 No Injection
B
Moving speed (cm/s)
N.S
6
4
2
0
M4 A36 No Injection
C
Total center time (sec)
N.S
3000
2000
1000
0
M4 A36 No Injection
D
Total rearing number
N.S
600
400
200
0
M4 A36 No Injection
E
P < 0.001 (No Injection; Time),
P < 0.01 (M4; Time)
Latency to fall (sec)
700
600
500
400
300
200
100
0
M4
A36
No Injection
P = 0.0109
N.S (A36; Time)
1 2 3 4 5
(Day)
F
s3Flag-scFv-A36-HA
s3Flag-scFv-M4-HA
TH
TH
Overlay
Overlay
200 μm

FIG. 13

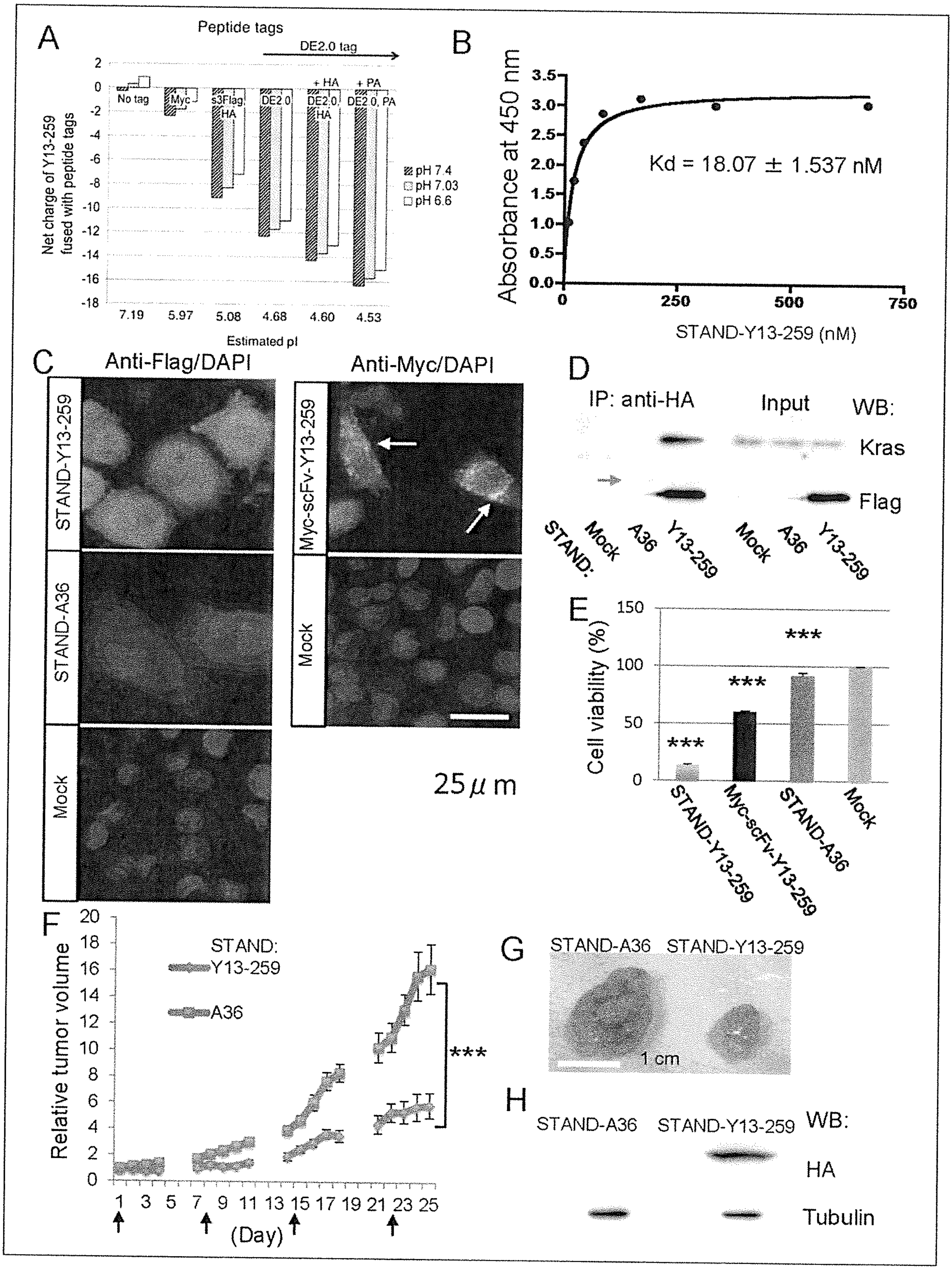
A
Peptide tags
DE2.0 tag
No tag
Myc
s3Flag
HA
DE2.0
+ HA
DE2.0
HA
+ PA
DE2.0, PA
Net charge of Y13-259 fused with peptide tags
pH 7.4
pH 7.03
pH 6.6
7.19 5.97 5.08 4.68 4.60 4.53
Estimated pI
B
Absorbance at 450 nm
Kd = 18.07 ± 1.537 nM
STAND-Y13-259 (nM)
C
Anti-Flag/DAPI
Anti-Myc/DAPI
STAND-Y13-259
STAND-A36
Mock
Myc-scFv-Y13-259
Mock
25μm
D
IP: anti-HA
Input
WB:
Kras
Flag
STAND:
Mock
A36
Y13-259
Mock
A36
Y13-259
E
Cell viability (%)
***
***
***
STAND-Y13-259
Myc-scFv-Y13-259
STAND-A36
Mock
F
Relative tumor volume
STAND:
Y13-259
A36
***
(Day)
G
STAND-A36
STAND-Y13-259
1 cm
H
STAND-A36
STAND-Y13-259
WB:
HA
Tubulin

FIG. 14

A

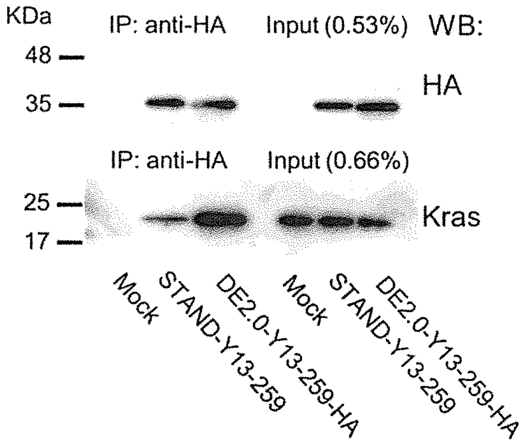


Interaction of endogenous Kras with STAND proteins in MIA PaCa2 cells

B

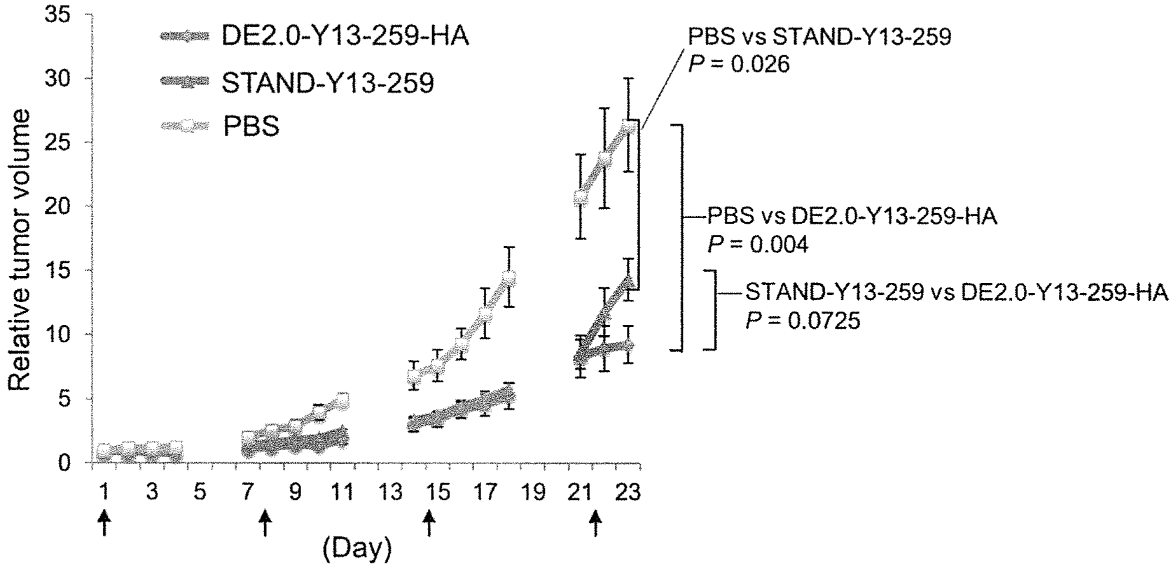


Total 44 μl of Lentiviral particles (4.2 × $10^6$ units) was injected into one site of the intratumor of nude mice

FIG. 15

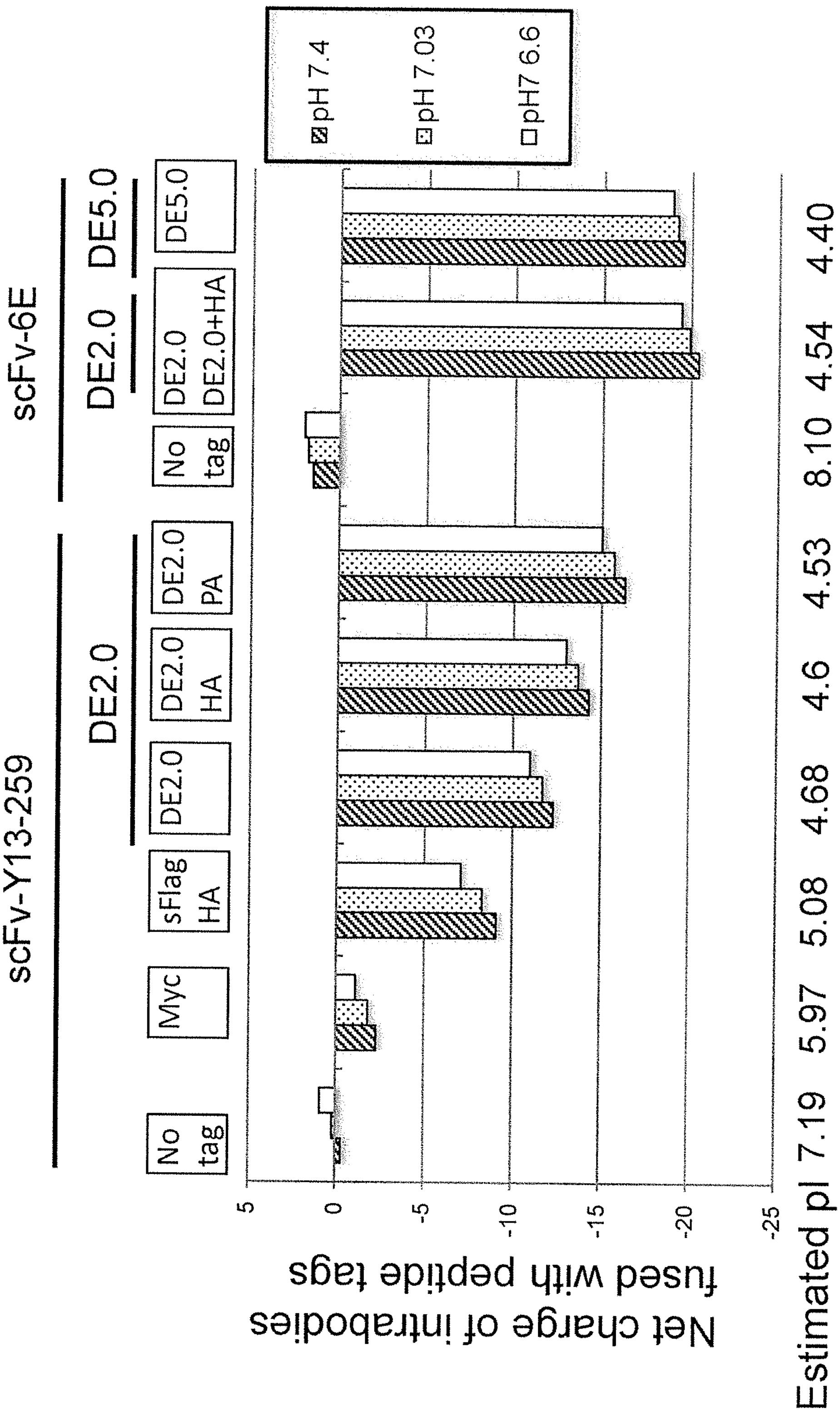
scFv-Y13-259
scFv-6E
DE2.0
DE2.0
DE5.0
No tag
Myc
sFlag HA
DE2.0
DE2.0 HA
DE2.0 PA
No tag
DE2.0 DE2.0+HA
DE5.0
pH 7.4
pH 7.03
pH7 6.6
Net charge of intrabodies fused with peptide tags
5
0
-5
-10
-15
-20
-25
Estimated pI 7.19 5.97 5.08 4.68 4.6 4.53 8.10 4.54 4.40

FIG. 16

CONFIRMATION OF EXPRESSION IN HeLa CELLS

STAND-6E-LYS

DE5.0-6E

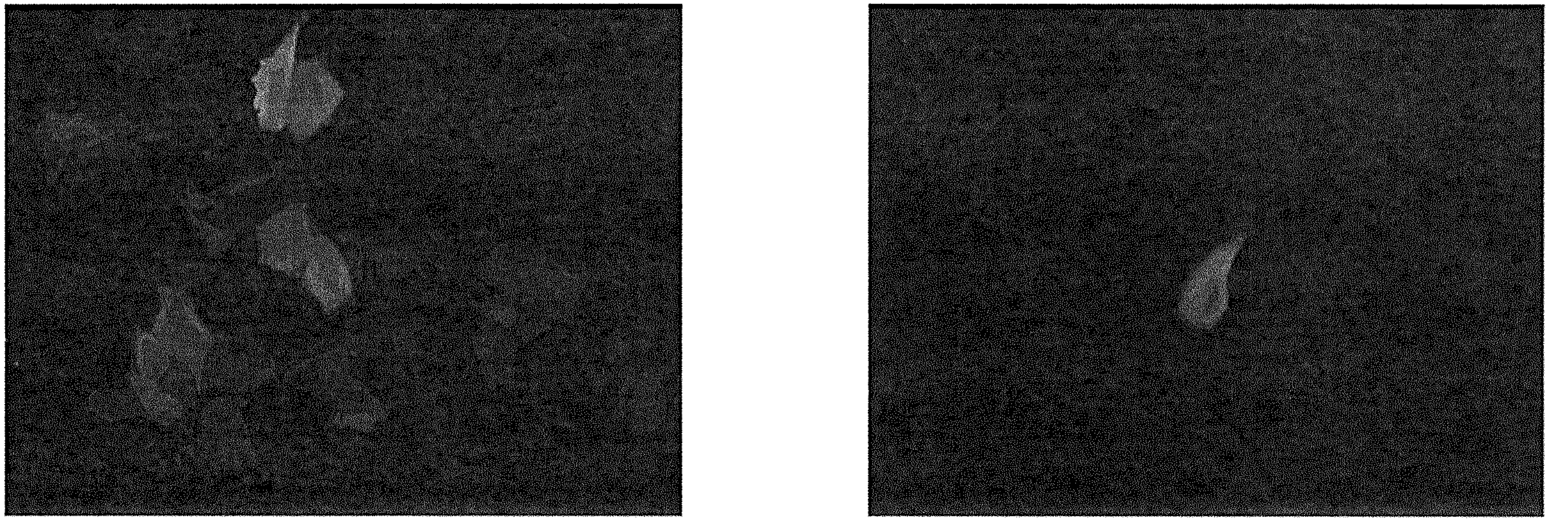

FIG. 17

α-synuclein AGGREGATION ASSAY (SHSY5Y CELLS)

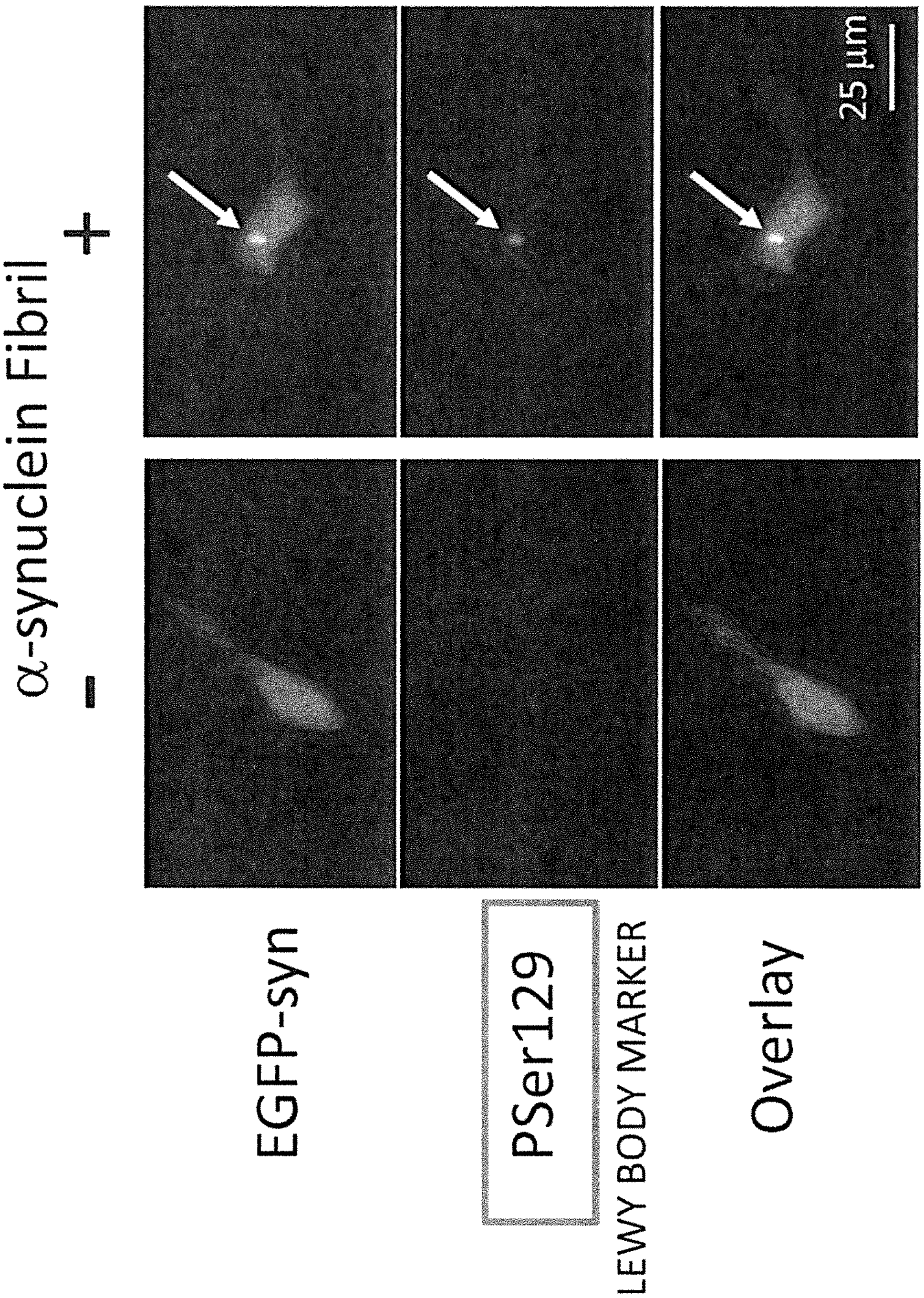

FIG. 18

EVALUATION OF α-synuclein AGGREGATION (SHSY5Y CELLS)

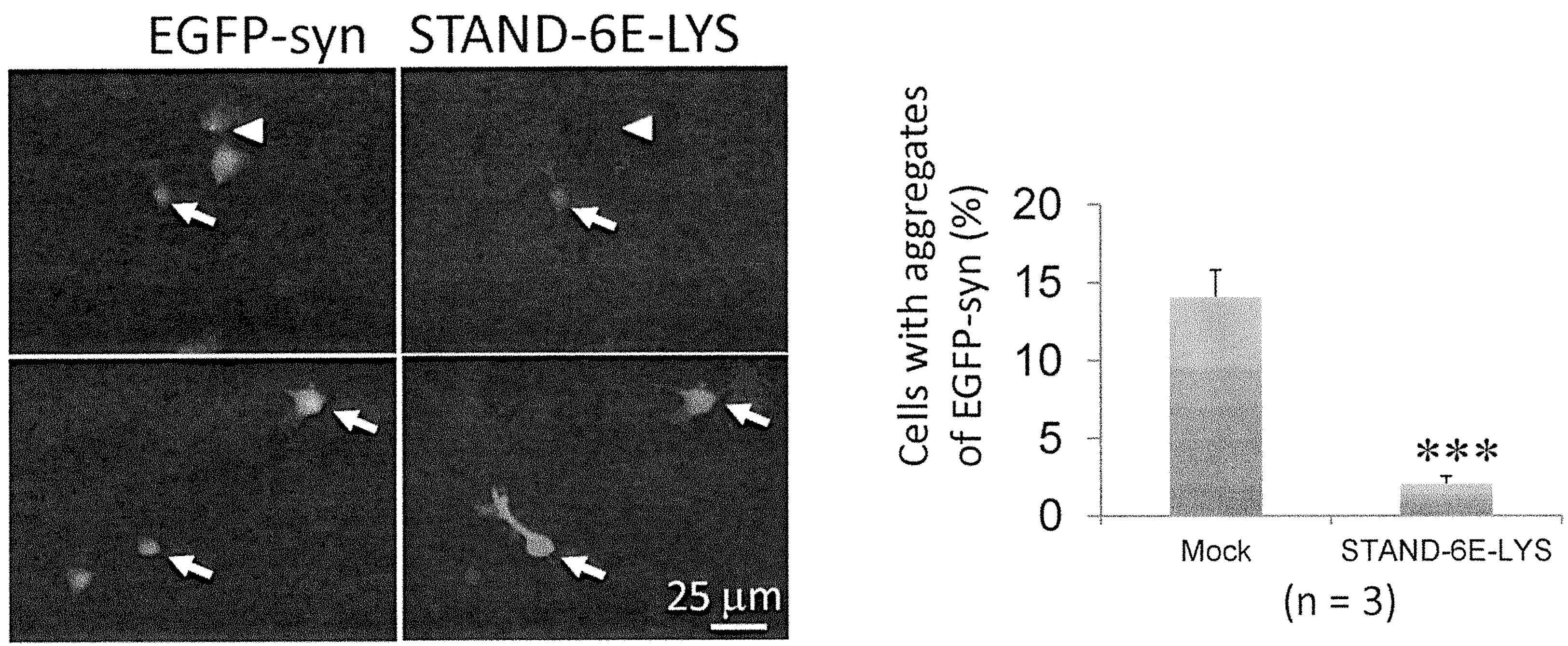

**INTERNATIONAL SEARCH REPORT**

International application No. PCT/JP2018/024197

A. CLASSIFICATION OF SUBJECT MATTER
Int.Cl. C12N15/62(2006.01)i, C07K7/00(2006.01)i, C07K14/00(2006.01)i, C07K16/00(2006.01)i, C07K19/00(2006.01)i, C12N1/15(2006.01)i, C12N1/19(2006.01)i, C12N1/21(2006.01)i, C12N5/10(2006.01)i

According to International Patent Classification (IPC) or to both national classification and IPC

B. FIELDS SEARCHED

Minimum documentation searched (classification system followed by classification symbols)
Int.Cl. C12N15/62, C07K7/00, C07K14/00, C07K16/00, C07K19/00, C12N1/15, C12N1/19, C12N1/21, C12N5/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS/WPIDS (STN), UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X<br>Y | KVAM, Erik et al., "Physico-chemical determinants of soluble intrabody expression in mammalian cell cytoplasm", Protein Enginnering, Design & Selection, 2010, vol. 23, no. 6, pp. 489-498, in particular, page 490, left column, last paragraph to right column, paragraph [0002], page 494, right column | 1-12<br>1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 September 2018 (18.09.2018) | 25 September 2018 (25.09.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/024197

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2010-536341 A (AMUNIX, INC.) 02 December 2010, claims, paragraph [0070], example 14, etc. & WO 2009/023270 A2, claims, paragraph [0186], example 14 & US 2009/0092582 A1 & EP 2185701 A2 & CA 2695374 A & KR 10-2010-0058541 A & CN 103298935 A | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03014960 A

### Non-patent literature cited in the description


- **BARKHORDARIAN et al.** *Protein Engineering, Design and Selection,* 01 November 2006, vol. 19 (11), 497-502
- **TARUTANI et al.** *Acta Neuropathol Commun.,* 18 April 2018, vol. 6 (1), 29

SEQUENCE LISTING

<110> RIKEN

<120> Fusion protein

<130> RK18120PCT

<150> JP2017-124596
<151> 2017-06-26

<160> 39

<170> PatentIn version 3.5

<210> 1
<211> 23
<212> PRT
<213> Artificial Sequence

<220>
<223> 3xFlag Tag

<400> 1

```
Met Asp Tyr Lys Asp His Asp Gly Asp Tyr Lys Asp His Asp Ile Asp
1               5                   10                  15

Tyr Lys Asp Asp Asp Asp Lys
            20
```

<210> 2
<211> 14
<212> PRT
<213> Artificial Sequence

<220>
<223> peptide for intracellular stability

<400> 2

```
Glu Glu Asp Gln Asp Asp Glu Asp Asp Glu Asp Gln Asp Asp
1               5                   10
```

<210> 3
<211> 15
<212> PRT
<213> Artificial Sequence

<220>
<223> peptide for intracellular stability

<400> 3

```
Asn Asp Glu Tyr Glu Asp Pro Asp Glu Gln Asp Asp Glu Asn Asp
1               5                   10                  15
```

<210> 4
<211> 15

<212> PRT
<213> Artificial Sequence

<220>
<223> peptide for intracellular stability

<400> 4

Gln Asp Glu Val Asp Glu Pro Glu Asp Glu Glu Asp Asn Asp Asp
1 5 10 15

<210> 5
<211> 15
<212> PRT
<213> Artificial Sequence

<220>
<223> peptide for intracellular stability

<400> 5

Gln Asp Glu Val Asp Glu Pro Glu Asp Glu Asp Glu Asn Asp Asp
1 5 10 15

<210> 6
<211> 15
<212> PRT
<213> Artificial Sequence

<220>
<223> peptide for intracellular stability

<400> 6

Gln Asp Glu Val Asp Glu Pro Glu Asp Glu Asp Glu Asn Gln Asp
1 5 10 15

<210> 7
<211> 15
<212> PRT
<213> Artificial Sequence

<220>
<223> peptide for intracellular stability

<400> 7

Gln Asp Asn Val Asp Glu Pro Glu Asp Asn Asp Glu Asn Gln Asp
1 5 10 15

<210> 8
<211> 15
<212> PRT
<213> Artificial Sequence

<220>
<223> peptide for intracellular stability

<400> 8

Gln Asp Asn Tyr Asp Glu Pro Glu Asp Asn Asp Glu Asn Gln Asp
1               5                   10                  15

<210> 9
<211> 15
<212> PRT
<213> Artificial Sequence

<220>
<223> peptide for intracellular stability

<400> 9

Glu Asp Asn Tyr Asp Glu Pro Glu Asp Asn Asp Glu Asn Gln Asp
1               5                   10                  15

<210> 10
<211> 15
<212> PRT
<213> Artificial Sequence

<220>
<223> peptide for intracellular stability

<400> 10

Asp Asn Asn Tyr Asp Glu Gln Asp Glu Asn Glu Gln Pro Glu Asp
1               5                   10                  15

<210> 11
<211> 16
<212> PRT
<213> Artificial Sequence

<220>
<223> peptide for intracellular stability

<400> 11

Gln Glu Asn Asp Tyr Asp Glu Pro Glu Val Asn Asp Glu Asn Gln Asp
1               5                   10                  15

<210> 12
<211> 18
<212> PRT
<213> Artificial Sequence

<220>
<223> peptide for intracellular stability

<400> 12

Asp Glu Gln Glu Asn Asp Tyr Asp Glu Pro Glu Val Asn Asp Glu Asn
1               5                   10                  15

Gln Asp

<210> 13
<211> 21
<212> PRT
<213> Artificial Sequence

<220>
<223> peptide for intracellular stability

<400> 13

Asp Glu Gln Glu Asn Asp Tyr Asp Glu Pro Glu Val Asn Asp Glu Asn
1               5                   10                  15

Gln Asp Tyr Asp Glu
            20

<210> 14
<211> 9
<212> PRT
<213> Artificial Sequence

<220>
<223> HA Tag

<400> 14

Tyr Pro Tyr Asp Val Pro Asp Tyr Ala
1               5

<210> 15
<211> 12
<212> PRT
<213> Artificial Sequence

<220>
<223> PA Tag

<400> 15

Gly Val Ala Met Pro Gly Ala Glu Asp Asp Val Val
1               5                   10

<210> 16
<211> 72
<212> DNA
<213> Artificial Sequence

<220>
<223> linker primer

<400> 16
gccgccacca tggctagcat gactggtgga cagcaaatgg gtggatccta tgcggcccag 60

ccggccaggg cc 72

<210> 17

<211> 61
<212> DNA
<213> Artificial Sequence

<220>
<223> linker primer

<400> 17
cggcggccgc tcaatgatga tgatgatgat gcaattgccg ttttatttcc aactttgtcc 60

c 61

<210> 18
<211> 29
<212> DNA
<213> Artificial Sequence

<220>
<223> linker primer

<400> 18
gcggatccat ggtgagcaag ggcgaggag 29

<210> 19
<211> 32
<212> DNA
<213> Artificial Sequence

<220>
<223> linker primer

<400> 19
cgagatcttc agaagaactc gtcaagaagg cg 32

<210> 20
<211> 66
<212> DNA
<213> Artificial Sequence

<220>
<223> linker primer

<220>
<221> misc_feature
<222> (18)..(41)
<223> n is a, c, g, or t

<400> 20
gcaaagcttc tggcttcnnn nnnnnnnnnn nnnnnnnnnn ntgggtgaag cagaggcctg 60

cacagg 66

<210> 21
<211> 83
<212> DNA
<213> Artificial Sequence

<220>

<223> linker primer

<220>
<221> misc_feature
<222> (31)..(54)
<223> n is a, c, g, or t

<400> 21
ggagacggtg accgtggtcc cttggcccca nnnnnnnnnn nnnnnnnnnn nnnnagcaca 60

gtaatagacg gcagtgtcct cag 83

<210> 22
<211> 242
<212> PRT
<213> Artificial Sequence

<220>
<223> Y13-259

<400> 22

Gln Val Gln Leu Gln Gln Ser Gly Gly Gly Leu Val Gln Pro Gly Arg
1 5 10 15

Ser Leu Lys Leu Ser Cys Val Val Ser Gly Phe Thr Phe Ser Asn Tyr
20 25 30

Gly Met Asn Trp Ile Arg Gln Thr Pro Gly Lys Gly Leu Glu Trp Val
35 40 45

Ala Tyr Ile Ser Ser Gly Ser Ser Tyr Leu Tyr Tyr Ala Glu Thr Val
50 55 60

Lys Gly Arg Phe Thr Ile Ser Arg Asp Asn Ala Lys Asn Thr Leu Tyr
65 70 75 80

Leu Gln Met Thr Ser Leu Arg Ser Glu Asp Thr Ala Leu Tyr Tyr Cys
85 90 95

Ala Arg His Glu Gly Thr Gly Thr Asp Phe Phe Asp Tyr Trp Gly Gln
100 105 110

Gly Thr Thr Val Thr Val Ser Ser Gly Gly Gly Gly Ser Gly Gly Gly
115 120 125

Gly Ser Gly Gly Gly Gly Ser Asp Ile Gln Leu Thr Gln Ser Pro His
130 135 140

Ser Leu Ser Ala Ser Leu Gly Glu Thr Val Ser Ile Glu Cys Leu Ala
145 150 155 160

Ser Glu Gly Ile Ser Asn Tyr Leu Ala Trp Tyr Gln Gln Lys Pro Gly
165 170 175

Lys Ser Pro Gln Leu Leu Ile Tyr Tyr Ala Ser Ser Leu Gln Asp Gly
180 185 190

Val Pro Ser Arg Phe Ser Gly Ser Gly Ser Gly Thr Gln Phe Ser Leu
195 200 205

Lys Ile Ser Asn Met Gln Pro Glu Asp Glu Gly Val Tyr Tyr Cys Gln
210 215 220

Gln Ala Tyr Lys Tyr Pro Ser Thr Phe Gly Ala Gly Thr Lys Leu Glu
225 230 235 240

Ile Lys

<210> 23
<211> 276
<212> PRT
<213> Artificial Sequence

<220>
<223> STAND-Y13-259

<400> 23

Met Asp Tyr Lys Asp His Asp Gly Asp Tyr Lys Asp His Asp Ile Asp
1 5 10 15

Tyr Lys Asp Asp Asp Asp Lys Gly Ser Gln Val Gln Leu Gln Gln Ser
20 25 30

Gly Gly Gly Leu Val Gln Pro Gly Arg Ser Leu Lys Leu Ser Cys Val
35 40 45

Val Ser Gly Phe Thr Phe Ser Asn Tyr Gly Met Asn Trp Ile Arg Gln
50 55 60

Thr Pro Gly Lys Gly Leu Glu Trp Val Ala Tyr Ile Ser Ser Gly Ser
65 70 75 80

Ser Tyr Leu Tyr Tyr Ala Glu Thr Val Lys Gly Arg Phe Thr Ile Ser
85 90 95

Arg Asp Asn Ala Lys Asn Thr Leu Tyr Leu Gln Met Thr Ser Leu Arg
100 105 110

Ser Glu Asp Thr Ala Leu Tyr Tyr Cys Ala Arg His Glu Gly Thr Gly
115 120 125

Thr Asp Phe Phe Asp Tyr Trp Gly Gln Gly Thr Thr Val Thr Val Ser
130 135 140

Ser Gly Gly Gly Gly Ser Gly Gly Gly Gly Ser Gly Gly Gly Gly Ser
145 150 155 160

Asp Ile Gln Leu Thr Gln Ser Pro His Ser Leu Ser Ala Ser Leu Gly
165 170 175

Glu Thr Val Ser Ile Glu Cys Leu Ala Ser Glu Gly Ile Ser Asn Tyr
180 185 190

Leu Ala Trp Tyr Gln Gln Lys Pro Gly Lys Ser Pro Gln Leu Leu Ile
195 200 205

Tyr Tyr Ala Ser Ser Leu Gln Asp Gly Val Pro Ser Arg Phe Ser Gly
210 215 220

Ser Gly Ser Gly Thr Gln Phe Ser Leu Lys Ile Ser Asn Met Gln Pro
225 230 235 240

Glu Asp Glu Gly Val Tyr Tyr Cys Gln Gln Ala Tyr Lys Tyr Pro Ser
245 250 255

Thr Phe Gly Ala Gly Thr Lys Leu Glu Ile Lys Tyr Pro Tyr Asp Val
260 265 270

Pro Asp Tyr Ala
275

<210> 24
<211> 831
<212> DNA
<213> Artificial Sequence

<220>
<223> STAND-Y13-259

<400> 24
atggattaca aggaccacga cggcgattac aaagaccacg acattgacta caaggacgac 60

gacgacaaag gttcccaggt gcagctgcag cagtctggag gaggactcgt gcagcctggc 120

cgatccctga agctctcttg cgtggtcagt ggcttcactt tttccaacta cggaatgaat 180

tggatcagac agacccctgg taaaggcctg gaatgggtgg cttacattag ctccgggtct 240

agttatctgt actatgcaga gacagtcaag ggcaggttca ctatcagcag agacaacgca 300

aaaaataccc tgtacctcca gatgacatca ctgcggagcg aagataccgc cctctactat 360

tgcgctcgcc acgagggaac tgggaccgac ttctttgatt attggggtca gggcaccaca 420

gtgacagtct caagcggtgg aggagggagt ggtggaggag ggtcaggtgg cggagggagc 480

gacattcagc tgacacagtc cccccatagt ctgtcagcca gcctcggaga gaccgtgagc 540

atcgaatgtc tggcatcaga ggggattagc aactacctcg cctggtatca gcagaagccc 600

ggcaaaagtc ctcagctgct catctactat gcttcctctc tgcaggacgg agtgccttcc 660

aggttctccg gatctgggag tggtacccag ttttccctga agatttctaa tatgcagcca 720

gaggatgaag gcgtctacta ttgtcagcag gcttacaaat atcccagcac cttcggggct 780

gggacaaaac tggaaatcaa atacccttac gatgtgcctg actacgcatg a 831

<210> 25
<211> 768
<212> DNA
<213> Artificial Sequence

<220>
<223> myc-Y13-259

<400> 25
atggcatcaa tgcagaagct gatctcagag gaggacctgc aggtccaact gcagcagtct 60

ggaggaggct tagtgcagcc tggaaggtcc ctgaaactct cctgtgtagt ctctggattc 120

actttcagta actatggaat gaactggatt cgccagactc cagggaaggg actggagtgg 180

gttgcataca ttagtagtgg tagcagttac ctctactatg cagaaacggt gaagggccga 240

ttcaccatct ccagagacaa tgccaagaac accctgtacc tgcaaatgac cagtctgagg 300

tctgaagaca ctgccttgta ttactgtgca agacatgagg gtacgggtac cgacttcttt 360

gattactggg gccaagggac cacggtcacc gtctcctcag gcggaggagg ctccggcggg 420

ggcggatctg gggggcggcgg atccgacatc cagctgaccc agtctccaca ttccctgtct 480

gcatctctgg gagaaactgt ctccatcgaa tgtctagcaa gtgagggcat ttccaattat 540

ttagcgtggt atcagcagaa gccagggaaa tctcctcagc tcctgatcta ttatgcaagt 600

agcttgcaag atggggtccc atcacggttc agtggcagtg gatctggcac acagttttct 660

ctcaagatca gcaacatgca acctgaagat gaaggggttt attactgtca acaggcttac 720

aagtatcctt ccacgtttgg agctgggacc aagctggaga tcaaatga 768

<210> 26
<211> 255
<212> PRT
<213> Artificial Sequence

<220>
<223> myc-Y13-259

<400> 26

```
Met Ala Ser Met Gln Lys Leu Ile Ser Glu Glu Asp Leu Gln Val Gln
1               5                   10                  15

Leu Gln Gln Ser Gly Gly Gly Leu Val Gln Pro Gly Arg Ser Leu Lys
            20                  25                  30

Leu Ser Cys Val Val Ser Gly Phe Thr Phe Ser Asn Tyr Gly Met Asn
        35                  40                  45

Trp Ile Arg Gln Thr Pro Gly Lys Gly Leu Glu Trp Val Ala Tyr Ile
    50                  55                  60

Ser Ser Gly Ser Ser Tyr Leu Tyr Tyr Ala Glu Thr Val Lys Gly Arg
65                  70                  75                  80

Phe Thr Ile Ser Arg Asp Asn Ala Lys Asn Thr Leu Tyr Leu Gln Met
                85                  90                  95

Thr Ser Leu Arg Ser Glu Asp Thr Ala Leu Tyr Tyr Cys Ala Arg His
            100                 105                 110

Glu Gly Thr Gly Thr Asp Phe Phe Asp Tyr Trp Gly Gln Gly Thr Thr
        115                 120                 125

Val Thr Val Ser Ser Gly Gly Gly Gly Ser Gly Gly Gly Gly Ser Gly
    130                 135                 140

Gly Gly Gly Ser Asp Ile Gln Leu Thr Gln Ser Pro His Ser Leu Ser
145                 150                 155                 160

Ala Ser Leu Gly Glu Thr Val Ser Ile Glu Cys Leu Ala Ser Glu Gly
                165                 170                 175

Ile Ser Asn Tyr Leu Ala Trp Tyr Gln Gln Lys Pro Gly Lys Ser Pro
            180                 185                 190

Gln Leu Leu Ile Tyr Tyr Ala Ser Ser Leu Gln Asp Gly Val Pro Ser
        195                 200                 205

Arg Phe Ser Gly Ser Gly Ser Gly Thr Gln Phe Ser Leu Lys Ile Ser
    210                 215                 220

Asn Met Gln Pro Glu Asp Glu Gly Val Tyr Tyr Cys Gln Gln Ala Tyr
225                 230                 235                 240

Lys Tyr Pro Ser Thr Phe Gly Ala Gly Thr Lys Leu Glu Ile Lys
```

245 250 255

<210> 27
<211> 266
<212> PRT
<213> Artificial Sequence

<220>
<223> DE2.0-Y13-259

<400> 27

Met Asp Glu Gln Glu Asn Asp Tyr Asp Glu Pro Glu Val Asn Asp Glu
1 5 10 15

Asn Gln Asp Tyr Asp Glu Gly Ser Gln Val Gln Leu Gln Gln Ser Gly
20 25 30

Gly Gly Leu Val Gln Pro Gly Arg Ser Leu Lys Leu Ser Cys Val Val
35 40 45

Ser Gly Phe Thr Phe Ser Asn Tyr Gly Met Asn Trp Ile Arg Gln Thr
50 55 60

Pro Gly Lys Gly Leu Glu Trp Val Ala Tyr Ile Ser Ser Gly Ser Ser
65 70 75 80

Tyr Leu Tyr Tyr Ala Glu Thr Val Lys Gly Arg Phe Thr Ile Ser Arg
85 90 95

Asp Asn Ala Lys Asn Thr Leu Tyr Leu Gln Met Thr Ser Leu Arg Ser
100 105 110

Glu Asp Thr Ala Leu Tyr Tyr Cys Ala Arg His Glu Gly Thr Gly Thr
115 120 125

Asp Phe Phe Asp Tyr Trp Gly Gln Gly Thr Thr Val Thr Val Ser Ser
130 135 140

Gly Gly Gly Gly Ser Gly Gly Gly Gly Ser Gly Gly Gly Gly Ser Asp
145 150 155 160

Ile Gln Leu Thr Gln Ser Pro His Ser Leu Ser Ala Ser Leu Gly Glu
165 170 175

Thr Val Ser Ile Glu Cys Leu Ala Ser Glu Gly Ile Ser Asn Tyr Leu
180 185 190

Ala Trp Tyr Gln Gln Lys Pro Gly Lys Ser Pro Gln Leu Leu Ile Tyr
195 200 205

Tyr Ala Ser Ser Leu Gln Asp Gly Val Pro Ser Arg Phe Ser Gly Ser
    210                 215                 220

Gly Ser Gly Thr Gln Phe Ser Leu Lys Ile Ser Asn Met Gln Pro Glu
225                 230                 235                 240

Asp Glu Gly Val Tyr Tyr Cys Gln Gln Ala Tyr Lys Tyr Pro Ser Thr
                245                 250                 255

Phe Gly Ala Gly Thr Lys Leu Glu Ile Lys
            260                 265

<210>  28
<211>  275
<212>  PRT
<213>  Artificial Sequence

<220>
<223>  DE2.0-Y13-259-HA

<400>  28

Met Asp Glu Gln Glu Asn Asp Tyr Asp Glu Pro Glu Val Asn Asp Glu
1               5                   10                  15

Asn Gln Asp Tyr Asp Glu Gly Ser Gln Val Gln Leu Gln Gln Ser Gly
            20                  25                  30

Gly Gly Leu Val Gln Pro Gly Arg Ser Leu Lys Leu Ser Cys Val Val
        35                  40                  45

Ser Gly Phe Thr Phe Ser Asn Tyr Gly Met Asn Trp Ile Arg Gln Thr
    50                  55                  60

Pro Gly Lys Gly Leu Glu Trp Val Ala Tyr Ile Ser Ser Gly Ser Ser
65                  70                  75                  80

Tyr Leu Tyr Tyr Ala Glu Thr Val Lys Gly Arg Phe Thr Ile Ser Arg
                85                  90                  95

Asp Asn Ala Lys Asn Thr Leu Tyr Leu Gln Met Thr Ser Leu Arg Ser
            100                 105                 110

Glu Asp Thr Ala Leu Tyr Tyr Cys Ala Arg His Glu Gly Thr Gly Thr
        115                 120                 125

Asp Phe Phe Asp Tyr Trp Gly Gln Gly Thr Thr Val Thr Val Ser Ser
    130                 135                 140

Gly Gly Gly Gly Ser Gly Gly Gly Gly Ser Gly Gly Gly Gly Ser Asp
145                 150                 155                 160

Ile Gln Leu Thr Gln Ser Pro His Ser Leu Ser Ala Ser Leu Gly Glu
                165                 170                 175

Thr Val Ser Ile Glu Cys Leu Ala Ser Glu Gly Ile Ser Asn Tyr Leu
            180                 185                 190

Ala Trp Tyr Gln Gln Lys Pro Gly Lys Ser Pro Gln Leu Leu Ile Tyr
        195                 200                 205

Tyr Ala Ser Ser Leu Gln Asp Gly Val Pro Ser Arg Phe Ser Gly Ser
    210                 215                 220

Gly Ser Gly Thr Gln Phe Ser Leu Lys Ile Ser Asn Met Gln Pro Glu
225                 230                 235                 240

Asp Glu Gly Val Tyr Tyr Cys Gln Gln Ala Tyr Lys Tyr Pro Ser Thr
                245                 250                 255

Phe Gly Ala Gly Thr Lys Leu Glu Ile Lys Tyr Pro Tyr Asp Val Pro
            260                 265                 270

Asp Tyr Ala
        275

<210> 29
<211> 280
<212> PRT
<213> Artificial Sequence

<220>
<223> DE2.0-Y13-259-PA

<400> 29

Met Asp Glu Gln Glu Asn Asp Tyr Asp Glu Pro Glu Val Asn Asp Glu
1               5                   10                  15

Asn Gln Asp Tyr Asp Glu Gly Ser Gln Val Gln Leu Gln Gln Ser Gly
            20                  25                  30

Gly Gly Leu Val Gln Pro Gly Arg Ser Leu Lys Leu Ser Cys Val Val
        35                  40                  45

Ser Gly Phe Thr Phe Ser Asn Tyr Gly Met Asn Trp Ile Arg Gln Thr
    50                  55                  60

```
Pro Gly Lys Gly Leu Glu Trp Val Ala Tyr Ile Ser Ser Gly Ser Ser
65                  70                  75                  80

Tyr Leu Tyr Tyr Ala Glu Thr Val Lys Gly Arg Phe Thr Ile Ser Arg
                85                  90                  95

Asp Asn Ala Lys Asn Thr Leu Tyr Leu Gln Met Thr Ser Leu Arg Ser
            100                 105                 110

Glu Asp Thr Ala Leu Tyr Tyr Cys Ala Arg His Glu Gly Thr Gly Thr
        115                 120                 125

Asp Phe Phe Asp Tyr Trp Gly Gln Gly Thr Thr Val Thr Val Ser Ser
    130                 135                 140

Gly Gly Gly Gly Ser Gly Gly Gly Gly Ser Gly Gly Gly Gly Ser Asp
145                 150                 155                 160

Ile Gln Leu Thr Gln Ser Pro His Ser Leu Ser Ala Ser Leu Gly Glu
                165                 170                 175

Thr Val Ser Ile Glu Cys Leu Ala Ser Glu Gly Ile Ser Asn Tyr Leu
            180                 185                 190

Ala Trp Tyr Gln Gln Lys Pro Gly Lys Ser Pro Gln Leu Leu Ile Tyr
        195                 200                 205

Tyr Ala Ser Ser Leu Gln Asp Gly Val Pro Ser Arg Phe Ser Gly Ser
    210                 215                 220

Gly Ser Gly Thr Gln Phe Ser Leu Lys Ile Ser Asn Met Gln Pro Glu
225                 230                 235                 240

Asp Glu Gly Val Tyr Tyr Cys Gln Gln Ala Tyr Lys Tyr Pro Ser Thr
                245                 250                 255

Phe Gly Ala Gly Thr Lys Leu Glu Ile Lys Glu Gly Gly Val Ala Met
            260                 265                 270

Pro Gly Ala Glu Asp Asp Val Val
        275                 280
```

<210>  30
<211>  828
<212>  DNA
<213>  Artificial Sequence

<220>
<223>  DE2.0-Y13-259-HA

<400> 30
atggatgaac aggagaacga ttatgatgag ccagaagtga acgacgagaa ccaggattat 60

gatgagggat cccaggtcca gctgcagcag tcaggaggag gcctggtgca gcctggccgg 120

tccctgaagc tgtcttgcgt ggtgagcggc ttcaccttta gcaactacgg catgaattgg 180

atcaggcaga caccaggcaa gggcctggag tgggtggcct acatcagctc cggctctagc 240

tatctgtact atgccgagac agtgaagggc cggttcacaa tctccagaga caacgccaag 300

aataccctgt acctgcagat gacatctctg aggagcgagg ataccgccct gtactattgc 360

gcaaggcacg agggaaccgg aacagacttc tttgattatt ggggccaggg caccacagtg 420

acagtgtcct ctggcggcgg cggctctgga ggaggaggaa gcggaggagg aggctccgac 480

atccagctga cccagtctcc acactctctg agcgcctccc tgggagagac agtgagcatc 540

gagtgtctgg cctccgaggg catctctaac tacctggcct ggtatcagca gaagcccggc 600

aagtcccctc agctgctgat ctactatgcc agctccctgc aggacggcgt gccttctcgg 660

ttctctggaa gcggctccgg aacccagttt agcctgaaga tctccaatat gcagccagag 720

gatgagggcg tgtactattg tcagcaggcc tacaagtatc ccagcacctt cggggctgga 780

actaaactgg aaatcaaata cccttacgat gtgcctgact acgcttga 828

<210> 31
<211> 22
<212> PRT
<213> Artificial Sequence

<220>
<223> HSC70 binding peptide

<400> 31

Met Ala Arg Val Lys Lys Asp Gln Ala Glu Pro Leu His Arg Lys Phe
1               5                   10                  15

Glu Arg Gln Pro Pro Gly
            20

<210> 32
<211> 320
<212> PRT
<213> Artificial Sequence

<220>
<223> STAND-6E-LYS

<400> 32

Met Asp Glu Gln Glu Asn Asp Tyr Asp Glu Pro Glu Val Asn Asp Glu
1               5                   10                  15

Asn Gln Asp Tyr Asp Glu Gly Ser Ala Glu Val Gln Leu Leu Glu Ser
20 25 30

Gly Gly Gly Leu Val Gln Pro Gly Gly Ser Leu Arg Leu Ser Cys Ala
35 40 45

Ala Ser Gly Phe Thr Phe Ser Ser Tyr Ala Met Ser Trp Val Arg Gln
50 55 60

Ala Pro Gly Lys Gly Leu Glu Trp Val Ser Tyr Ile Ala Ser Gly Gly
65 70 75 80

Asp Thr Thr Asn Tyr Ala Asp Ser Val Lys Gly Arg Phe Thr Ile Ser
85 90 95

Arg Asp Asn Ser Lys Asn Thr Leu Tyr Leu Gln Met Asn Ser Leu Arg
100 105 110

Ala Glu Asp Thr Ala Val Tyr Tyr Cys Ala Lys Gly Ala Ser Ala Phe
115 120 125

Asp Tyr Trp Gly Gln Gly Thr Leu Val Thr Val Ser Ser Gly Gly Gly
130 135 140

Gly Ser Gly Gly Gly Gly Ser Gly Gly Gly Gly Ser Thr Asp Ile Gln
145 150 155 160

Met Thr Gln Ser Pro Ser Ser Leu Ser Ala Ser Val Gly Asp Arg Val
165 170 175

Thr Ile Thr Cys Arg Ala Ser Gln Ser Ile Ser Ser Tyr Leu Asn Trp
180 185 190

Tyr Gln Gln Lys Pro Gly Lys Ala Pro Lys Leu Leu Ile Tyr Ala Ala
195 200 205

Ser Tyr Leu Gln Ser Gly Val Pro Ser Arg Phe Ser Gly Ser Gly Ser
210 215 220

Gly Thr Asp Phe Thr Leu Thr Ile Ser Ser Leu Gln Pro Glu Asp Phe
225 230 235 240

Ala Thr Tyr Tyr Cys Gln Gln Ser Ser Asn Asp Pro Tyr Thr Phe Gly
245 250 255

Gln Gly Thr Lys Val Glu Ile Lys Arg Arg Ser Met Asp Glu Gln Glu
260 265 270

Asn Asp Tyr Asp Glu Pro Glu Val Asn Asp Glu Asn Gln Asp Tyr Asp
275 280 285

Glu Met Ala Arg Val Lys Lys Asp Gln Ala Glu Pro Leu His Arg Lys
290 295 300

Phe Glu Arg Gln Pro Pro Gly Tyr Pro Tyr Asp Val Pro Asp Tyr Ala
305 310 315 320

<210> 33
<211> 22
<212> PRT
<213> Artificial Sequence

<220>
<223> DE5.0 tag

<400> 33

Asp Asp Asp Glu Asp Glu Asp Asp Glu Asp Glu Asp Glu Asp Glu Asp
1 5 10 15

Glu Asp Glu Asp Glu Asp
20

<210> 34
<211> 11
<212> PRT
<213> Artificial Sequence

<220>
<223> T7 tag

<400> 34

Met Ala Ser Met Thr Gly Gly Gln Gln Met Gly
1 5 10

<210> 35
<211> 279
<212> PRT
<213> Artificial Sequence

<220>
<223> DE5.0-6E

<400> 35

Met Asp Asp Asp Glu Asp Glu Asp Asp Glu Asp Glu Asp Glu Asp Glu
1 5 10 15

Asp Glu Asp Glu Asp Glu Asp Gly Ser Ala Glu Val Gln Leu Leu Glu
20 25 30

```
Ser Gly Gly Gly Leu Val Gln Pro Gly Gly Ser Leu Arg Leu Ser Cys
        35                  40                  45

Ala Ala Ser Gly Phe Thr Phe Ser Ser Tyr Ala Met Ser Trp Val Arg
    50                  55                  60

Gln Ala Pro Gly Lys Gly Leu Glu Trp Val Ser Tyr Ile Ala Ser Gly
65                  70                  75                  80

Gly Asp Thr Thr Asn Tyr Ala Asp Ser Val Lys Gly Arg Phe Thr Ile
                85                  90                  95

Ser Arg Asp Asn Ser Lys Asn Thr Leu Tyr Leu Gln Met Asn Ser Leu
            100                 105                 110

Arg Ala Glu Asp Thr Ala Val Tyr Tyr Cys Ala Lys Gly Ala Ser Ala
        115                 120                 125

Phe Asp Tyr Trp Gly Gln Gly Thr Leu Val Thr Val Ser Ser Gly Gly
    130                 135                 140

Gly Gly Ser Gly Gly Gly Gly Ser Gly Gly Gly Gly Ser Thr Asp Ile
145                 150                 155                 160

Gln Met Thr Gln Ser Pro Ser Ser Leu Ser Ala Ser Val Gly Asp Arg
                165                 170                 175

Val Thr Ile Thr Cys Arg Ala Ser Gln Ser Ile Ser Ser Tyr Leu Asn
            180                 185                 190

Trp Tyr Gln Gln Lys Pro Gly Lys Ala Pro Lys Leu Leu Ile Tyr Ala
        195                 200                 205

Ala Ser Tyr Leu Gln Ser Gly Val Pro Ser Arg Phe Ser Gly Ser Gly
    210                 215                 220

Ser Gly Thr Asp Phe Thr Leu Thr Ile Ser Ser Leu Gln Pro Glu Asp
225                 230                 235                 240

Phe Ala Thr Tyr Tyr Cys Gln Gln Ser Ser Asn Asp Pro Tyr Thr Phe
                245                 250                 255

Gly Gln Gly Thr Lys Val Glu Ile Lys Arg Arg Ser Met Ala Ser Met
            260                 265                 270

Thr Gly Gly Gln Gln Met Gly
        275
```

<210> 36
<211> 840
<212> DNA
<213> Artificial Sequence

<220>
<223> DE5.0-6E

<400> 36
atggatgacg acgaggacga agacgacgaa gatgaggatg aagatgaaga cgaggatgag 60

gacgaggacg gatctgccga agtccagctt ctcgagtctg gcggcgggtt ggtgcagcct 120

ggaggatccc tgagattatc ctgtgcagct agcgggttca ctttctcgtc ctatgccatg 180

tcatgggttc gccaagctcc cggcaaaggg ttggagtggg tgagctacat tgccagtgga 240

ggcgatacta ccaattacgc ggacagtgtg aagggtaggt ttaccattag ccgggataac 300

agcaagaaca ctctctacct gcagatgaac tctctgagag cggaggatac agccgtgtac 360

tattgcgcaa aaggcgcatc cgctttcgac tattggggtc aaggcactct cgtaaccgtg 420

tctagtggcg gaggtggttc aggggcggc ggatcaggag ggggaggcag tacagatatc 480

cagatgacac agagtccctc ctctctgtcc gcttctgtcg gcgatcgagt cacgattacg 540

tgtcgtgcta gtcagtctat cagctcatac ctcaattggt accagcagaa gcctggaaaa 600

gcccccaaac tgctgatcta tgccgcctcc tacctacaga gcggtgttcc aagccgcttt 660

tcaggcagcg ggtctgggac agatttcact ctgaccatat cctcgcttca gccagaggac 720

tttgcaacct actattgcca gcagtcctca aatgacccgt atacctttgg acaagggacc 780

aaggtggaaa tcaagcggag gagcatggcc agcatgacag gtgggcaaca aatgggctga 840

<210> 37
<211> 969
<212> DNA
<213> Artificial Sequence

<220>
<223> STAND-6E-LYS

<400> 37
gccaccatgg atgaacagga gaacgattat gatgagccag aagtgaacga cgagaaccag 60

gattatgatg agggatccgc cgaggtgcag ctgttggagt ctgggggagg cttggtacag 120

cctggggggt ccctgagact ctcctgtgca gcctctggat tcacctttag cagctatgcc 180

atgagctggg tccgccaggc tccagggaag gggctggagt gggtctcata tattgctagt 240

ggtggtgata ctacaaatta cgcagactcc gtgaagggcc ggttcaccat ctccagagac 300

aattccaaga acacgctgta tctgcaaatg aacagcctga gagccgagga cacggccgta 360

tattactgtg cgaaaggtgc ttctgctttt gactactggg gccagggaac cctggtcacc 420

gtctcgagcg gtggaggcgg ttcaggcgga ggtggcagcg gcggtggcgg gtcgacggac 480

```
atccagatga cccagtctcc atcctccctg tctgcatctg taggagacag agtcaccatc      540

acttgccggg caagtcagag cattagcagc tatttaaatt ggtatcagca gaaaccaggg      600

aaagccccta agctcctgat ctatgctgca tcctatttgc aaagtggggt cccatcaagg      660

ttcagtggca gtggatctgg gacagatttc actctcacca tcagcagtct gcaacctgaa      720

gattttgcaa cttactactg tcaacagagt tctaatgatc cttatacgtt cggccaaggg      780

accaaggtgg aaatcaaacg gagatctatg gatgaacagg agaacgatta tgatgagcca      840

gaagtgaacg acgagaacca ggattatgat gagatggcca gagtgaagaa ggaccaggct      900

gagcctctcc atagaaagtt tgagagacag ccaccagggt acccttacga tgtgcctgac      960

tacgcttga                                                               969


<210>  38
<211>  5
<212>  PRT
<213>  Artificial Sequence

<220>
<223>  HSC70 binding motif

<400>  38

Val Lys Lys Asp Gln
1               5


<210>  39
<211>  5
<212>  PRT
<213>  Artificial Sequence

<220>
<223>  HSC70 binding consensus motif

<400>  39

Lys Phe Glu Arg Gln
1               5
```